# EUROPEAN PATENT SPECIFICATION

(11) **EP 3 972 589 B1**
(45) Date of publication and mention of the grant of the patent: **03.07.2024**
(21) Application number: 20808662.9
(22) Date of filing: 18.05.2020
(51) Int. Cl.: A61P 13/12, A61P 3/10, C07D 401/12, C07D 401/14, C07D 413/14, C07D 487/10, C07D 491/107, C07D 498/08, A61K 31/435

(54) **NATRIURETIC PEPTIDE RECEPTOR A AGONISTS USEFUL FOR THE TREATMENT OF CARDIOMETABOLIC DISEASES, KIDNEY DISEASE AND DIABETES**
NATRIURETISCHE PEPTID-REZEPTOR-A-AGONISTEN ZUR BEHANDLUNG VON KARDIOMETABOLISCHEN ERKRANKUNGEN, NIERENERKRANKUNGEN UND DIABETES
AGONISTES DU RÉCEPTEUR DU PEPTIDE NATRIURÉTIQUE A UTILES POUR LE TRAITEMENT DE MALADIES CARDIOMÉTABOLIQUES, DE MALADIES RÉNALES ET DU DIABÈTE

(30) Priority: 22.05.2019 US 201962851534 P
(43) Date of publication of application: 30.03.2022
(73) Proprietor: Merck Sharp & Dohme LLC, Rahway, New Jersey 07065 (US)
(72) Inventor: BENNETT, Frank, Kenilworth, New Jersey 07033 (US); IMBRIGLIO, Jason, E., Kenilworth, New Jersey 07033 (US); KEREKES, Angela, D., Kenilworth, New Jersey 07033 (US); KHAN, Tanweer, Bridgewater, New Jersey 08807 (US); LANKIN, Claire, Kenilworth, New Jersey 07033 (US); LI, Derun, Boston, Massachusetts 02115-5727 (US); WU, Zhicai, Kenilworth, New Jersey 07033 (US); XIONG, Yusheng, Plainsboro, New Jersey 08536 (US); YOUM, Hyewon, Kenilworth, New Jersey 07033 (US); YU, Yang, Kenilworth, New Jersey 07033 (US); KURISSERY, Anthappan Tony, Bangalore 560100 (IN); KOMANDURI, Venukrishnan, Manchester M14 5SL (GB); YE, Feng, deceased (US)
(74) Representative: Merck Sharp & Dohme LLC
(86) International application number: PCT/US2020/033356
(87) International publication number: WO 2020/236690

(56) References cited:
- WO-A1-2018/057618
- WO-A1-2018/098473
- WO-A1-2018/175534
- WO-A1-2020/236688
- WU et al.: "Chemical Approaches to Intervening in Ubiquitin Specific Protease 7 ( USP 7) Function for Oncology and Immune Oncology Therapies", J. Med. Chem., vol. 61, 2018, pages 422-443, XP055744660, DOI: 10.1021/acs.jmedchem.7b00498
- DATABASE PubChem Compound 5 January 2010 (2010-01-05), "9-Amino-N-phenyl-5,6,7,8-tetrahydroacridi ne-2-carboxamide", XP055761765, Database accession no. CID 44537311

## Description

### FIELD OF THE INVENTION

The present invention relates to compounds useful for activating Natriuretic Peptide Receptor A (NPRA) and for use in treating or prevention of cardiometabolic diseases including high blood pressure, heart failure, kidney disease, and diabetes.

### BACKGROUND OF THE INVENTION

Natriuretic Peptide Receptor A (NPRA) is a receptor widely distributed in the human myocardium. (Molecular Biology of the Natriuretic Peptide System Implications for Physiology and Hypertension David G. Gardner, Songcang Chen, Denis J. Glenn, Chris L. Grigsby Hypertension. 2007;49:419-426). The peptide hormone Atrial natriuretic peptide (ANP) and B-type natriuretic peptide (BNP), secreted from heart, and their homolog urodilatin (URO), secreted from vasculature and kidney, all activate NPRA to stimulate the production of cyclic guanosine monophosphate ("cGMP"). (Potter LR, Abbey-Hosch S, Dickey DM. Natriuretic peptides, their receptors, and cyclic guanosine monophosphate-dependent signaling functions. Endocr Rev. 2006;27:47-72).

The biologic effects of these natriuretic peptides range from acute vassal dilation, diuresis and natriuresis to long lasting effect of anti-proliferation, tissue remodeling, and energy metabolism. Recombinant ANP (Carperitide) and BNP (Nesiritide) have been used as treatment for congestive heart failure. But the very short half-lives of these peptide hormones (2 to 20 min), and complex processing and clearance of ANP and BNP in local tissues are part of the difficulties in studying the impact of sustained activation of NPRA over long period in clinical settings. (The Pharmacokinetics of Alpha-Human Atrial Natriuretic Polypeptide in Healthy Subjects; Nakao K, Sugawara A, Morii N, Sakamoto M, Yamada T, Itoh H et al (1986). Eur J Clin Pharmacol 31:101-103). Small molecules that activate NPRA over long periods of time can mimic the beneficial effects of the natriuretic peptides. WO 2020/236688 and WO 2018/175534 disclose other NPRA agonist compounds.

Thus, there is a need for small molecule NPRA agonists that are useful in treating cardiometabolic diseases including high blood pressure, heart failure, kidney disease, and diabetes.

### SUMMARY OF THE INVENTION

The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

In one aspect, the present invention provides compounds of Formula I or pharmaceutically acceptable salts thereof: 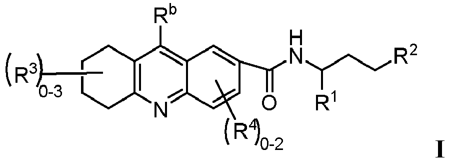
**R^{b}** is hydrogen, halogen, C₁₋₆ alkyl, hydroxy, -(C₁₋₁₀ alkyl)OH, or C₁₋₆haloalkyl;
**R¹** is selected from phenyl, pyridyl, thiazolyl, imidazolyl, pyrazinyl, and oxadiazolyl, wherein **R¹** is substituted by 0, 1, 2, or 3, **R⁶**;
**R²** is independently selected from:
   C₁₋₁₀ alkyl,
   arylC₀₋₁₀ alkyl,
   C₃₋₁₂ cycloalkylC₀₋₁₀ alkyl,
   heteroarylC₀₋₁₀ alkyl,
   heterocyclylC₀₋₁₀ alkyl,
   C₁₋₁₀alkylaminoC₀₋₁₀ alkyl,
   heteroarylC₀₋₁₀alkylaminoC₀₋₁₀ alkyl,
   heterocyclylC₀₋₁₀alkylaminoC₀₋₁₀ alkyl,
   C₁₋₁₀ heteroalkyl aminoC₀₋₁₀ alkyl,
   C₃₋₁₂ cycloalkyl C₀₋₁₀ alkylaminoC₀₋₁₀ alkyl,
   aryl C₀₋₁₀ alkylaminoC₀₋₁₀ alkyl,
   amino, and
   (C₁₋₁₀ alkyl)₁₋₂ aminoC₀₋₁₀ alkyl, wherein **R²** is each substituted with 0,1, 2, 3, or 4 **R⁵** substituents;
each **R³** is independently selected from halogen, C₁₋₆ alkyl, C₃₋₁₂ cycloalkyl,
   -Si(CH₃)₃ and C₁₋₆haloalkyl, wherein each **R³** is independently substituted with 0,1, or 2, **R⁸** substituents and each **R⁸** is independently selected from: C₁₋₆ alkyl, C₁₋₆ alkoxy, halogen, and C1-6haloalkyl;
each **R⁴** is independently selected from halogen, C₁₋₆ alkyl, and C₁₋₆haloalkyl;
each **R⁵** is independently selected from:
   halogen,
   C₁₋₁₀ alkyl,
   C₁₋₁₀ heteroalkyl,
   aryl C₀₋₁₀ alkyl,
   C₃₋₁₂ cycloalkyl C₀₋₁₀ alkyl,
   heteroaryl C₀₋₁₀ alkyl,
   heterocyclylC₀₋₁₀ alkyl,
   amino C₀₋₁₀ alkyl,
   ((C₁₋₁₀alkyl)₁₋₂amino)C₀₋₁₀ alkyl,
   -CO₂(C₁₋₁₀ alkyl),
   -(C₀₋₁₀ alkyl)CO₂H,
   -(C₀₋₁₀ alkyl)CO₂(C₁₋₁₀ alkyl),
   Oxo (=O),
   hydroxy,
   -(C₁₋₁₀ alkyl)OH,
   C₁₋₁₀ alkoxyC₀₋₁₀ alkyl,
   cyano, and
   C₁₋₆haloalkyl, wherein each **R⁵** is independently substituted with 0,1, 2, 3, or 4 **R⁹** and each **R⁹** is independently selected from: C₁₋₆ alkyl, hydroxy, C₁₋₆ alkoxy, halogen, and C₁₋₆haloalkyl;
**R⁶** is independently selected from:
   halogen,
   C₁₋₁₀ alkyl,
   aryl C₀₋₁₀ alkyl,
   C₃₋₁₂ cycloalkylC₀₋₁₀ alkyl,
   heteroaryl C₀₋₁₀ alkyl,
   heterocyclyl C₀₋₁₀ alkyl,
   ((C₀₋₁₀)alkyl)₁₋₂aminocarbonyl,
   C₁₋₁₀ alkoxy(C₀₋₁₀)alkyl,
   (C₀₋₁₀)alkylaminocarbonyl,
   (C₁₋₁₀)heteroalkylaminocarbonyl,
   aryl(C₁₋₁₀)alkylaminocarbonyl,
   (C₃₋₁₂)cycloalkyl(C₁₋₁₀)alkylaminocarbonyl,
   heteroaryl(C₁₋₁₀)alkylaminocarbonyl,
   heterocyclyl(C₁₋₁₀)alkylaminocarbonyl,
   -NHSO₂(C₀₋₆ alkyl),
   -SO₂N(C₁₋₆ alkyl)₀₋₂,
   -SO₂CF₃,
   -SO₂CF₂H,
   amino,
   (C₀₋₁₀ alkyl)₁₋₂ amino,
   hydroxy,
   -(C₁₋₁₀ alkyl)OH,
   cyano,
   C₁₋₆haloalkyl,
   -CO₂(C₁₋₁₀ alkyl),
   Oxo (=O);
   C₁₋₁₀ alkylS(O)₁₋₂,
   C₁₋₁₀ heteroalkyl S(O)₁₋₂,
   (C₃₋₁₂) cycloalkylS(O)₁₋₂,
   heterocyclyl S(O)₁₋₂,
   heteroarylS(O)₁₋₂, and
   arylS(O)₁₋₂;
wherein **R⁶** is each substituted with 0,1, 2, 3, or 4 **R⁷;**
each **R⁷** is independently selected from:
   halogen,
   C₁₋₁₀ alkyl,
   C₁₋₆ haloalkyl,
   C₁₋₁₀ heteroalkyl,
   aryl C₀₋₁₀ alkyl,
   C₃₋₁₂ cycloalkyl C₀₋₁₀ alkyl,
   heteroaryl C₀₋₁₀ alkyl,
   heterocyclyl C₀₋₁₀ alkyl,
   amino C₀₋₁₀ alkyl,
   ((C₁₋₁₀)alkyl)₁₋₂amino,
   -CO₂(C₁₋₁₀ alkyl),
   -(C₀₋₁₀ alkyl)CO₂H,
   Oxo (=O),
   hydroxy,
   -(C₁₋₁₀ alkyl)OH,
   C₁₋₁₀ alkoxy,
   cyano, and
   aminocarbonyl.

The Compounds of Formula I and pharmaceutically acceptable salts thereof may be useful, for example, for activating NPRA and for treating or preventing cardiometabolic diseases including high blood pressure, heart failure, kidney disease, and diabetes.

Accordingly, the present invention provides methods for treating or preventing cardiometabolic diseases including high blood pressure, heart failure, kidney disease, and diabetes, in a subject, comprising administering to the subject an effective amount of at least one compound of Formula I.

The present invention also includes pharmaceutical compositions containing a compound of the present invention and methods of preparing such pharmaceutical compositions.

Other embodiments, aspects and features of the present invention are either further described in or will be apparent from the ensuing description, examples and appended claims.

### DETAILED DESCRIPTION OF THE INVENTION

The present invention includes compounds of formula I above, and pharmaceutically acceptable salts thereof. The compounds of formula I are agonists of Natriuretic Peptide Receptor A (NPRA) and are useful for treating or prevention of cardiometabolic diseases including high blood pressure, heart failure, kidney disease, and diabetes.

In a first embodiment of the invention, **R¹** is phenyl or pyridyl, wherein **R¹** is substituted by 0, 1, 2, or 3, **R⁶** and the other groups are as provided in the general formula above.

In a second embodiment of the invention, each **R⁵** is independently selected from:
C₁₋₁₀ alkyl, C₃₋₁₂ cycloalkyl C₀₋₁₀ alkyl, heteroaryl C₀₋₁₀ alkyl, heterocyclylC₀₋₁₀ alkyl, ((C₁₋₁₀alkyl)₁₋₂amino)C₀₋₁₀ alkyl, -CO₂(C₁₋₁₀ alkyl), -(C₀₋₁₀ alkyl)CO₂H, -(C₀₋₁₀ alkyl)CO₂(C₁₋₁₀ alkyl), Oxo (=O), hydroxy, -(C₁₋₁₀ alkyl)OH, C₁₋₁₀ alkoxyC₀₋₁₀ alkyl, and C₁₋₆haloalkyl, wherein each **R⁵** is independently substituted with 0,1, 2, 3, or 4 **R⁹** and the other groups are as provided in the general formula above, or as in the first embodiment.

In a third embodiment of the invention, each **R⁵** is independently selected from:
C₁₋₁₀ alkyl, heterocyclylC₀₋₁₀ alkyl, ((C₁₋₁₀alkyl)₁₋₂amino)C⁰⁻¹⁰ alkyl, -CO₂(C₁₋₁₀ alkyl), -(C₀₋₁₀ alkyl)CO₂H, -(C₀₋₁₀ alkyl)CO₂(C₁₋₁₀ alkyl), hydroxy, -(C₁₋₁₀ alkyl)OH, C₁₋₁₀ alkoxyC₀₋₁₀ alkyl, and C₁₋₆haloalkyl, wherein each **R⁵** is independently substituted with 0,1, 2, 3, or 4 **R⁹**, and the other groups are as provided in the general formula above, or as in the first embodiment.

In a fourth embodiment of the invention, each **R⁵** is independently selected from: methyl, isopropyl, ethyl, butyl, *tert*-butyl, (cyclopropyl)methyl, morpholinomethyl, methoxymethyl, methoxypropyl, methoxy, (dimethylamino)ethyl, (dimethylamino)methyl, hydroxymethyl, carboxy, hydroxy(methylethyl), trifluoromethyl, wherein each **R⁵** is independently substituted with 0,1, 2, 3, or 4 **R⁹**, and the other groups are provided in the general formula above, or as in the first embodiment.

In a fifth embodiment of the invention, each **R⁹** is independently selected from: methyl, ethyl, hydroxy, methoxy, ethoxy, halogen, and trifluoromethyl; and the other groups are provided in the general formula above, or as in the first through fourth embodiments.

In a sixth embodiment of the invention, each **R⁹** is independently selected from: hydroxy, methoxy, ethoxy, halogen, and trifluoromethyl; and the other groups are provided in the general formula above, or as in the first through fourth embodiments.

In a seventh embodiment of the invention, each **R⁶** is independently selected from: halogen, heteroaryl C₀₋₁₀ alkyl, heterocyclyl C₀₋₁₀ alkyl, heterocyclyl(C₀₋₁₀)alkylaminocarbonyl, -NHSO₂(C₀₋₆ alkyl), -SO₂N(C₁₋₆ alkyl)₀₋₂, C₁₋₁₀ alkylS(O)₁₋₂, C₁₋₁₀ heteroalkyl S(O)₁₋₂, (C₃₋₁₂) cycloalkylS(O)₁₋₂, heterocyclyl S(O)₁₋₂, heteroarylS(O)₁₋₂, and arylS(O)₁₋₂, wherein **R⁶** is each substituted with 0,1, 2, 3, or 4 **R⁷** substituents, and the other groups are provided in the general formula above, or as in the first through sixth embodiments.

In an eighth embodiment of the invention, each **R⁶** is independently selected from: halogen, heteroaryl C₀₋₁₀ alkyl, heterocyclyl C₀₋₁₀ alkyl, heterocyclyl(C₀₋₁₀)alkylaminocarbonyl, -NHSO₂(C₀₋₆ alkyl), -SO₂N(C₁₋₆ alkyl)₀₋₂, and C₁₋₁₀ alkylS(O)₁₋₂, wherein **R⁶** is each substituted with 0,1, 2, 3, or 4 **R⁷** substituents, and the other groups are provided in the general formula above, or as in the first through sixth embodiments.

In an ninth embodiment of the invention, each **R⁶** is independently selected from fluoro, chloro, bromo, iodo, oxazolidinyl, pyridazinyl, piperidinyl, pyridinyl, oxa-azabicyclo-[2.2.2]octanyl, 2-oxa-5-azabicyclo-[2.2.2]octanyl, imidazolinyl, ethylsulfonyl, pyrazolyl, azetidinylcarbamoyl, and -NS(O)₂H, wherein **R⁶** is each substituted with 0,1, or 2, **R⁷** substituents, and the other groups are provided in the general formula above, or as in the first through sixth embodiments.

In a tenth embodiment of the invention, each **R⁷** is independently selected from: halogen, C₁₋₁₀ alkyl, C₁₋₆ haloalkyl, amino C₀₋₁₀ alkyl, ((C₁₋₁₀)alkyl)₁₋₂amino, Oxo (=O), hydroxy, -(C₁₋₁₀ alkyl)OH, and C₁₋₁₀ alkoxy, and the other groups are provided in the general formula above, or as in the first through ninth embodiments.

In an eleventh embodiment of the invention, each **R⁷** is independently selected from: halogen, methyl, ethyl, propyl, *tert*-butyl, dimethylamino, oxo, and hydroxy, and the other groups are provided in the general formula above, or as in the first through ninth embodiments.

In an twelfth embodiment of the invention, each **R⁷** is independently selected from: halogen, methyl, oxo, and hydroxy, and the other groups are provided in the general formula abouve, or as in the first through ninth embodiments.

In a thirteenth embodiment of the invention, each **R²** is independently selected from: C₁₋₁₀ alkyl, C₃₋₁₂ cycloalkylC₀₋₁₀ alkyl, heteroarylC₀₋₁₀ alkyl, heterocyclylC₀₋₁₀ alkyl, C₃₋12 cycloalkyl C₀₋₁₀ alkylaminoC₀₋₁₀ alkyl, amino, and (C₁₋₁₀ alkyl)₁₋₂ aminoC₀₋₁₀ alkyl, wherein **R²** is each substituted with 0,1, 2, 3, or 4 **R⁵** substituents, and the other groups are provided in the general formula above, or as in the first through twelfth embodiments.

In a fourteenth embodiment of the invention, each **R²** is independently selected from: cyclopropylmethyl, dimethylamino, pyrrolidinyl, oxazepanyl, azetidinyl, azabicyclo[3.1.1]heptyl, decahydroisoquinolinyl, azaspiro[2.5]octanyl, 6-azaspiro[2.5]octanyl, azaspiro[4.5]decanyl, oxa-azaspiro[4.5]decyl, and oxa-azaspiro[3.5]nonyl, piperidinyl, wherein **R⁵** is each substituted with 0,1, 2, 3, or 4 **R⁵**, and the other groups are provided in the general formula above, or as in the first through twelfth embodiments.

In a fifteenth embodiment of the invention, each **R³** is independently selected from halogen, isopropyl, methyl, ethyl *tert*-butyl, difluoromethyl, cyclopropyl, and -Si(CH₃)₃, wherein each **R³** is independently substituted with 0,1, or 2, **R⁸** substituents, and the other groups are provided in the general formula above, or as in the first through fourteenth embodiments.

In a sixteenth embodiment of the invention, each **R³** is independently selected from fluoro, isopropyl, *tert*-butyl, cyclopropyl, and -Si(CH₃)₃, wherein each **R³** is independently substituted with 0,1, or 2, **R⁸** substituents, and the other groups are provided in the general formula above, or as in the first through fourteenth embodiments.

In a seventeenth embodiment of the invention, each **R⁸** is independently selected from: methyl, ethyl, propyl, butyl, methoxy, difluoromethyl, fluoro, and chloro, and the other groups are provided in the general formula above, or as in the first through sixteenth embodiments.

In an eighteenth embodiment of the invention, each **R⁸** is independently selected from: methyl, difluoromethyl, and fluoro, and the other groups are provided in the general formula above, or as in the first through sixteenth embodiments.

In a nineteenth embodiment of the invention, each **R⁴** is independently selected from fluoro, chloro and methyl, and the other groups are provided in the general formula above, or as in the first through eighteenth embodiments.

Non-limiting examples of the Compounds of Formula I include compounds 1-68 as set forth in the Examples below:
(S)-N-((R)-3-(4-hydroxypiperidin-1-yl)-1-(4-(2-oxooxazolidin-3-yl)phenyl)propyl)-7-(1-methylcyclopropyl)-5,6,7,8-tetrahydroacridine-2-carboxamide;
(S)-N-((R)-3-((S)-2-(hydroxymethyl)pyrrolidin-1-yl)-1-(4-(2-oxooxazolidin-3-yl)phenyl)propyl)-7-(1-methylcyclopropyl)-5,6,7,8-tetrahydroacridine-2-carboxamide;
(7S)-7-tert-butyl-N-{(1R)-3-(4-hydroxypiperidin-1-yl)-1-[4-(2-oxo-1,3-oxazolidin-3-yl)phenyl]propyl}-5,6,7,8-tetrahydroacridine-2-carboxamide;
(7S)-N-{(1R)-3-(4-hydroxypiperidin-1-yl)-1-[4-(2-oxo-1,3-oxazolidin-3-yl)phenyl]propyl}-7-(trimethylsilyl)-5,6,7,8-tetrahydroacridine-2-carboxamide;
(7S)-4,7-difluoro-N-{(1R)-3-(4-hydroxypiperidin-1-yl)-1-[4-(2-oxo-1,3-oxazolidin-3-yl)phenyl]propyl}-7-(1-methylethyl)-5,6,7,8-tetrahydroacridine-2-carboxamide;
(7S)-3,4,7-trifluoro-N-{(1R)-3-(4-hydroxypiperidin-1-yl)-1-[4-(2-oxo-1,3-oxazolidin-3-yl)phenyl]propyl}-7-(1-methylethyl)-5,6,7,8-tetrahydroacridine-2-carboxamide;
7-tert-butyl-3-fluoro-N-{(1R)-3-(4-hydroxypiperidin-1-yl)-1-[4-(2-oxo-1,3-oxazolidin-3-yl)phenyl]propyl}-5,6,7,8-tetrahydroacridine-2-carboxamide;
(S)-7-(tert-butyl)-4-chloro-N-((R)-3-(4-hydroxypiperidin-1-yl)-1-(4-(2-oxooxazolidin-3-yl)phenyl)propyl)-5,6,7,8-tetrahydroacridine-2-carboxamide;
7-(tert-butyl)-N-((R)-3-(4-hydroxypiperidin-1-yl)-1-(4-(2-oxooxazolidin-3-yl)phenyl)propyl)-4-methyl-5,6,7,8-tetrahydroacridine-2-carboxamide;
(7S)-4,7-difluoro-N-[(1R)-3-(4-hydroxypiperidin-1-yl)-1-(6-pyridazin-4-ylpyridin-3-yl)propyl]-7-(1-methylethyl)-5,6,7,8-tetrahydroacridine-2-carboxamide;
(7S)-4,7-difluoro-N-[(1R)-3-(4-hydroxy-2,2-dimethylpiperidin-1-yl)-1-(6-pyridazin-4-ylpyridin-3-yl)propyl]-7-(1-methylethyl)-5,6,7,8-tetrahydroacridine-2-carboxamide;
(7S)-4,7-difluoro-N-[(1R)-3-(4-hydroxy-4-methylpiperidin-1-yl)-1-(6-pyridazin-4-ylpyridin-3-yl)propyl]-7-(1-methylethyl)-5,6,7,8-tetrahydroacridine-2-carboxamide;
1-[(3R)-3-({[(7S)-4,7-difluoro-7-(1-methylethyl)-5,6,7,8-tetrahydroacridin-2-yl]carbonyl}amino)-3-(6-pyridazin-4-ylpyridin-3-yl)propyl]-4-hydroxypiperidine-4-carboxylic acid;
(7S)-4,7-difluoro-N-[(1R)-3-[6-hydroxy-6-(trifluoromethyl)-3-azabicyclo[3.1.1]hept-3-yl]-1-(6-pyridazin-4-ylpyridin-3-yl)propyl]-7-(1-methylethyl)-5,6,7,8-tetrahydroacridine-2-carboxamide;
(7S)-4,7-difluoro-N-[(1R)-3-(4a-hydroxyoctahydroisoquinolin-2(1H)-yl)-1-(6-pyridazin-4-ylpyridin-3-yl)propyl]-7-(1-methylethyl)-5,6,7,8-tetrahydroacridine-2-carboxamide;
(7S)-4,7-difluoro-7-(1-methylethyl)-N-[(1R)-3-(1-oxa-8-azaspiro[4.5]dec-8-yl)-1-(6-pyridazin-4-ylpyridin-3-yl)propyl]-5,6,7,8-tetrahydroacridine-2-carboxamide;
(7S)-4,7-difluoro-7-(1-methylethyl)-N-[(1R)-3-(1-oxa-7-azaspiro[3.5]non-7-yl)-1-(6-pyridazin-4-ylpyridin-3-yl)propyl]-5,6,7,8-tetrahydroacridine-2-carboxamide;
(7S)-4,7-difluoro-N-[(1R)-3-(4-methoxypiperidin-1-yl)-1-(6-pyridazin-4-ylpyridin-3-yl)propyl]-7-(1-methylethyl)-5,6,7,8-tetrahydroacridine-2-carboxamide;
(7S)-4,7-difluoro-N-[(1R)-3-[4-hydroxy-4-(hydroxymethyl)piperidin-1-yl]-1-(6-pyridazin-4-ylpyridin-3-yl)propyl]-7-(1-methylethyl)-5,6,7,8-tetrahydroacridine-2-carboxamide;
(7S)-4,7-difluoro-N-[(1R)-1-(5-fluoro-6-pyridazin-4-ylpyridin-3-yl)-3-(4-hydroxypiperidin-1-yl)propyl]-7-(1-methylethyl)-5,6,7,8-tetrahydroacridine-2-carboxamide;
(S)-N-((R)-3-((S)-2-(hydroxymethyl)pyrrolidin-1-yl)-1-(3-((1-methylazetidin-3-yl)carbamoyl)phenyl)propyl)-7-(1-methylcyclopropyl)-5,6,7,8-tetrahydroacridine-2-carboxamide;
(S)-N-((R)-3-((S)-2-(hydroxymethyl)pyrrolidin-1-yl)-1-(3-((1-methylazetidin-3-yl)carbamoyl)phenyl)propyl)-7-(1-(trifluoromethyl)cyclopropyl)-5,6,7,8-tetrahydroacridine-2-carboxamide;
((S)-7-(1-(difluoromethyl)cyclopropyl)-N-((R)-3-((S)-2-(hydroxymethyl)pyrrolidin-1-yl)-1-(3-((1-methylazetidin-3-yl)carbamoyl)phenyl)propyl)-5,6,7,8-tetrahydroacridine-2-carboxamide;
methyl 1-((R)-3-((S)-4,7-difluoro-7-isopropyl-5,6,7,8-tetrahydroacridine-2-carboxamido)-3-(4-(5-fluoro-6-hydroxypyridin-3-yl)phenyl)propyl)piperidine-4-carboxylate;
1-((R)-3-((S)-4,7-difluoro-7-isopropyl-5,6,7,8-tetrahydroacridine-2-carboxamido)-3-(4-(5-fluoro-6-hydroxypyridin-3-yl)phenyl)propyl)piperidine-4-carboxylic acid;
(S)-N-((R)-1-(6-((N,N-dimethylsulfamoyl)amino)pyridin-3-yl)-3-(4-hydroxypiperidin-1-yl)propyl)-4,7-difluoro-7-isopropyl-5,6,7,8-tetrahydroacridine-2-carboxamide;
(S)-N-((R)-1-(6-((N,N-dimethylsulfamoyl)amino)-5-fluoropyridin-3-yl)-3-(4-hydroxypiperidin-1-yl)propyl)-4,7-difluoro-7-isopropyl-5,6,7,8-tetrahydroacridine-2-carboxamide;
(S)-N-((R)-1-(6-(2,4-dioxoimidazolidin-1-yl)pyridin-3-yl)-3-(4-hydroxypiperidin-1-yl)propyl)-4,7-difluoro-7-isopropyl-5,6,7,8-tetrahydroacridine-2-carboxamide;
(7S)-4,7-difluoro-N-((1R)-3-(4-hydroxypiperidin-1-yl)-1-(6-(5-methyl-2,4-dioxoimidazolidin-1-yl)pyridin-3-yl)propyl)-7-isopropyl-5,6,7,8-tetrahydroacridine-2-carboxamide;
(S)-N-((R)-1-(6-(ethylsulfonyl)pyridin-3-yl)-3-(4-hydroxypiperidin-1-yl)propyl)-4,7-difluoro-7-isopropyl-5,6,7,8-tetrahydroacridine-2-carboxamide;
(S)-9-(hydroxymethyl)-N-((R)-3-(4-hydroxypiperidin-1-yl)-1-(4-(2-oxooxazolidin-3-yl)phenyl)propyl)-7-(1-methylcyclopropyl)-5,6,7,8-tetrahydroacridine-2-carboxamide;
(S)-N-((R)-1-(6-(1H-pyrazol-4-yl)pyridin-3-yl)-3-(4-hydroxypiperidin-1-yl)propyl)-4,7-difluoro-7-isopropyl-5,6,7,8-tetrahydroacridine-2-carboxamide;
(S)-4,7-difluoro-N-((R)-1-(6-((3R,4R)-3-fluoro-4-hydroxypiperidin-1-yl)pyridin-3-yl)-3-(4-hydroxypiperidin-1-yl)propyl)-7-isopropyl-5, 6,7, 8-tetrahydroacridine-2-carboxamide;
(7S)-N-((1R)-1-(6-(2-oxa-5-azabicyclo[2.2.2]octan-5-yl)pyridin-3-yl)-3-(4-hydroxypiperidin-1-yl)propyl)-4,7-difluoro-7-isopropyl-5,6,7,8-tetrahydroacridine-2-carboxamide;
(7S)-4,7-difluoro-7-isopropyl-N-[(1R)-1-(6-pyridazin-4-yl-3-pyridyl)-3-pyrrolidin-1-ium-1-yl-propyl]-6,8-dihydro-5H-acridine-2-carboxamide;2,2,2-trifluoroacetate;
(7S)-4,7-difluoro-7-isopropyl-N-[(1R)-1-(6-pyridazin-4-yl-3-pyridyl)-3-[(3S)-3-hydroxypiperidin-1-ium-1-yl]propyl]-6,8-dihydro-5H-acridine-2-carboxamide;2,2,2-trifluoroacetate;
dimethyl-[(3R)-3-(6-pyridazin-4-yl-3-pyridyl)-3-[[(7S)-4,7-difluoro-7-isopropyl-6,8-dihydro-5H-acridine-2-carbonyl]amino]propyl]ammonium;2,2,2-trifluoroacetate;
(7S)-4,7-difluoro-7-isopropyl-N-[(1R)-3-(2-oxa-8-azoniaspiro[4.5]decan-8-yl)-1-(6-pyridazin-4-yl-3-pyridyl)propyl]-6,8-dihydro-5H-acridine-2-carboxamide;2,2,2-trifluoroacetate;
(7S)-4,7-difluoro-7-isopropyl-N-[(1R)-3-[2-(hydroxymethyl)-6-azoniaspiro[2.5]octan-6-yl]-1-(6-pyridazin-4-yl-3-pyridyl)propyl]-6,8-dihydro-5H-acridine-2-carboxamide;2,2,2-trifluoroacetate;
(7S)-4,7-difluoro-7-isopropyl-N-[(1R)-1-(6-pyridazin-4-yl-3-pyridyl)-3-[(3R)-3-hydroxypiperidin-1-ium-1-yl]propyl]-6,8-dihydro-5H-acridine-2-carboxamide;2,2,2-trifluoroacetate;
(7S)-4,7-difluoro-7-isopropyl-N-[(1R)-3-[4-(1-hydroxyethyl)piperidin-1-ium-1-yl]-1-(6-pyridazin-4-yl-3-pyridyl)propyl]-6,8-dihydro-5H-acridine-2-carboxamide;2,2,2-trifluoroacetate;
(7S)-4,7-difluoro-7-isopropyl-N-[(1R)-3-(1,2,3,4,4a,5,6,7,8,8a-decahydroisoquinolin-2-ium-2-yl)-1-(6-pyridazin-4-yl-3-pyridyl)propyl]-6,8-dihydro-5H-acridine-2-carboxamide;2,2,2-trifluoroacetate;
(7S)-4,7-difluoro-7-isopropyl-N-[(1R)-3-[2-[2-(dimethylamino)ethyl]piperidin-1-ium-1-yl]-1-(6-pyridazin-4-yl-3-pyridyl)propyl]-6,8-dihydro-5H-acridine-2-carboxamide;2,2,2-trifluoroacetate;
(7S)-4,7-difluoro-7-isopropyl-N-[(1R)-3-(2-methylpiperidin-1-ium-1-yl)-1-(6-pyridazin-4-yl-3-pyridyl)propyl]-6,8-dihydro-5H-acridine-2-carboxamide;2,2,2-trifluoroacetate;
(7S)-4,7-difluoro-7-isopropyl-N-[(1R)-3-[4-(hydroxymethyl)piperidin-1-ium-1-yl]-1-(6-pyridazin-4-yl-3-pyridyl)propyl]-6,8-dihydro-5H-acridine-2-carboxamide;2,2,2-trifluoroacetate;
(7S)-4,7-difluoro-7-isopropyl-N-[(1R)-3-[4-(methoxymethyl)piperidin-1-ium-1-yl]-1-(6-pyridazin-4-yl-3-pyridyl)propyl]-6,8-dihydro-5H-acridine-2-carboxamide;2,2,2-trifluoroacetate;
(7S)-4,7-difluoro-7-isopropyl-N-[(1R)-3-[4-(1-hydroxy-1-methyl-ethyl)piperidin-1-ium-1-yl]-1-(6-pyridazin-4-yl-3-pyridyl)propyl]-6,8-dihydro-5H-acridine-2-carboxamide;2,2,2-trifluoroacetate;
(7S)-4,7-difluoro-7-isopropyl-N-[(1R)-1-(6-pyridazin-4-yl-3-pyridyl)-3-[(3S)-3-hydroxy-8-azaspiro[4.5]decan-8-yl]propyl]-6,8-dihydro-5H-acridine-2-carboxamide;
(7S)-4,7-difluoro-7-isopropyl-N-[(1R)-1-(6-pyridazin-4-yl-3-pyridyl)-3-[(3R)-3-hydroxy-8-azaspiro[4.5]decan-8-yl]propyl]-6,8-dihydro-5H-acridine-2-carboxamide;
(7S)-4,7-difluoro-7-isopropyl-N-[(1R)-3-(2-oxa-7-azaspiro[3.5]nonan-7-yl)-1-(6-pyridazin-4-yl-3-pyridyl)propyl]-6,8-dihydro-5H-acridine-2-carboxamide;
(7S)-4,7-difluoro-7-isopropyl-N-[(1R)-3-[4-(3-methoxypropyl)-1-piperidyl]-1-(6-pyridazin-4-yl-3-pyridyl)propyl]-6,8-dihydro-5H-acridine-2-carboxamide;
(7S)-4,7-difluoro-7-isopropyl-N-[(1R)-3-(4a-hydroxy-1,3,4,5,6,7,8,8a-octahydroisoquinolin-2-yl)-1-(6-pyridazin-4-yl-3-pyridyl)propyl]-6,8-dihydro-5H-acridine-2-carboxamide;
(7S)-4,7-difluoro-7-isopropyl-N-[(1R)-3-[2-(2-hydroxyethyl)-1-piperidyl]-1-(6-pyridazin-4-yl-3-pyridyl)propyl]-6,8-dihydro-5H-acridine-2-carboxamide;
(7S)-4,7-difluoro-7-isopropyl-N-[(1R)-3-[4-[hydroxy-[1-(hydroxymethyl)cyclopropyl]methyl]-1-piperidyl]-1-(6-pyridazin-4-yl-3-pyridyl)propyl]-6,8-dihydro-5H-acridine-2-carboxamide;
(7S)-4,7-difluoro-7-isopropyl-N-[(1R)-3-(1-oxa-8-azaspiro[4.5]decan-8-yl)-1-(6-pyridazin-4-yl-3-pyridyl)propyl]-6,8-dihydro-5H-acridine-2-carboxamide;
(7S)-4,7-difluoro-7-isopropyl-N-[(1R)-3-(4-isopropyl-1-piperidyl)-1-(6-pyridazin-4-yl-3-pyridyl)propyl]-6,8-dihydro-5H-acridine-2-carboxamide;
(7S)-4,7-difluoro-7-isopropyl-N-[(1R)-3-(1,4-oxazepan-4-yl)-1-(6-pyridazin-4-yl-3-pyridyl)propyl]-6,8-dihydro-5H-acridine-2-carboxamide;
(7S)-4,7-difluoro-7-isopropyl-N-[(1R)-3-[2-(hydroxymethyl)-1-piperidyl]-1-(6-pyridazin-4-yl-3-pyridyl)propyl]-6,8-dihydro-5H-acridine-2-carboxamide;
(7S)-4,7-difluoro-7-isopropyl-N-[(1R)-3-[2-[(dimethylamino)methyl]-1-piperidyl]-1-(6-pyridazin-4-yl-3-pyridyl)propyl]-6,8-dihydro-5H-acridine-2-carboxamide;
(7S)-4,7-difluoro-7-isopropyl-N-[(1R)-3-[4-(morpholinomethyl)-1-piperidyl]-1-(6-pyridazin-4-yl-3-pyridyl)propyl]-6,8-dihydro-5H-acridine-2-carboxamide;
(7S)-4,7-difluoro-7-isopropyl-N-[(1R)-1-(6-pyridazin-4-yl-3-pyridyl)-3-[(2R,6S)-2,6-dimethyl-1-piperidyl]propyl]-6,8-dihydro-5H-acridine-2-carboxamide;
(7S)-4,7-difluoro-7-isopropyl-N-[(1R)-3-(1-oxa-7-azaspiro[3.5]nonan-7-yl)-1-(6-pyridazin-4-yl-3-pyridyl)propyl]-6,8-dihydro-5H-acridine-2-carboxamide;
(7S)-4,7-difluoro-7-isopropyl-N-[(1R)-3-(3-hydroxyazetidin-1-ium-1-yl)-1-(6-pyridazin-4-yl-3-pyridyl)propyl]-6,8-dihydro-5H-acridine-2-carboxamide;2,2,2-trifluoroacetate;
(7S)-4,7-difluoro-7-isopropyl-N-[(1R)-1-(6-pyridazin-4-yl-3-pyridyl)-3-[4-(trifluoromethyl)-1-piperidyl]propyl]-6,8-dihydro-5H-acridine-2-carboxamide;
methyl 2-[1-[(3R)-3-[3-[(1-methylazetidin-3-yl)carbamoyl]phenyl]-3-[[(7S)-7-(1-methylcyclopropyl)-5,6,7,8-tetrahydroacridine-2-carbonyl]amino]propyl]-4-piperidyl]acetate;
2-[1-[(3R)-3-[3-[(1-methylazetidin-3-yl)carbamoyl]phenyl]-3-[[(7S)-7-(1-methylcyclopropyl)-5,6,7,8-tetrahydroacridine-2-carbonyl]amino]propyl]-4-piperidyl]acetic acid;
(7S)-7-(1-methylcyclopropyl)-N-[(1R)-3-(dimethylamino)-1-[3-[(1-methylazetidin-3-yl)carbamoyl]phenyl]propyl]-5,6,7,8-tetrahydroacridine-2-carboxamide; and
(7S)-7-(1-methylcyclopropyl)-N-[(1R)-3-(4-hydroxy-1-piperidyl)-1-[3-[(1-methylazetidin-1-ium-3-yl)carbamoyl]phenyl]propyl]-5,6,7,8-tetrahydroacridine-2-carboxamide;2,2,2-trifluoroacetate.

The present invention encompasses for each of the various embodiments of the compounds of the invention described herein, including those of Formula I and the various embodiments thereof and the compounds of the examples, all forms of the compounds such as, for example, any solvates, hydrates, stereoisomers, and tautomers of said compounds and of any pharmaceutically acceptable salts thereof. Additionally, in the examples described herein, the compounds of the invention may be depicted in the salt form. In such cases, it is to be understood that the compounds of the invention include the free acid or free base forms of such salts, and any pharmaceutically acceptable salt of said free acid or free base forms.

In addition, when a compound of the invention contains both a basic moiety, such as, but not limited to an aliphatic primary, secondary, tertiary or cyclic amine, an aromatic or heteroaryl amine, pyridine or imidazole, and an acidic moiety, such as, but not limited to tetrazole or carboxylic acid, zwitterions ("inner salts") may be formed and are included within the term "salt(s)" as used herein. It is undertood that certain compounds of the invention may exist in zwitterionic form, having both anionic and cationic centers within the same compound and a net neutral charge. Such zwitterions are included within the invention.

Other embodiments of the present invention include the following:
(a) A pharmaceutical composition comprising a compound of formula I or a pharmaceutically acceptable salt thereof and a pharmaceutically acceptable carrier.
(b) The pharmaceutical composition of (a), further comprising a second therapeutic agent.
(c) A pharmaceutical combination that is (i) a compound of formula I or a pharmaceutically acceptable salt thereof, and (ii) a second therapeutic agent wherein the compound of formula I and the second therapeutic agent are each employed in an amount that renders the combination effective for treatment or prophylaxis of cardiometabolic diseases, kidney disease, or diabetes.
(d) A compound of formula I, or a pharmaceutically acceptable salt thereof, for use in therapy.
(e) A compound of formula I, or a pharmaceutically acceptable salt thereof, for use in the treatment of cardiometabolic diseases, kidney disease, or diabetes.
(f) A use of a compound of formula I or a pharmaceutically acceptable salt thereof, in the preparation of a medicament for modulating NPRA activity in a subject in need thereof.
(g) A use of a compound of formula I or a pharmaceutically acceptable salt thereof, in the preparation of a medicament for treatment or prophylaxis of cardiometabolic diseases, kidney disease, or diabetes in a subject in need thereof.
(h) A use of a compound of formula I or a pharmaceutically acceptable salt thereof, in the preparation of a medicament for treatment of cardiometabolic diseases, kidney disease, or diabetes in a subject in need thereof.
(i) A method of treating cognitive impairments associated with cardiometabolic diseases, kidney disease, or diabetes, and/or reducing the likelihood or severity of symptoms of cardiometabolic diseases, kidney disease, or diabetes, in a subject in need thereof, which comprises administering to the subject an effective amount of a compound of formula I or a pharmaceutically acceptable salt thereof.
(j) The method of (f), wherein the compound of formula I or a pharmaceutically acceptable salt thereof, is administered in combination with an effective amount of at least one second therapeutic agent.
(k) A method of modulating NPRA activity in a subject in need thereof, which comprises administering to the subject the pharmaceutical composition of (a) or (b), or the combination of (c).
(l) A method of treating cognitive impairments associated with cardiometabolic diseases, kidney disease, or diabetes and/or reducing the likelihood or severity of symptoms of cognitive impairments associated with cardiometabolic diseases, kidney disease, or diabetes in a subject in need thereof, which comprises administering to the subject the pharmaceutical composition of (a) or (b), or the combination of (c).

In the embodiments of the compounds and salts provided above, it is to be understood that each embodiment may be combined with one or more other embodiments, to the extent that such a combination provides a stable compound or salt and is consistent with the description of the embodiments. It is further to be understood that the embodiments of compositions and methods provided as (a) through (l) above are understood to include all embodiments of the compounds and/or salts, including such embodiments as result from combinations of embodiments.

Additional embodiments of the invention include the pharmaceutical compositions, combinations, uses and methods set forth in (a) through (l) above, wherein the compound of the present invention employed therein is a compound of one of the embodiments, aspects, classes, sub-classes, or features of the compounds described above. In all of these embodiments, the compound may optionally be used in the form of a pharmaceutically acceptable salt as appropriate.

The present invention also includes a compound of the present invention for use (i) in, (ii) as a medicament for, or (iii) in the preparation of a medicament for: (a) preventing or treating cardiometabolic diseases, kidney disease, or diabetes or (c) use in medicine. In these uses, the compounds of the present invention can optionally be employed in combination with one or more second therapeutic agents.

Additional embodiments of the invention include the pharmaceutical compositions, combinations and methods set forth in (a)-(l) above and the uses set forth in the preceding paragraph, wherein the compound of the present invention employed therein is a compound of one of the embodiments, aspects, classes, sub-classes, or features of the compounds described above. In all of these embodiments, the compound may optionally be used in the form of a pharmaceutically acceptable salt or hydrate as appropriate.

It is further to be understood that the embodiments of compositions and methods provided as (a) through (l) above are understood to include all embodiments of the compounds, including such embodiments as result from combinations of embodiments.

The present invention also relates to processes for the preparation of the compounds of Formula I which are described in the following and by which the compounds of the invention are obtainable.

Exemplifying the invention is the use of the compounds disclosed in the Examples and herein.

The compounds of Formula I according to the invention effect an increase of the cGMP concentration via the activation of NPRA, and they are therefore useful agents for the therapy and prophylaxis of disorders which are associated with a low or decreased cGMP level or which are caused thereby, or for whose therapy or prophylaxis an increase of the present cGMP level is desired. The activation of NPRA by the compounds of the Formula I can be examined, for example, in the activity assay described below.

Disorders and pathological conditions which are associated with a low cGMP level or in which an increase of the cGMP level is desired and for whose therapy and prophylaxis it is possible to use compounds of the Formula I are, for example, cardiovascular diseases, such as endothelial dysfunction, diastolic dysfunction, atherosclerosis, hypertension, heart failure, pulmonary hypertension, stable and unstable angina pectoris, thromboses, restenosis, myocardial infarction, strokes, cardiac insufficiency or pulmonary hypertonia, or, for example, erectile dysfunction, asthma bronchial, chronic kidney insufficiency and diabetes. Compounds of the Formula I can additionally be used in the therapy of cirrhosis of the liver and also for improving a restricted memory performance or ability to learn.

The invention also relates to the use of compounds of the invention for the preparation of a medicament for the treatment and/or prophylaxis of the above-mentioned diseases.

The compounds of the Formula I and their physiologically acceptable salts can be administered to animals, preferably to mammals, and in particular to humans, as pharmaceuticals by themselves, in mixtures with one another or in the form of pharmaceutical preparations. A subject of the present invention therefore also are the compounds of the Formula I and their physiologically acceptable salts for use as pharmaceuticals, their use for activating NPRA, for normalizing a disturbed cGMP balance and in particular their use in the therapy and prophylaxis of the abovementioned syndromes as well as their use for preparing medicaments for these purposes.

Furthermore, a subject of the present invention are pharmaceutical preparations (or pharmaceutical compositions) which comprise as active component an effective dose of at least one compound of the Formula I and/or a physiologically acceptable salt thereof and a customary pharmaceutically acceptable carrier, i.e., one or more pharmaceutically acceptable carrier substances and/or additives.

Thus, a subject of the invention are, for example, said compound and its physiologically acceptable salts for use as a pharmaceutical, pharmaceutical preparations which comprise as active component an effective dose of said compound and/or a physiologically acceptable salt thereof and a customary pharmaceutically acceptable carrier, and the uses of said compound and/or a physiologically acceptable salt thereof in the therapy or prophylaxis of the abovementioned syndromes as well as their use for preparing medicaments for these purposes.

The pharmaceuticals according to the invention can be administered orally, for example in the form of pills, tablets, lacquered tablets, sugar-coated tablets, granules, hard and soft gelatin capsules, aqueous, alcoholic or oily solutions, syrups, emulsions or suspensions, or rectally, for example in the form of suppositories. Administration can also be carried out parenterally, for example subcutaneously, intramuscularly or intravenously in the form of solutions for injection or infusion. Other suitable administration forms are, for example, percutaneous or topical administration, for example in the form of ointments, tinctures, sprays or transdermal therapeutic systems, or the inhalative administration in the form of nasal sprays or aerosol mixtures, or, for example, microcapsules, implants or rods. The preferred administration form depends, for example, on the disease to be treated and on its severity.

For the production of pills, tablets, sugar-coated tablets and hard gelatin capsules it is possible to use, for example, lactose, starch, for example maize starch, or starch derivatives, talc, stearic acid or its salts, etc. Carriers for soft gelatin capsules and suppositories are, for example, fats, waxes, semisolid and liquid polyols, natural or hardened oils, etc. Suitable carriers for the preparation of solutions, for example of solutions for injection, or of emulsions or syrups are, for example, water, physiologically sodium chloride solution, alcohols such as ethanol, glycerol, polyols, sucrose, invert sugar, glucose, mannitol, vegetable oils, etc. It is also possible to lyophilize the compounds of the Formula I and their physiologically acceptable salts and to use the resulting lyophilisates, for example, for preparing preparations for injection or infusion. Suitable carriers for microcapsules, implants or rods are, for example, copolymers of glycolic acid and lactic acid.

Besides the active compounds and carriers, the pharmaceutical preparations can also contain customary additives, for example fillers, disintegrants, binders, lubricants, wetting agents, stabilizers, emulsifiers, dispersants, preservatives, sweeteners, colorants, flavorings, aromatizers, thickeners, diluents, buffer substances, solvents, solubilizers, agents for achieving a depot effect, salts for altering the osmotic pressure, coating agents or antioxidants.

Compositions containing a compound of Formula I described herein will provide immediate release of the drug after administration as that term is understood in the art, but the compositions can be formulated to modify the release rate to achieve controlled, extended or delayed release and the like (collectively referred to herein as controlled release). Controlled release dosage forms can be prepared by methods known to those skilled in the art, for example, by granule or tablet enteric coatings or by admixture of a controlled release matrix component in the composition. For example, a fixed-dose combination composition containing a compound of Formula I admixed with one or more additional pharmaceutically active agents may have an immediate release or controlled release tablet dissolution profile.

The compounds of the Formula I activate Natriuretic Peptide Receptor A (NPRA). Due to this property, apart from use as pharmaceutically active compounds in human medicine and veterinary medicine, they can also be employed as a scientific tool or as aid for biochemical investigations in which such an effect on cGMP is intended, and also for diagnostic purposes, for example in the in vitro diagnosis of cell samples or tissue samples. The compounds of the Formula I and salts thereof can furthermore be employed, as already mentioned above, as intermediates for the preparation of other pharmaceutically active compounds.

The above-mentioned compounds of Formula I are also of use in combination with other pharmacologically active compounds. The additional active agent (or agents) is intended to mean a medicinal compound that is different from the compound of Formula I, and which is a pharmaceutically active agent (or agents) that is active in the body, including pro-drugs, for example esterified forms, that convert to pharmaceutically active form after administration, and also includes free-acid, free-base and pharmaceutically acceptable salts of said additional active agents when such forms are sold commercially or are otherwise chemically possible. Generally, any suitable additional active agent or agents, including but not limited to anti-hypertensive agents, additional diuretics, anti-atherosclerotic agents such as a lipid modifying compound, antidiabetic agents and/or anti-obesity agents may be used in any combination with the compound of Formula I in a single dosage formulation (a fixed dose drug combination), or may be administered to the patient in one or more separate dosage formulations which allows for concurrent or sequential administration of the active agents (co-administration of the separate active agents).

When administered to a subject, the Compounds of Formula I may be administered as a component of a composition that comprises a pharmaceutically acceptable carrier or vehicle. The present invention provides pharmaceutical compositions comprising an effective amount of at least one Compound of Formula I and a pharmaceutically acceptable carrier. In the pharmaceutical compositions and methods of the present invention, the active ingredients will typically be administered in admixture with suitable carrier materials suitably selected with respect to the intended form of administration, *i.e.,* oral tablets, capsules (either solid-filled, semi-solid filled or liquid filled), powders for constitution, oral gels, elixirs, dispersible granules, syrups, suspensions, and the like, and consistent with conventional pharmaceutical practices. For example, for oral administration in the form of tablets or capsules, the active drug component may be combined with any oral non-toxic pharmaceutically acceptable inert carrier, such as lactose, starch, sucrose, cellulose, magnesium stearate, dicalcium phosphate, calcium sulfate, talc, mannitol, ethyl alcohol (liquid forms) and the like. Solid form preparations include powders, tablets, dispersible granules, capsules, cachets and suppositories. Powders and tablets may be comprised of from about 0.5 to about 95 percent inventive composition. Tablets, powders, cachets and capsules may be used as solid dosage forms suitable for oral administration.

Moreover, when desired or needed, suitable binders, lubricants, disintegrating agents and coloring agents may also be incorporated in the mixture. Suitable binders include starch, gelatin, natural sugars, corn sweeteners, natural and synthetic gums such as acacia, sodium alginate, carboxymethylcellulose, polyethylene glycol and waxes. Among the lubricants there may be mentioned for use in these dosage forms, boric acid, sodium benzoate, sodium acetate, sodium chloride, and the like. Disintegrants include starch, methylcellulose, guar gum, and the like. Sweetening and flavoring agents and preservatives may also be included where appropriate.

Liquid form preparations include solutions, suspensions and emulsions and may include water or water-propylene glycol solutions for parenteral injection.

Liquid form preparations may also include solutions for intranasal administration.

Also included are solid form preparations which are intended to be converted, shortly before use, to liquid form preparations for either oral or parenteral administration. Such liquid forms include solutions, suspensions and emulsions.

For preparing suppositories, a low melting wax such as a mixture of fatty acid glycerides or cocoa butter is first melted, and the active ingredient is dispersed homogeneously therein as by stirring. The molten homogeneous mixture is then poured into convenient sized molds, allowed to cool and thereby solidify.

Additionally, the compositions of the present invention may be formulated in sustained release form to provide the rate controlled release of any one or more of the components or active ingredients to optimize therapeutic effects, *i.e.,* antiviral activity and the like. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

In one embodiment, the one or more Compounds of Formula I are administered orally.

In another embodiment, the one or more Compounds of Formula I are administered intravenously.

In one embodiment, a pharmaceutical preparation comprising at least one Compound of Formula I is in unit dosage form. In such form, the preparation is subdivided into unit doses containing effective amounts of the active components.

Compositions may be prepared according to conventional mixing, granulating or coating methods, respectively, and the present compositions can contain, in one embodiment, from about 0.1% to about 99% of the Compound(s) of Formula I by weight or volume. In various embodiments, the present compositions can contain, in one embodiment, from about 1% to about 70% or from about 5% to about 60% of the Compound(s) of Formula I by weight or volume.

The compounds of Formula I may be administered orally in a dosage range of 0.001 to 1000 mg/kg of mammal (e.g., human) body weight per day in a single dose or in divided doses. One dosage range is 0.01 to 500 mg/kg body weight per day orally in a single dose or in divided doses. Another dosage range is 0.1 to 100 mg/kg body weight per day orally in single or divided doses. For oral administration, the compositions may be provided in the form of tablets or capsules containing 1.0 to 500 milligrams of the active ingredient, particularly 1, 5, 10, 15, 20, 25, 50, 75, 100, 150, 200, 250, 300, 400, and 500 milligrams of the active ingredient for the symptomatic adjustment of the dosage to the subject to be treated. The specific dose level and frequency of dosage for any particular subject may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

For convenience, the total daily dosage may be divided and administered in portions during the day if desired. In one embodiment, the daily dosage is administered in one portion. In another embodiment, the total daily dosage is administered in two divided doses over a 24 hour period. In another embodiment, the total daily dosage is administered in three divided doses over a 24 hour period. In still another embodiment, the total daily dosage is administered in four divided doses over a 24 hour period.

The unit dosages of the Compounds of Formula I may be administered at varying frequencies. In one embodiment, a unit dosage of a Compound of Formula I may be administered once daily. In another embodiment, a unit dosage of a Compound of Formula I may be administered twice weekly. In another embodiment, a unit dosage of a Compound of Formula I may be administered once weekly. In still another embodiment, a unit dosage of a Compound of Formula I may be administered once biweekly. In another embodiment, a unit dosage of a Compound of Formula I may be administered once monthly. In yet another embodiment, a unit dosage of a Compound of Formula I may be administered once bimonthly. In another embodiment, a unit dosage of a Compound of Formula I may be administered once every 3 months. In a further embodiment, a unit dosage of a Compound of Formula I may be administered once every 6 months. In another embodiment, a unit dosage of a Compound of Formula I may be administered once yearly.

The amount and frequency of administration of the Compounds of Formula I will be regulated according to the judgment of the attending clinician considering such factors as age, condition and size of the subject as well as severity of the symptoms being treated. The compositions of the invention can further comprise one or more additional therapeutic agents, selected from those listed above herein.

The present invention is not to be limited by the specific embodiments disclosed in the examples that are intended as illustrations of a few aspects of the invention. Indeed, various modifications of the invention in addition to those shown and described herein will become apparent to those skilled in the art and are intended to fall within the scope of the appended claims.

The compounds of Formula I are of use in combination with other pharmacologically active compounds comprising angiotensin converting enzyme inhibitors (e.g, alacepril, benazepril, captopril, ceronapril, cilazapril, delapril, enalapril, enalaprilat, fosinopril, imidapril, lisinopril, moveltipril, perindopril, quinapril, ramipril, spirapril, temocapril, or trandolapril), angiotensin II receptor antagonists (e.g., losartan, valsartan, candesartan, olmesartan, telmesartan) neutral endopeptidase inhibitors (e.g., thiorphan and phosphoramidon), aldosterone antagonists, renin inhibitors (e.g. urea derivatives of di- and tri-peptides (See U.S. Pat. No. 5,116,835), amino acids and derivatives (U.S. Patents 5,095,119 and 5,104,869), amino acid chains linked by non-peptidic bonds (U.S. Patent 5,114,937), di- and tri-peptide derivatives (U.S. Patent 5,106,835), peptidyl amino diols (U.S. Patents 5,063,208 and 4,845,079) and peptidyl beta-aminoacyl aminodiol carbamates (U.S. Patent 5,089,471); also, a variety of other peptide analogs as disclosed in the following U.S. Patents 5,071,837; 5,064,965; 5,063,207; 5,036,054; 5,036,053; 5,034,512 and 4,894,437, and small molecule renin inhibitors (including diol sulfonamides and sulfinyls (U.S. Patent 5,098,924), N-morpholino derivatives (U.S. Patent 5,055,466), N-heterocyclic alcohols (U.S. Patent 4,885,292) and pyrolimidazolones (U.S. Patent 5,075,451); also, pepstatin derivatives (U.S. Patent 4,980,283) and fluoro- and chloro-derivatives of statone-containing peptides (U.S. Patent 5,066,643), enalkrein, RO 42-5892, A 65317, CP 80794, ES 1005, ES 8891, SQ 34017, aliskiren (2(S),4(S),5(S),7(S)-N-(2-carbamoyl-2-methylpropyl)-5-amino-4-hydroxy-2,7-diisopropyl-8-[4-methoxy-3-(3-methoxypropoxy)-phenyl]-octanamid hemifumarate) SPP600, SPP630 and SPP635), endothelin receptor antagonists, vasodilators, calcium channel blockers (e.g., amlodipine, nifedipine, veraparmil, diltiazem, gallopamil, niludipine, nimodipins, nicardipine), potassium channel activators (e.g., nicorandil, pinacidil, cromakalim, minoxidil, aprilkalim, loprazolam), diuretics (e.g., hydrochlorothiazide), sympatholitics, beta-adrenergic blocking drugs (e.g., propranolol, atenolol, bisoprolol, carvedilol, metoprolol, or metoprolol tartate), alpha adrenergic blocking drugs (e.g., doxazocin, prazocin or alpha methyldopa) central alpha adrenergic agonists, peripheral vasodilators (e.g. hydralazine), lipid lowering agents (e.g., simvastatin, lovastatin, ezetamibe, atorvastatin, pravastatin), metabolic altering agents including insulin sensitizing agents and related compounds (e.g., muraglitazar, glipizide, metformin, rosiglitazone) or with other drugs beneficial for the prevention or the treatment of the above-mentioned diseases including nitroprusside and diazoxide.

Examples of other active ingredients that may be administered in combination with a compound of Formula I, and either administered separately or in the same pharmaceutical composition, include, but are not limited to:
(a) PPAR gamma agonists and partial agonists, including both glitazones and non-glitazones (e.g. troglitazone, pioglitazone, englitazone, MCC-555, rosiglitazone, balaglitazone, netoglitazone, T-131, LY-300512, LY-818, and compounds disclosed in WO02/08188, WO2004/020408, and WO2004/020409.
(b) biguanides, such as metformin and phenformin;
(c) protein tyrosine phosphatase-1B (PTP-1B) inhibitors;
(d) dipeptidyl peptidase-IV (DPP-4) inhibitors, such as sitagliptin, saxagliptin, vildagliptin, and alogliptin;
(e) insulin or insulin mimetics;
(f) sulfonylureas such as tolbutamide, glimepiride, glipizide, and related materials;
(g) α-glucosidase inhibitors (such as acarbose);
(h) agents which improve a patient's lipid profile, such as (i) HMG-CoA reductase inhibitors (lovastatin, simvastatin, rosuvastatin, pravastatin, fluvastatin, atorvastatin, rivastatin, itavastatin, ZD-4522 and other statins), (ii) bile acid sequestrants (cholestyramine, colestipol, and dialkylaminoalkyl derivatives of a cross-linked dextran), (iii) niacin receptor agonists, nicotinyl alcohol, nicotinic acid, or a salt thereof, (iv) PPARα agonists, such as fenofibric acid derivatives (gemfibrozil, clofibrate, fenofibrate and bezafibrate), (v) cholesterol absorption inhibitors, such as ezetimibe, (vi) acyl CoA:cholesterol acyltransferase (ACAT) inhibitors, such as avasimibe, (vii) CETP inhibitors, such as torcetrapib, and (viii) phenolic antioxidants, such as probucol;
(i) PPARα/γ dual agonists, such as muraglitazar, tesaglitazar, farglitazar, and JT-501;
(j) PPARδ agonists, such as those disclosed in WO97/28149;
(k) anti-obesity compounds, such as fenfluramine, dexfenfluramine, phentiramine, subitramine, orlistat, neuropeptide Y Y5 inhibitors, MC4R agonists, cannabinoid receptor 1 (CB-1) antagonists/inverse agonists (e.g., rimonabant and taranabant), and β₃ adrenergic receptor agonists;
(l) ileal bile acid transporter inhibitors;
(m) agents intended for use in inflammatory conditions, such as aspirin, non-steroidal anti-inflammatory drugs, glucocorticoids, azulfidine, and cyclooxygenase-2 (Cox-2) selective inhibitors;
(n) glucagon receptor antagonists;
(o) GLP-1;
(p) GIP-1;
(q) GLP-1 analogs and derivatives, such as exendins, (e.g., exenatide and liruglatide), and
(r) 11β-hydroxysteroid dehydrogenase-1 (HSD-1) inhibitors.

Other examples of additional pharmacologically active agents that may be administered in combination with a compound of Formula I include but are not limited to thiazide-like diuretics, e.g., hydrochlorothiazide (HCTZ or HCT); angiotensin converting enzyme inhibitors (e.g, alacepril, benazepril, captopril, ceronapril, cilazapril, delapril, enalapril, enalaprilat, fosinopril, imidapril, lisinopril, moveltipril, perindopril, quinapril, ramipril, spirapril, temocapril, or trandolapril); dual inhibitors of angiotensin converting enzyme (ACE) and neutral endopeptidase (NEP) such as omapatrilat, sampatrilat and fasidotril; angiotensin II receptor antagonists, also known as angiotensin receptor blockers or ARBs, which may be in free-base, free-acid, salt or pro-drug form, such as azilsartan, e.g., azilsartan medoxomil potassium (EDARBI^{®}), candesartan, e.g., candesartan cilexetil (ATACAND^{®}), eprosartan, e.g., eprosartan mesylate (TEVETAN^{®}), irbesartan (AVAPRO^{®}), losartan, e.g., losartan potassium (COZAAR^{®}), olmesartan, e.g, olmesartan medoximil (BENICAR^{®}), telmisartan (MICARDIS^{®}), valsartan (DIOVAN^{®}), and any of these drugs used in combination with a thiazide-like diuretic such as hydrochlorothiazide (e.g., HYZAAR^{®}, DIOVAN HCT^{®}, ATACAND HCT^{®}), etc.); potassium sparing diuretics such as amiloride HCl, spironolactone, epleranone, triamterene, each with or without HCTZ; carbonic anhydrase inhibitors, such as acetazolamide; neutral endopeptidase inhibitors (e.g., thiorphan and phosphoramidon); aldosterone antagonists; aldosterone synthase inhibitors; renin inhibitors (e.g. urea derivatives of di- and tri-peptides (See U.S. Pat. No. 5,116,835), amino acids and derivatives (U.S. Patents 5,095,119 and 5,104,869), amino acid chains linked by non-peptidic bonds (U.S. Patent 5,114,937), di- and tri-peptide derivatives (U.S. Patent 5,106,835), peptidyl amino diols (U.S. Patents 5,063,208 and 4,845,079) and peptidyl beta-aminoacyl aminodiol carbamates (U.S. Patent 5,089,471); also, a variety of other peptide analogs as disclosed in the following U.S. Patents 5,071,837; 5,064,965; 5,063,207; 5,036,054; 5,036,053; 5,034,512 and 4,894,437, and small molecule renin inhibitors (including diol sulfonamides and sulfinyls (U.S. Patent 5,098,924), N-morpholino derivatives (U.S. Patent 5,055,466), N-heterocyclic alcohols (U.S. Patent 4,885,292) and pyrolimidazolones (U.S. Patent 5,075,451); also, pepstatin derivatives (U.S. Patent 4,980,283) and fluoro- and chloro-derivatives of statone-containing peptides (U.S. Patent 5,066,643); enalkrein; RO 42-5892; A 65317; CP 80794; ES 1005; ES 8891; SQ 34017; aliskiren (2(*S*),4(*S*),5(*S*),7(*S*)-N-(2-carbamoyl-2-methylpropyl)-5-amino-4-hydroxy-2,7-diisopropyl-8-[4-methoxy-3-(3-methoxypropoxy)-phenyl]-octanamid hemifumarate) SPP600, SPP630 and SPP635); endothelin receptor antagonists; vasodilators (e.g. nitroprusside); calcium channel blockers (e.g., amlodipine, nifedipine, verapamil, diltiazem, , felodipine, gallopamil, niludipine, nimodipine, nicardipine, bepridil, nisoldipine); potassium channel activators (e.g., nicorandil, pinacidil, cromakalim, minoxidil, aprilkalim, loprazolam); sympatholitics; beta-adrenergic blocking drugs (e.g., acebutolol, atenolol, betaxolol, bisoprolol, carvedilol, metoprolol, metoprolol tartate, nadolol, propranolol, sotalol, timolol); alpha adrenergic blocking drugs (e.g., doxazocin, prazocin or alpha methyldopa); central alpha adrenergic agonists; peripheral vasodilators (e.g. hydralazine); nitrates or nitric oxide donating compounds, e.g. isosorbide mononitrate; lipid lowering agents, e.g., HMG-CoA reductase inhibitors such as simvastatin and lovastatin which are marketed as ZOCOR^{®} and MEVACOR^{®} in lactone pro-drug form and function as inhibitors after administration, and pharmaceutically acceptable salts of dihydroxy open ring acid HMG-CoA reductase inhibitors such as atorvastatin (particularly the calcium salt sold in LIPITOR^{®}), rosuvastatin (particularly the calcium salt sold in CRESTOR^{®}), pravastatin (particularly the sodium salt sold in PRAVACHOL^{®}), and fluvastatin (particularly the sodium salt sold in LESCOL^{®}); a cholesterol absorption inhibitor such as ezetimibe (ZETIA^{®}), and ezetimibe in combination with any other lipid lowering agents such as the HMG-CoA reductase inhibitors noted above and particularly with simvastatin (VYTORIN^{®}) or with atorvastatin calcium; niacin in immediate-release or controlled release forms, and particularly niacin in combination with a DP antagonist such as laropiprant and/or with an HMG-CoA reductase inhibitor; niacin receptor agonists such as acipimox and acifran, as well as niacin receptor partial agonists; metabolic altering agents including insulin sensitizing agents and related compounds for the treatment of diabetes such as biguanides (e.g., metformin), meglitinides (e.g., repaglinide, nateglinide), sulfonylureas (e.g., chlorpropamide, glimepiride, glipizide, glyburide, tolazamide, tolbutamide), thiazolidinediones also referred to as glitazones (e.g., pioglitazone, rosiglitazone), alpha glucosidase inhibitors (e.g., acarbose, miglitol), dipeptidyl peptidase inhibitors, (e.g., sitagliptin (JANUVIA^{®}), alogliptin, vildagliptin, saxagliptin, linagliptin, dutogliptin, gemigliptin), ergot alkaloids (e.g., bromocriptine), combination medications such as JANUMET^{®} (sitagliptin with metformin), and injectable diabetes medications such as exenatide and pramlintide acetate; phosphodiesterase-5 (PDE5) inhibitors such as sildenafil (Revatio, Viagra), tadalafil (Cialis, Adcirca) vardenafil HCl (Levitra); or with other drugs beneficial for the prevention or the treatment of the above-mentioned diseases including but not limited to diazoxide; and including the free-acid, free-base, and pharmaceutically acceptable salt forms, pro-drug forms (including but not limited to esters), and salts of pro-drugs of the above medicinal agents where chemically possible. Trademark names of pharmaceutical drugs noted above are provided for exemplification of the marketed form of the active agent(s); such pharmaceutical drugs could be used in a separate dosage form for concurrent or sequential administration with a compound of Formula I, or the active agent(s) therein could be used in a fixed dose drug combination including a compound of Formula I.

In one aspect, the present invention provides a kit comprising a therapeutically effective amount of at least one Compound of Formula I, or a pharmaceutically acceptable salt of said compound and a pharmaceutically acceptable carrier, vehicle or diluent.
In another aspect the present invention provides a kit comprising an amount of at least one Compound of Formula I, or a pharmaceutically acceptable salt of said compound and an amount of at least one additional therapeutic agent listed above, wherein the amounts of the two or more active ingredients result in a desired therapeutic effect. In one embodiment, the one or more Compounds of Formula I and the one or more additional therapeutic agents are provided in the same container. In one embodiment, the one or more Compounds of Formula I and the one or more additional therapeutic agents are provided in separate containers.

The terms used herein have their ordinary meaning and the meaning of such terms is independent at each occurrence thereof. That notwithstanding and except where stated otherwise, the following definitions apply throughout the specification and claims. Chemical names, common names, and chemical structures may be used interchangeably to describe the same structure. These definitions apply regardless of whether a term is used by itself or in combination with other terms, unless otherwise indicated. Hence, the definition of "alkyl" applies to "alkyl" as well as the "alkyl" portions of "hydroxyalkyl," "haloalkyl," "-O-alkyl," etc.

As used herein, the term "administration" and variants thereof (e.g., "administering" a compound) in reference to a compound of the invention means providing the compound to the individual in need of treatment. When a compound of the invention is provided in combination with one or more other active agents (e.g., angiotensin converting enzyme inhibitors), "administration" and its variants are each understood to include concurrent and sequential administration of the compound or salt and other agents.

A "subject" (alternatively referred to herein as "patient") is a human or non-human mammal. In one embodiment, a subject is a human. In another embodiment, a subject is a primate. In another embodiment, a subject is a monkey. In another embodiment, a subject is a chimpanzee. In still another embodiment, a subject is a rhesus monkey.

The term "effective amount" as used herein means that amount of active compound or pharmaceutical agent that elicits the biological or medicinal response in a tissue, system, animal or human that is being sought by a researcher, veterinarian, medical doctor or other clinician. In one embodiment, the effective amount is a "therapeutically effective amount" for the alleviation of one or more symptoms of the disease or condition being treated. In another embodiment, the effective amount is a "prophylactically effective amount" for reduction of the severity or likelihood of one or more symptoms of the disease or condition. The term also includes herein the amount of active compound sufficient to modulate NPRA activity and thereby elicit the response being sought (*i.e.,* a "therapeutically effective amount"). When the active compound (*i.e.,* active ingredient) is administered as the salt, references to the amount of active ingredient are to the free acid or free base form of the compound.

The terms "treating" or "treatment" as used herein with respect to cardiometabolic diseases including high blood pressure, heart failure, kidney disease, and diabetes, includes inhibiting the severity of the cardiometabolic diseases including high blood pressure, heart failure, kidney disease, and diabetes, i.e., arresting or reducing the development of the diseases or its clinical symptoms; or relieving the diseases, i.e., causing regression of the severity of the diseases or their clinical symptoms.

The terms "preventing," or "prophylaxis," as used herein with respect to the cardiometabolic diseases including high blood pressure, heart failure, kidney disease, and diabetes, refers to reducing the likelihood or severity of the diseases.

The term "C₀" as employed in expressions such as "C₀₋₆ alkyl" means a direct covalent bond; or when the term appears at the terminus of a substituent, C₀₋₆ alkyl means hydrogen or C₁₋₆alkyl. Similarly, when an integer defining the presence of a certain number of atoms in a group is equal to zero, it means that the atoms adjacent thereto are connected directly by a bond. For example, in the structure 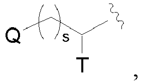 wherein s is an integer equal to zero, 1 or 2, the structure is 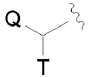 when s is zero.

The term "alkyl," as used herein, refers to an aliphatic hydrocarbon group having one of its hydrogen atoms replaced with a bond. An alkyl group may be straight or branched and contain from about 1 to about 20 carbon atoms. In one embodiment, an alkyl group contains from about 1 to about 12 carbon atoms. In different embodiments, an alkyl group contains from 1 to 6 carbon atoms (C₁-C₆ alkyl) or from about 1 to about 4 carbon atoms (C₁-C₄ alkyl). Non-limiting examples of alkyl groups include methyl, ethyl, n-propyl, isopropyl, n-butyl, sec-butyl, isobutyl, tert-butyl, n-pentyl, neopentyl, isopentyl, n-hexyl, isohexyl and neohexyl. In one embodiment, an alkyl group is linear. In another embodiment, an alkyl group is branched. Unless otherwise indicated, an alkyl group is unsubstituted.

The term "carbonyl" means a functional group composed of a carbon atom double-bonded to an oxygen atom, C=O.

The term "cycloalkyl" means a monocyclic or bicyclic saturated aliphatic hydrocarbon group having the specified number of carbon atoms. For example, "cycloalkyl" includes cyclopropyl, cyclobutyl, cyclopentyl, cyclohexyl, and so on. Bicyclic cycloalkyl ring systems include fused ring systems, where two rings share two atoms, and spiro ring systems, where two rings share one atom.

The term "heteroalkyl" refers to an alkyl group where 1, 2, or 3 of the carbon atoms is substituted by a heteroatom independently chosen from N, O, or S.

The term "alkoxy" refers to an alkyl (carbon and hydrogen chain) group singularly bonded to oxygen (R-O). Non-limiting examples of alkoxy are methoxy (CH₃ O-)., ethoxy (CH₃ CH₂ O-) and butoxy (CH₃ CH₂ CH₂ O-).

"Aryl" means a monocyclic, bicyclic or tricyclic carbocyclic aromatic ring or ring system containing 5-14 carbon atoms, wherein at least one of the rings is aromatic. Examples of aryl include phenyl and naphthyl. In on embodiment of the present invention, aryl is phenyl.

The term "halogen" includes fluorine, chlorine, bromine, and iodine.

"Haloalkyl" refers to an alkyl group as described above wherein one or more (in particular 1 to 5) hydrogen atoms have been replaced by halogen atoms, with up to complete substitution of all hydrogen atoms with halo groups. C₁₋₆ haloalkyl, for example, includes - CF₃, -CF₂CF₃, -CHFCH₃, and the like.

"Hydroxyalkyl" refers to an alkyl group as described above in which one or more (in particular 1 to 3) hydrogen atoms have been replaced by hydroxy groups. Examples include CH₂OH, CH₂CHOH and CHOHCH₃.

The term "heteroaryl", as used herein, represents a stable monocyclic, bicyclic or tricyclic ring system containing 5-14 carbon atoms and containing at least one ring heteroatom selected from N, S (including SO and SO₂) and O, wherein at least one of the heteroatom containing rings is aromatic. In the case of a heteroaryl ring system where one or more of the rings are saturated and contain one or more N atoms, the N can be in the form of quarternary amine. Bicyclic heteroaryl ring systems include fused ring systems, where two rings share two atoms, and spiro ring systems, where two rings share one atom. Heteroaryl groups within the scope of this definition include but are not limited to: azaindolyl, benzoimidazolyl, benzisoxazolyl, benzofuranyl, benzofurazanyl, benzopyrazolyl, benzotriazolyl, benzothiophenyl, benzothiazolyl, benzo[d]isothiazole, benzoxazolyl, carbazolyl, carbolinyl, cinnolinyl, furanyl, imidazolyl, indolinyl, indolyl, indolazinyl, indazolyl, isobenzofuranyl, isoindolyl, isoquinolyl, isothiazolyl, isoxazolyl, naphthpyridinyl, oxadiazolyl, oxazolyl, oxazoline, isoxazoline, pyranyl, pyrazinyl, pyrazolyl, pyrrolyl, pyrazolopyrimidinyl, pyridazinyl, pyridyl, pyrimidyl, pyrimidinyl, pyrrolyl, quinazolinyl, quinolyl, quinoxalinyl, tetrazolyl, tetrazolopyridyl, thiadiazolyl, 5H-pyrrolo[3,4-b]pyridine, thiazolyl, thienyl, triazolyl, triazinyl, benzothiazolyl, benzothienyl, quinolinyl, quinazolinyl, and isoquinolinyl, and oxazolyl. If the heteroaryl contains nitrogen atoms, it is understood that the corresponding N-oxides thereof are also encompassed by this definition.

The term "heterocyclyl" as used herein is intended to mean a stable nonaromatic monocyclic or bicyclic ring system of up to 10 atoms in each ring, unless otherwise specified, containing from 1 to 4 heteroatoms selected from the group consisting of O, N, S, SO, or SO₂. Bicyclic heterocyclic ring systems include fused ring systems, where two rings share two atoms, and spiro ring systems, where two rings share one atom. In a bicyclic ring system, the second ring may be a heteroaryl, heterocycle or a saturated, partially unsaturated or aromatic carbocycle, and the point(s) of attachment to the rest of the molecule may be on either ring. "Heterocyclyl" therefore include dihydro and tetrahydro analogs of heteroaryls (for example, a bicyclic having an aromatic ring and non-aromatic ring, such as, dihydrobenzoimidazolyl, dihydroquinolinyl). Attachment of a heterocyclyl substituent can occur via a carbon atom or via a heteroatom.

"Heterocyclyl" therefore includes, but is not limited to the following: azaspirononanyl, azabicyclo[3.1.0]hexanyl, azaspirooctanyl, azetidinyl, dioxanyl, diazapanyl, diazaspiroheptanyl, diazaspirodecanyl, diazaspirononanyl, dihydropyridazinyl, dihydropyridinyl, dihydrobenzoxazolyl, morpholinyl, octahydropyrrolopyrrolyl, octahydropyranopyridinyl, octahydropyrrolooxazinyl, oxazolidinyl, oxaazaspitodecanyl, oxaazobicyclo[2.2.1]heptanyl, oxadiazaspirodecanyl, oxadiazaspirononanyl, oxaspirooctanyl, oxazolidinonyl, oxazepanyl, oxathiazinanyl, oxetanyl, piperazinyl, piperidyl, piperidinyl, pyrrolidinyl, morpholinyl, thiomorpholinyl, tetrahydrofurnayl, tetrahydropyrimidinyl, tetrahydropyridyl, tetrahydropyranyl, dihydropiperidinyl, dihydroquinolinyl, dihydroindolyl,tetrahydrothiophenyl, dihydrobenzofuranyl, dihydrobenzoimidazolyl, tetra-hydroquinoline, methylenedioxybenzene, dihydrobenzodioxinyl, and the like. If the heterocycle contains a nitrogen, it is understood that the corresponding N-oxides thereof are also encompassed by this definition.

"Oxo" means an oxygen atom connected to another atom by a double boind and is represed by "=O" herein.

The term "sulfamoyl" is a suffix to denote radicals derived from sulfamide such as - SO₂NH₂, --SO₂NHR and -SO₂N(RR¹).

By "pharmaceutically acceptable" is meant that the ingredients of the pharmaceutical composition must be compatible with each other and not deleterious to the recipient thereof.

Where any amine is present in the compound, the N atom may be optionally in the form of a quaternary amine having one or more appropriate additional substitutions, as further described herein.

When any variable (*e.g*., n, R^{a}, R^{b}, etc.) occurs more than one time in any constituent or in Formula I, its definition on each occurrence is independent of its definition at every other occurrence. Also, combinations of substuents and/or variables are permissible only if such combinations result in stable compounds.

When any ring atom is specified as being optionally substituted with, or in a specified form, for example, S substituted with oxo groups, or N in the form of a N-oxide, this does not preclude the substitution of any ring atom with the other listed optional substuents when not substituted with oxo groups or in the form of a N-oxide.

"Celite^{®}" (Fluka) diatomite is diatomaceous earth, and can be referred to as "celite".

The term "substituted" means that one or more hydrogens on the designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds.

By "stable compound" or "stable structure" is meant a compound that is sufficiently robust to survive isolation to a useful degree of purity from a reaction mixture, and formulation into an efficacious therapeutic agent. The compounds of the present invention are limited to stable compounds embraced by Formula I.

The term "compound" refers to the compound and, in certain imbodiments, to the extent they are stable, any hydrate or solvate thereof. A hydrate is the compound complexed with water, and a solvate is the compound complexed with an organic solvent.

The term "in substantially purified form," as used herein, refers to the physical state of a compound after the compound is isolated from a synthetic process (*e.g*., from a reaction mixture), a natural source, or a combination thereof. The term "in substantially purified form," also refers to the physical state of a compound after the compound is obtained from a purification process or processes described herein or well-known to the skilled artisan (*e.g*., chromatography, recrystallization and the like), in sufficient purity to be characterizable by standard analytical techniques described herein or well-known to the skilled artisan.

It should also be noted that any carbon as well as heteroatom with unsatisfied valences in the text, schemes, examples and tables herein is assumed to have the sufficient number of hydrogen atom(s) to satisfy the valences.

When a functional group in a compound is termed "protected", this means that the group is in modified form to preclude undesired side reactions at the protected site when the compound is subjected to a reaction. Suitable protecting groups will be recognized by those with ordinary skill in the art as well as by reference to standard textbooks such as, for example, T. W. Greene et al, Protective Groups in Organic Synthesis (1991), Wiley, New York.

Lines drawn into the ring systems from substituents indicate that the indicated bond can be attached to any of the substitutable ring atoms. If the ring system is polycyclic, it is intended that the bond be attached to any of the suitable carbon atoms on the proximal ring only.

Under standard nomenclature used throughout this disclosure, the terminal portion of the designated side chain is described last, preceded by the adjacent functionality toward the point of attachment. For example, a C1-5 alkylcarbonylamino C1-6 alkyl substituent is equivalent to

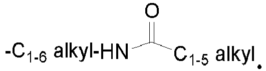

Structural representations of compounds having substituents terminating with a methyl group may display the terminal methyl group either using the characters "CH3", e.g. "-CH3" or using a straight line representing the presence of the methyl group, e.g. "-", i.e., 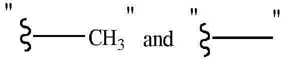 have equivalent meanings.

For variable definitions containing terms having repeated terms, e.g., **(CRiRj)r,** where r is the integer 2, **Ri** is a defined variable, and **Rj** is a defined variable, the value of **Ri** may differ in each instance in which it occurs, and the value of **Rj** may differ in each instance in which it occurs. For example, if **Ri** and **Rj** are independently selected from the group consisting of methyl, ethyl, propyl and butyl, then **(CRiRj)2** can be

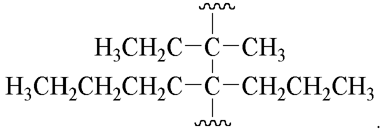

Unless expressly stated to the contrary, all ranges cited herein are inclusive. For example, a heteroaromatic ring described as containing from "1 to 4 heteroatoms" means the ring can contain, 1, 2, 3 or r heteroatoms. It is also to be understood that any range cited herein includes within its scope all of the sub-ranges within that range. Thus, for example, a heterocyclic ring described as containing from "1 to 4 heteroatoms" is intended to include as aspects thereof, heterocyclic rings containing 2 to 4 heteroatoms, 3 or 4 heteroatoms, 1 to 3 heteroatoms, 2 or 3 heteroatoms, 1 or 2 heteroatoms, 1 heteroatom, 2 heteroatims, 3 heteroatoms, and 4 heteroatoms. Similarly, C₁-C₆ when used with a chain, for example an alkyl chains means that the chain can contain 1, 2, 3, 4, 5, or 6 carbon atoms. It also includes all ranges contained therein including C₁-C₅, C₁-C₄, C₁-C₃, C₁-C₂, C₂-C₆, C₃-C₆, C₄-C₆, C₅-C₆, and all other possible combinations.

In choosing compounds of the present invention, one of ordinary skill in the art will recognize that the various substuents, *i.e.* R¹, R^{A}, etc., are to be chosen in conformity with well-known principles of chemical structure connectivity and stability.

As used herein, the term "composition" is intended to encompass a product comprising the specified ingredients in the specified amounts, as well as any product which results from combination of the specified ingredients in the specified amounts.

Prodrugs and solvates of the compounds of the invention are also described herein. A discussion of prodrugs is provided in T. Higuchi and V. Stella, Pro-drugs as Novel Delivery Systems (1987) 14 of the A.C.S. Symposium Series, and in Bioreversible Carriers in Drug Design, (1987) Edward B. Roche, ed., American Pharmaceutical Association and Pergamon Press. The term "prodrug" means a compound (*e.g.,* a drug precursor) that is transformed *in vivo* to provide a compound of Formula I or a pharmaceutically acceptable salt of the compound. The transformation may occur by various mechanisms (*e.g.,* by metabolic or chemical processes), such as, for example, through hydrolysis in blood. For example, if a compound of Formula I or a pharmaceutically acceptable salt, hydrate or solvate of the compound contains a carboxylic acid functional group, a prodrug can comprise an ester formed by the replacement of the hydrogen atom of the acid group with a group such as, for example, (C₁-C₈)alkyl, (C₂-C₁₂)alkanoyloxymethyl, 1-(alkanoyloxy)ethyl having from 4 to 9 carbon atoms, 1-methyl-1-(alkanoyloxy)-ethyl having from 5 to 10 carbon atoms, alkoxycarbonyloxymethyl having from 3 to 6 carbon atoms, 1-(alkoxycarbonyloxy)ethyl having from 4 to 7 carbon atoms, 1-methyl-1-(alkoxycarbonyloxy)ethyl having from 5 to 8 carbon atoms, N-(alkoxycarbonyl)aminomethyl having from 3 to 9 carbon atoms, 1-(N-(alkoxycarbonyl)amino)ethyl having from 4 to 10 carbon atoms, 3-phthalidyl, 4-crotonolactonyl, gamma-butyrolacton-4-yl, di-*N,N*-(C₁-C₂)alkylamino(C₂-C₃)alkyl (such as β-dimethylaminoethyl), carbamoyl-(C₁-C₂)alkyl, *N,N*-di (C₁-C₂)alkylcarbamoyl-(C₁-C₂)alkyl and piperidino-, pyrrolidino- or morpholino(C₂-C₃)alkyl, and the like.

Similarly, if a compound of Formula I contains an alcohol functional group, a prodrug can be formed by the replacement of one or more of the hydrogen atoms of the alcohol groups with a group such as, for example, (C₁-C₆)alkanoyloxymethyl, 1-((C₁-C₆)alkanoyloxy)ethyl, 1-methyl-1-((C₁-C₆)alkanoyloxy)ethyl, (C₁-C₆)alkoxycarbonyloxymethyl, N-(C₁-C₆)alkoxycarbonylaminomethyl, succinoyl, (C₁-C₆)alkanoyl, α-amino(C₁-C₄)alkyl, α-amino(C₁-C₄)alkylene-aryl, arylacyl and α-aminoacyl, or α-aminoacyl-α-aminoacyl, where each α-aminoacyl group is independently selected from the naturally occurring L-amino acids, or glycosyl (the radical resulting from the removal of a hydroxyl group of the hemiacetal form of a carbohydrate).

If a compound of Formula I incorporates an amine functional group, a prodrug can be formed by the replacement of a hydrogen atom in the amine group with a group such as, for example, R-carbonyl-, RO-carbonyl-, NRR'-carbonyl- wherein R and R' are each independently (C₁-C₁₀)alkyl, (C₃-C₇) cycloalkyl, benzyl, a natural α aminoacyl, -C(OH)C(O)OY¹ wherein Y¹ is H, (C₁-C₆)alkyl or benzyl, -C(OY²)Y³ wherein Y² is (C₁-C₄) alkyl and Y³ is (C₁-C₆)alkyl; carboxy (C₁-C₆)alkyl; amino(C₁-C₄)alkyl or mono-N- or di-N,N-(C₁-C₆)alkylaminoalkyl; - C(Y⁴)Y⁵ wherein Y⁴ is H or methyl and Y⁵ is mono-N- or di-N,N-(C₁-C₆)alkylamino morpholino; piperidin-1-yl or pyrrolidin-1-yl, and the like.

Pharmaceutically acceptable esters of the present compounds include the following groups: (1) carboxylic acid esters obtained by esterification of the hydroxy group of a hydroxyl compound, in which the non-carbonyl moiety of the carboxylic acid portion of the ester grouping is selected from straight or branched chain alkyl (*e.g*., methyl, ethyl, n-propyl, isopropyl, t-butyl, sec-butyl or n-butyl), alkoxyalkyl (*e.g.*, methoxymethyl), aralkyl (*e.g*., benzyl), aryloxyalkyl (for example, phenoxymethyl), aryl (*e.g*., phenyl optionally substituted with, for example, halogen, C₁₋₄alkyl, -O-(C₁₋₄alkyl) or amino); (2) sulfonate esters, such as alkyl- or aralkylsulfonyl (for example, methanesulfonyl); (3) amino acid esters, including those corresponding to both natural and non-natural amino acids (*e.g*., L-valyl or L-isoleucyl); (4) phosphonate esters and (5) mono-, di- or triphosphate esters. The phosphate esters may be further esterified by, for example, a C₁₋₂₀ alcohol or reactive derivative thereof, or by a 2,3-di (C₆₋₂₄)acyl glycerol.

One or more compounds of the invention may exist in unsolvated as well as solvated forms with pharmaceutically acceptable solvents such as water, ethanol, and the like, and it is intended that the invention embrace both solvated and unsolvated forms. "Solvate" means a physical association of a compound of this invention with one or more solvent molecules. This physical association involves varying degrees of ionic and covalent bonding, including hydrogen bonding. In certain instances the solvate will be capable of isolation, for example when one or more solvent molecules are incorporated in the crystal lattice of the crystalline solid. "Solvate" encompasses both solution-phase and isolatable solvates. Non-limiting examples of solvates include ethanolates, methanolates, and the like. A "hydrate" is a solvate wherein the solvent molecule is water.

One or more compounds of the invention may optionally be converted to a solvate. Preparation of solvates is generally known. Thus, for example, M. Caira et al, J. Pharmaceutical Sci., 93(3), 601-611 (2004) describe the preparation of the solvates of the antifungal fluconazole in ethyl acetate as well as from water. Similar preparations of solvates, hemisolvates, hydrates and the like are described by E. C. van Tonder et al, AAPS PharmSciTech., 5(1), article 12 (2004); and A. L. Bingham et al, Chem. Commun., 603-604 (2001). Atypical, non-limiting, process involves dissolving the inventive compound in desired amounts of the desired solvent (organic or water or mixtures thereof) at a higher than room temperature, and cooling the solution at a rate sufficient to form crystals which are then isolated by standard methods. Analytical techniques such as, for example IR spectroscopy, show the presence of the solvent (or water) in the crystals as a solvate (or hydrate).

The compound of Formula I can form salts which are also within the scope of this invention. Reference to a compound of Formula I herein is understood to include reference to salts thereof, unless otherwise indicated. The term "salt(s)", as employed herein, denotes acidic salts formed with inorganic and/or organic acids, as well as basic salts formed with inorganic and/or organic bases. In addition, when a compound of Formula I contains both a basic moiety, such as, but not limited to a pyridine or imidazole, and an acidic moiety, such as, but not limited to a carboxylic acid, zwitterions ("inner salts") may be formed and are included within the term "salt(s)" as used herein. In one embodiment, the salt is a pharmaceutically acceptable (*i.e*., non-toxic, physiologically acceptable) salt. In another embodiment, the salt is other than a pharmaceutically acceptable salt. Salts of the Compounds of Formula I may be formed, for example, by reacting a compound of Formula I with an amount of acid or base, such as an equivalent amount, in a medium such as one in which the salt precipitates or in an aqueous medium followed by lyophilization.

Exemplary acid addition salts include acetates, ascorbates, benzoates, benzenesulfonates, bisulfates, borates, butyrates, citrates, camphorates, camphorsulfonates, fumarates, hydrochlorides, hydrobromides, hydroiodides, lactates, maleates, methanesulfonates, naphthalenesulfonates, nitrates, oxalates, phosphates, propionates, salicylates, succinates, sulfates, tartarates, thiocyanates, toluenesulfonates (also known as tosylates) and the like. Additionally, acids which are generally considered suitable for the formation of pharmaceutically useful salts from basic pharmaceutical compounds are discussed, for example, by P. Stahl et al, Camille G. (eds.) Handbook of Pharmaceutical Salts. Properties, Selection and Use. (2002) Zurich: Wiley-VCH; S. Berge et al, Journal of Pharmaceutical Sciences (1977) 66(1) 1-19; P. Gould, International J. of Pharmaceutics (1986) 33 201-217; Anderson et al, The Practice of Medicinal Chemistry (1996), Academic Press, New York; and in The Orange Book (Food & Drug Administration, Washington, D.C. on their website).

Exemplary basic salts include ammonium salts, alkali metal salts such as sodium, lithium, and potassium salts, alkaline earth metal salts such as calcium and magnesium salts, salts with organic bases (for example, organic amines) such as dicyclohexylamine, t-butyl amine, choline, and salts with amino acids such as arginine, lysine and the like. Basic nitrogen-containing groups may be quarternized with agents such as lower alkyl halides (*e.g.*, methyl, ethyl, and butyl chlorides, bromides and iodides), dialkyl sulfates (*e.g.*, dimethyl, diethyl, and dibutyl sulfates), long chain halides (*e.g*., decyl, lauryl, and stearyl chlorides, bromides and iodides), arylalkyl halides (*e.g*., benzyl and phenethyl bromides), and others.

All such acid salts and base salts are intended to be pharmaceutically acceptable salts within the scope of the invention and all acid and base salts are considered equivalent to the free forms of the corresponding compounds for purposes of the invention.

Diastereomeric mixtures can be separated into their individual diastereomers on the basis of their physical chemical differences by methods well-known to those skilled in the art, such as, for example, by chromatography and/or fractional crystallization. Enantiomers can be separated by converting the enantiomeric mixture into a diastereomeric mixture by reaction with an appropriate optically active compound (*e.g.*, chiral auxiliary such as a chiral alcohol or Mosher's acid chloride), separating the diastereomers and converting (*e.g.*, hydrolyzing) the individual diastereomers to the corresponding pure enantiomers. Sterochemically pure compounds may also be prepared by using chiral starting materials or by employing salt resolution techniques. Also, some of the compound of Formula I may be atropisomers (*e.g*., substituted biaryls) and are considered as part of this invention. Enantiomers can also be directly separated using chiral chromatographic techniques.

It is also possible that the compound of Formula I may exist in different tautomeric forms, and all such forms are embraced within the scope of the invention. For example, all keto-enol and imine-enamine forms of the compounds are included in the invention.

Unless otherwise indicated, all stereoisomers (for example, geometric isomers, optical isomers and the like) of the present compounds (including those of the salts, solvates and hydratesof the compounds), such as those which may exist due to asymmetric carbons on various substituents, including enantiomeric forms (which may exist even in the absence of asymmetric carbons), rotameric forms, atropisomers, and diastereomeric forms, are contemplated within the scope of this invention. If a compound of Formula I incorporates a double bond or a fused ring, both the cis- and trans-forms, as well as mixtures, are embraced within the scope of the invention.

When a subsituent on a chiral carbon atom is depicted without specific stereochemistry (by using a straight line bond to a chiral center), it is to be understood that both the alpha and beta configurations of said subtituent group are to be considered part of the present invention. For example, the compound of the present invention, which is drawn as follows: 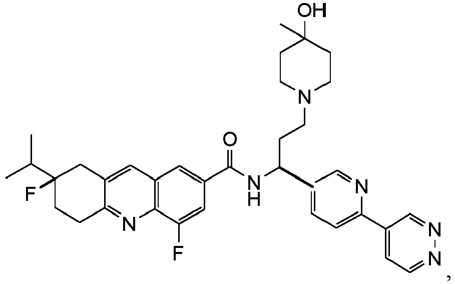 is understood to encompass both stereoisomers at the indicated chiral center located at the carbon atom attached to the carboxamide portion of the compound, the structures of which are as follows: 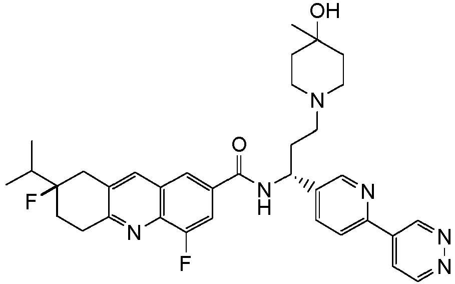 and

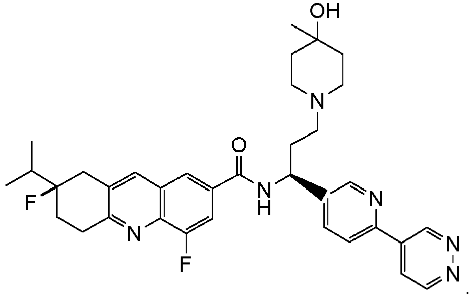

In the Examples section below, compounds of the present invention that have been purified as individual stereoisomers are sometimes depicted in non-stereospecific form but identifed using one or more of the terms: "diastereomer 1," "diastereomer 2," "isomer 1," "isomer 2," "enantiomer A" and "enantiomer B." In this instance, the absolute stereochemistry of each isolated diastereomer and enantiomeric center has not been determined and the terms used above are used to represent each individual purified stereochemically pure compound.

Individual stereoisomers of the compounds of the invention may, for example, be substantially free of other isomers, or may be admixed, for example, as racemates or with all other, or other selected, stereoisomers. The chiral centers of the present invention can have the S or R configuration as defined by the *IUPAC* 1974 Recommendations. The use of the terms "salt", "solvate", "ester", "prodrug" and the like, is intended to apply equally to the salt, solvate, ester and prodrug of enantiomers, stereoisomers, rotamers, tautomers, racemates or prodrugs of the inventive compounds.

In the Compounds of Formula I, the atoms may exhibit their natural isotopic abundances, or one or more of the atoms may be artificially enriched in a particular isotope having the same atomic number, but an atomic mass or mass number different from the atomic mass or mass number predominantly found in nature. The present invention is meant to include all suitable isotopic variations of the compounds of generic Formula I. For example, different isotopic forms of hydrogen (H) include protium (¹H) and deuterium (²H). Protium is the predominant hydrogen isotope found in nature. Enriching for deuterium may provide certain therapeutic advantages, such as increasing *in vivo* half-life or reducing dosage requirements, or may provide a compound useful as a standard for characterization of biological samples. Isotopically-enriched Compounds of Formula I can be prepared without undue experimentation by conventional techniques well known to those skilled in the art or by processes analogous to those described in the Schemes and Examples herein using appropriate isotopically-enriched reagents and/or intermediates. In one embodiment, a Compound of Formula I has one or more of its hydrogen atoms replaced with deuterium.

In another embodiment, the Compounds of Formula I are in substantially purified form.

### Biological Activity Determination

### NPRA Functional Cell Assay

### Materials

Assay Buffer components HEPES and Opti-MEM^{®} I Reduced-Serum with Glutamine (no phenol red) were from Gibco/Invitrogen (Thermo Fisher Scientific, Waltham, MA USA). 3-Isobutyl-1-methylxanthine (IBMX) and 4-(3-Butoxy-4-methoxybenzyl)imidazolidin-2-one (RO20) were obtained from Sigma-Aldrich (St. Louis, MO, USA). DPBS (Dulbecco's phosphate-buffered saline) without Ca2+ and Mg2+ was purchased from GE Healthcare Bio-Sciences (Pittsburgh, PA, USA). 384 well white Optiplates were from Perkin Elmer (Atlantic Highlands, NJ, USA). Human ANP (Atrial Natriuretic Peptide) (1-28) was purchased from Sigma-Aldrich (Catalog # A1663) and rat ANP (1-28) from Bachem (Torrance, CA, USA) (Catalog # H2100). Human-BNP (Human- Brain Natriuretic Peptide) (1- 32 AA) was purchased from American Peptide Company (Sunnyvale, CA, USA) (product No. 14-1-90, ). cGMP kits were purchased from Cisbio. The assay ready frozen (ARF) Human/rat/dog NPRA HEK JumpIn Stable frozen cells (low passage number 5-11) were prepared in-house.

### Methods

Test compounds were titrated in DMSO in a 10-point dose response in a separate step followed by a 100-fold dilution into the reaction. Positive response for each assay was determined using 10 nM rat-ANP peptide (rat cells) or 50 nM h-ANP peptide (human and dog cells).

Cells were thawed, washed with DPBS and resuspended in assay buffer (Opti-MEM + Glutamine, phenol red free media, 10 mM HEPES, 100 uM IBMX and 100 uM RO20) that was warmed to 37°C. Cells were then transferred to microplates via a Microplate Combi dispenser at a density of 1500, 400 and 1200 cells/well for human, rat and dog cells respectively, followed by acoustic transfer (Echo) of compound.

Compounds and cells were incubated at 37°C with 5% CO₂ for 1 hour, after which the cells were lysed and cGMP was captured using a CisBio cGMP HTRF assay kit. The TRF signal was measured with an Envision plate reader (emission set to 615 and 665 nm) after 1 hour incubation at ambient temperature. The TRF signal was converted to [cGMP] through the use of a cGMP calibration curve on each microplate. EC₅₀'s were generated from the resulting dose response curves by use of a 4 parameter logistic algorithm.

### METHODS OF SYNTHESIS

The compounds of the present invention can be prepared according to the following general schemes and specific examples, or modifications thereof, using readily available starting materials, reagents and conventional synthetic procedures. In these reactions, it is also possible to make use of variants which are themselves known to those of ordinary skill in this art but are not mentioned in greater detail. The general procedures for making the compounds claimed in this invention can be readily understood and appreciated by one skilled in the art from viewing the following schemes.

In some cases, the order of carrying out the foregoing reaction schemes may be varied to facilitate the reaction or to avoid unwanted reaction products. Additionally, various protecting group strategies familiar to one skilled in the art of organic synthesis may be employed to facilitate the reaction or to avoid unwanted reaction products.

In some cases, the final product may be further modified, for example, by manipulation of substituents. These manipulations may include, but are not limited to, reduction, oxidation, alkylation, acylation, and hydrolysis reactions which are commonly known to those skilled in the art.

The following examples are provided so that the invention might be more fully understood. These examples are illustrative only and should not be construed as limiting the invention in any way. Wherein a racemic mixture is produced, the enantiomers may be separated using SFC reverse or normal phase chiral resolution conditions either after isolation of the final product or at a suitable Intermediate, followed by processing of the single isomers individually. It is understood that alternative methodologies may also be employed in the synthesis of these key intermediates and examples. Asymmetric methodologies (e.g. chiral catalysis, auxiliaries) may be used where possible and appropriate. The exact choice of reagents, solvents, temperatures, and other reaction conditions, depends upon the nature of the intended product.

Unless otherwise indicated, when ratios of compounds (such as for examples solvents) are given, the ratio is on a volume to volume basis. For example, a 1:1 mixture of THF/H₂O means a mixture of 1 parts by volume THF to 1 parts by volume of H₂O. Additionally, unless otherwise specifically indicated, all reagents are commercially available, known in the literature, or readily synthesized by one skilled in the art. Straightforward protecting group strategies were applied in some routes.

The following abbreviations are used throughout the text:

| | |
|---|---|
| Ac | acetyl |
| aq | aqueous |
| Ar | aryl |
| Boc | *tert*-butoxycarbonyl |
| Boc₂O | di-*tert*-butyl dicarbonate |
| BrettPhos precatalyst | [(2-Di-cyclohexylphosphino-3,6-dimethoxy-2',4',6'-triisopropyl-1,1'-biphenyl)-2-(2'-amino-1,1'- biphenyl)]palladium(II) methanesulfonate methanesulfonate |
| Bu | butyl |
| Celite^{®} | diatomaceous earth |
| DAST | (diethylamino)sulfur trifluoride |
| DCE | 1,2-dichloroethane |
| DCM | dichloromethane |
| DEA | diethylamine |
| Dess-Martin periodinane, Dess Martin Agent | 1,1,1-Tris(acetyloxy)-1,1-dihydro-1,2-benziodoxol-3-(1*H*)-one |
| DIAD | diisopropyl azodicarboxylate |
| DIEA, DIPEA | *N,N*-diisopropylethylamine |
| di-*t*-Bu | di-*tert*-butyl |
| DMAP | 4-(dimethylamino)pyridine |
| DMF | *N,N*-dimethylformamide |
| DMP | 1,1,1-Tris(acetyloxy)-1,1-dihydro-1,2-benziodoxol-3-(1*H*)-one |
| DMSO | dimethylsulfoxide |
| EA/PE | Ethyl acetate/ petroleum ether |
| EDC | *N-*(3-dimethylaminopropyl)-*N*'-ethylcarbodiimide hydrochloride |
| Et₃N | triethylamine |
| EtOAc | Ethyl acetate |
| Eq, equiv. | equivalents |
| ESI | electrospray ionization |
| Et | ethyl |
| h | hours |
| HATU | *O*-(7-azabenzotriazol-1-yl)-*N,N,N'N'*-tetramethyluronium hexafluorophosphate |
| HTRF | Homogeneous Time Resolved Fluorescence |
| HOAc | Acetic acid |
| HOAt | 1-hydroxy-7-azabenzotriazole |
| HOBt | 1-hydroxybenzotriazole |
| HPLC | high performance liquid chromatography |
| *i*-Pr | isopropyl |
| KOAc | Potassium acetate |
| LCMS | liquid chromatography-mass spectrometry |
| M | molar |
| Me | methyl |
| min | minutes |
| MsCl | methanesulfonylchloride |
| MW | molecular weight |
| *n*-BuLi | *n*-butyllithium |
| NMR | nuclear magnetic resonance |
| OPMP | |
| PPh₃ | triphenylphosphine |
| Pd(OAc)₂ | Palladium(II) acetate |
| Pd/C | palladium on carbon |
| Pd₂(dba)₃ | Tri s(dibenzylideneacetone)dipalladium(0) |
| PdCl₂(dtbpf) | [1,1'-Bis(di-*tert-*butylphosphino)ferrocene]dichloropalladium(II) |
| Pd(dppf)Cl₂ | [1,1'-Bis(diphenylphosphino)ferrocene]dichloropalladium(II) |
| Ph | phenyl |
| psi | pounds per square inch |
| RB | Round bottomed |
| Rt. RT | ambient temperature |
| SFC | supercritical fluid chromatography |
| SM | starting material |
| *t*-Bu | *tert*-butyl |
| TBME | Methyl *tert*-butyl ether |
| TBTU | 2-(1H-benzotriazole-1-yl)-1,1,3,3-tetramethylaminium tetrafluorob orate |
| TEA | triethylamine |
| TEMPO | 2,2,6,6-Tetramethyl-1-piperidinyloxy |
| TFA | trifluoroacetic acid |
| Tf | trifluoromethanesulfonyl |
| THF | tetrahydrofuran |
| TMSCl | Trimethylsilyl chloride |
| TRF | Time Resolved Fluorescence |
| Ts-OH | p-Toluenesulfonic acid |
| V/V | volume to volume |
| Xantphos | (9, 9-dimethyl-9*H*-xanthene-4,5-diyl)bis(diphenylphosphane) |

### General Procedures

Starting materials and intermediates are purchased or are made using known procedures, or as otherwise illustrated. The general route applied to the synthesis of compounds of Formula I is described in the Schemes that follows. In some cases the order of carrying out the reaction steps in the schemes may be varied to facilitate the reaction or to avoid unwanted reaction products.

Reactions sensitive to moisture or air were performed under nitrogen or argon using anhydrous solvents and reagents. The progress of reactions was determined by either analytical thin layer chromatography (TLC) usually performed with E. Merck pre-coated TLC plates, silica gel 60F-254, layer thickness 0.25 mm or liquid chromatography-mass spectrometry (LC/MS).

### Biological Activity Testing

### NPRA Functional Cell Assay

### Materials:

Assay Buffer components HEPES and Opti-MEM^{®} I Reduced-Serum with Glutamine (no phenol red) were from Gibco/Invitrogen. 3-Isobutyl-1-methylxanthine (IBMX) and 4-(3-Butoxy-4-methoxybenzyl)imidazolidin-2-one (RO20) were obtained from Sigma. DPBS (Dulbecco's phosphate-buffered saline) without Ca2+ and Mg2+ was purchased from GE Healthcare Bio-Sciences (Pittsburgh, PA, USA). 384 well white Optiplates were from Perkin Elmer (Atlantic Highlands, NJ, USA). Human ANP (1-28) was purchased from Sigma (A1663)and rat ANP (1-28) from Bachem (H2100). Human-BNP (1- 32 AA) was purchased from American Peptide Company (Sunnyvale, CA, USA) (product No. 14-1-90). cGMP kits were purchased from Cisbio (Bedford, MA, USA). The assay ready frozen (ARF) Human/rat/dog NPRA HEK JumpIn Stable frozen cells (low passage number 5-11) were prepared in-house.

### Methods:

Test compounds were titrated in DMSO in a 10-point dose response in a separate step followed by a 100-fold dilution into the reaction. Positive response for each assay was determined using 10 nM rat-ANP peptide (rat cells) or 50 nM h-ANP peptide (human and dog cells).

Cells were thawed, washed with DPBS and resuspended in assay buffer (Opti-MEM + Glutamine, phenol red free media, 10 mM HEPES, 100 uM IBMX and 100 uM RO20) that was warmed to 37°C. Cells were then transferred to microplates via a Microplate Combi dispenser at a density of 1500, 400 and 1200 cells/well for human, rat and dog cells respectively, followed by acoustic transfer (Echo) of compound.

Compounds and cells were incubated at 37°C with 5% CO₂ for 1 hour, after which the cells were lysed and cGMP was captured using a CisBio cGMP HTRF assay kit. The TRF signal was measured with an Envision^{®} plate reader (Perkin Elmer)(emission set to 615 and 665 nm) after 1 hour incubation at ambient temperature. The TRF signal was converted to [cGMP] through the use of a cGMP calibration curve on each microplate. EC₅₀'s were generated from the resulting dose response curves by use of a 4 parameter logistic algorithm.

hNPRA EC₅₀ (nM) are were determined for the compounds of Example 1 through 273 and are reported in the Experimental section of this application.

### INTERMEDIATES

The following experimental procedures detail the preparation of intermediates used in n the synthesis of examples of the instant invention. The exemplified procedures are for illustrative purposes only, and are not intended to limit the scope of the instant invention in any way.

### Scheme A: Synthesis of intermediate I-1 4-(1-(trifluoromethyl)cyclopropyl)cyclohexan-1-one (I-1)

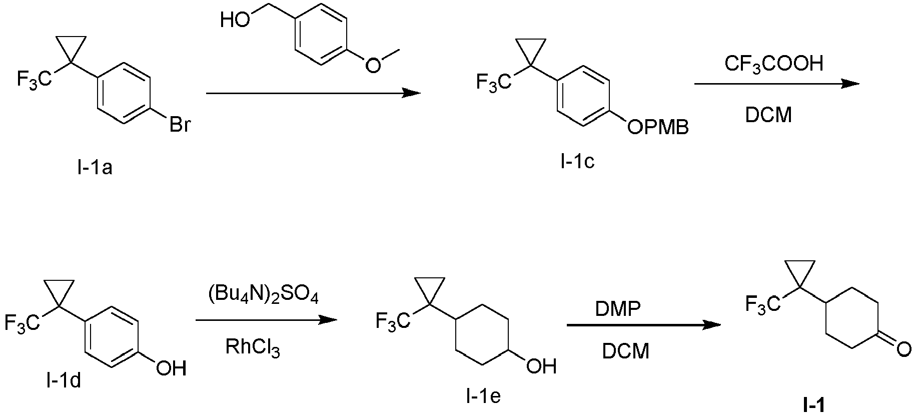

### Step 1 1-methoxy-4-((4-(1-(trifluoromethyl)cyclopropyl)phenoxy)methyl)benzene (I-1c)

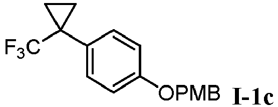

Into a 100-mL 3-necked round-bottom flask purged and maintained with an atmosphere of nitrogen was placed cesium carbonate (1.352 g, 4.14 mmol), copper iodide (72 mg, 0.38 mmol), (4-methoxyphenyl)methanol (782 mg, 5.66 mmol), 2,3,6,7-tetramethylpyrido[3,2-g]quinoline (178 mg, 0.75 mmol), a solution of 1-bromo-4-[1-(trifluoromethyl)cyclopropyl]benzene (1 g, 3.77 mmol) in toluene (50 mL). The resulting solution was stirred at 110°C for 24 h. The reaction was cooled to RT and diluted with 100 mL of EtOAc. The reaction was then quenched by the addition of saturated NH₄Cl aqueous solution. The solid was filtered out. The separated organic phase was washed with brine, dried over anhydrous sodium sulfate and concentrated under reduced pressure. The residue was purified by silica gel chromatography (EtOAc/petroleum ether) to give the title compound **I-1c**. MS: 346 (M+23).

### Step 2 4-(1-(trifluoromethyl)cyclopropyl)phenol (I-1d)

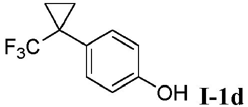

Into a 100-mL 3-necked round-bottom flask purged and maintained with an atmosphere of nitrogen was placed a solution of 1-[(4-methoxyphenyl)methoxy]-4-[1-(trifluoromethyl)cyclopropyl]benzene (**I-1c**, 550 mg, 1.71 mmol) in dichloromethane (10 mL). This was followed by the addition of TFA (389 mg, 3.41 mmol) dropwise with stirring at 0°C. The resulting solution was stirred at 0°C for 2 h. The resulting solution was diluted with 100 mL of DCM, then quenched by the addition of saturated NaHCOs aqueous solution. The organic layer was washed with 50 mL of brine, dried over anhydrous sodium sulfate and concentrated under vacuum. The residue was purified by silica gel chromatography (EtOAc/petroleum ether) to give the title compound, **I-1d**. MS: 253 (M+51).

### Step 3 4-(1-(trifluoromethyl)cyclopropyl)cyclohexan-1-ol (I-1e)

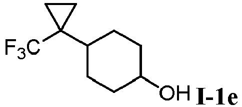

Into a 2000-mL pressure tank reactor was placed a mixture of 4-[1-(trifluoromethyl)cyclopropyl]phenol (**I-1d**, 22 g, 108.82 mmol) in hexane (650 mL), tetrabutylammonium sulphate (7.6 g, 13.04 mmol), phosphate buffer (650 mL), rhodium chloride (2.2 g, 10.51 mmol). The resulting solution was stirred for 20 h at RT under 60 psi H₂. The solid was filtered out. The filtrate was washed with 500 mL of brine. The organic layer was dried over anhydrous sodium sulfate and concentrated under reduced pressure. The residue was used in the next step without further purification. MS: 242 (M+34).

### Step 4 4-(1-(trifluoromethyl)cyclopropyl)cyclohexan-1-one (I-1)

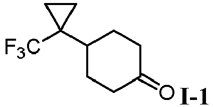

Into a 5000-mL 4-necked round-bottom flask purged and maintained with an atmosphere of nitrogen was placed a solution of 4-[1-(trifluoromethyl)cyclopropyl]cyclohexan-1-ol (**I-1e**, 56 g, 268.95 mmol) in dichloromethane (1500 mL). This was followed by the addition of Dess-Martin agent (148 g, 349.06 mmol) in several batches at 0°C. The resulting solution was stirred and gradually warmed to RT for 3 h. The reaction was then quenched by the addition of aq. NaHCO₃ and Na₂SO₃ solution. The organic phase was separated. The water phase was extracted with 2 x1000 mL of dichloromethane. The organic layers were combined, dried over Na₂SO₄, filtered and concentrated under reduced pressure. The residue was purified by silica gel chromatography (EtOAc/petroleum ether) to give the title compound, **I-1.** MS: 207 (M+1).

### Scheme B: Synthesis of intermediate 1-2 4-fluoro-4-isopropylcyclohexan-1-one (I-2)

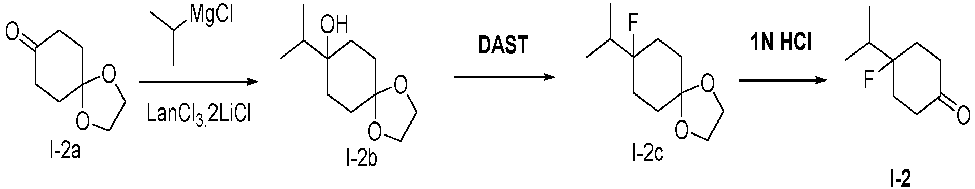

### Step 1 8-isopropyl-1,4-dioxaspiro[4.5]decan-8-ol (I-2b)

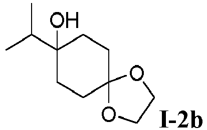

The lanthanum trichloride bis lithium chloride complex (0.6 M in THF, 117 mL, 70.4 mmol) was added to a solution of the 1,4-dioxaspiro[4.5]decan-8-one (**I-2a,** 10 g, 64.0 mmol) in THF (20 mL). The resulting mixture was stirred at RT under an nitrogen atmosphere for 1h, then cooled in an ice-water bath. Isopropylmagnesium chloride (2.0 M in THF, 35.2 mL, 70.4 mmol) was added and the ice bath was removed. The reaction mixture was stirred at RT for 2h. The reaction was quenched with sat. aq. ammonium chloride and most of the THF was removed under reduced pressure. The residue was extracted with EtOAc. The organic layer was dried over Na₂SO₄, filtered and concentrated under reduced pressure. The residue was purified by silica gel chromatography (EtOAc/petroleum ether) to give the title compound, **I-2b.** MS: 183 (M-H₂O+1).

### Step 2 8-fluoro-8-isopropyl-1,4-dioxaspiro[4.5]decane (I-2c)

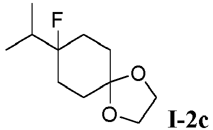

(8-isopropyl-1,4-dioxaspiro[4.5]decan-8-ol (**I-2b**, 4.63 g, 23.12 mmol) was dissolved in anhydrous toluene (50 mL) and cooled in an ice-water bath under the nitrogen atmosphere. DAST (7.45 g, 46.2 mmol) was added dropwise and the resulting reaction mixture was allowed to warm to RT and stirred overnight. The reaction was quenched sat. aq. sodium bicarbonate, and extracted with EtOAc. The aqueous phase was further extracted with EtOAc (X2). The combined organic phases were dried over Na₂SO₄, filtered and concentrated under reduced pressure. The residue was purified by silica gel chromatography (EtOAc/petroleum ether) to give the title compound, which contains 8-isopropyl-1,4-dioxaspiro[4.5]dec-7-ene, **I-2c.** The mixture was used in the next step without further purification. MS: 203 (M+1).

### Step 3 4-fluoro-4-isopropylcyclohexan-1-one (I-2)

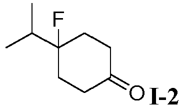

8-fluoro-8-isopropyl-1,4-dioxaspiro[4.5]decane **(I-2c,** 8 g, 39.6 mmol) in THF (15 mL) was treated with aqueous HCl (1N, 79 mL, 79 mmol) while cooled in an ice-water bath and the resulting reaction mixture was allowed to warm to RT and stirred overnight. The reaction was quenched with sat. aq. sodium bicarbonate in ice-water bath. The reaction was extracted with ether. The organic phase was dried over Na₂SO₄, filtered and concentrated under reduced pressure. The residue was purified by silica gel chromatography (EtOAc/petroleum ether) to give the title compound, **I-2.** MS: 158 (M+1).

### Scheme C: Synthesis of intermediate I-3 (7S)-7-(1-methylcyclopropyl)-5,6,7,8-tetrahydroacridine-2-carboxylic acid (I-3)

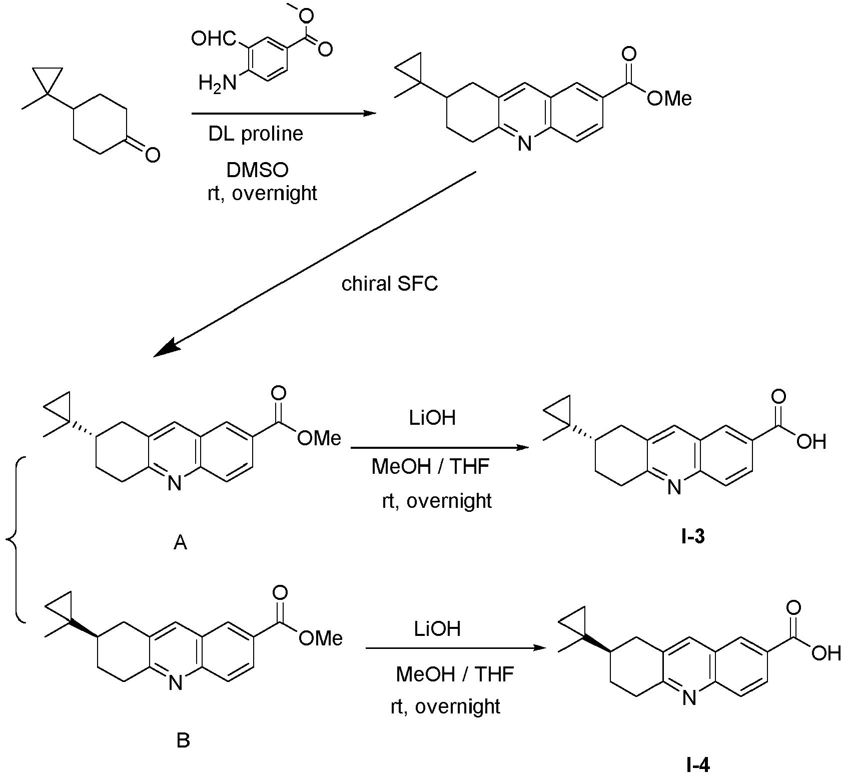

### Step 1 methyl (S)-7-(1-methylcyclopropyl)-5,6,7,8-tetrahydroacridine-2-carboxylate (A and B)

Into a 5000-mL 4-necked round-bottom flask was placed a solution of 4-(1-methylcyclopropyl)cyclohexan-1-one (prepared from the precedure of WO 2010039789, 81.6 g, 536.02 mmol, 1.60 equiv) in DMSO (1500 mL), methyl 4-amino-3-formylbenzoate (60 g, 334.87 mmol, 1.00 equiv), (D,L)-proline (19.3 g, 167.64 mmol, 0.50 equiv). The resulting solution was stirred overnight at room temperature. The reaction was then quenched by pouring into 6000 mL of sodium bicarbonate solution (aq.). The resulting solution was extracted with 3X4000 mL of ethyl acetate. The organic layers were combined, washed with 2X4500 mL of water and 5000 mL of brine, dried over anhydrous sodium sulfate and concentrated under vacuum. The crude product was re-crystallized from ether:hexane (1:1). The product was separated with preparative SFC (Lux Cellulose-4 (5*25cm, 5um), CO₂:IPA (0.1%DEA) = 65:35, 180g/min, back pressure 100 bar) to afford the title compound **A** as the first eluting peak, and the compound **B** as the second eluting peak. LC-MS 296 (M+1).

### Step 2 (7S)-7-(1-methylcyclopropyl)-5,6,7,8-tetrahydroacridine-2-carboxylic acid (I-3)

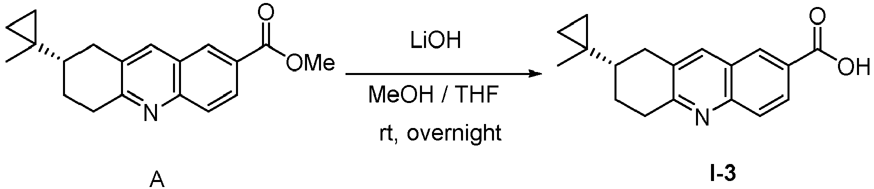

Into a 2000-mL round-bottom flask was placed a solution of methyl (7S)-7-(1-methylcyclopropyl)-5,6,7,8-tetrahydroacridine-2-carboxylate (A, 25 g, 84.64 mmol, 1.00 equiv) in methanol/THF (1:1, 500 mL), aq. lithium hydroxide (160 mL, 1M, 1.90 equiv). The resulting solution was stirred overnight at room temperature. Then it was concentrated under vacuum. The residual solution was adjusted to pH 5-6 with HCl (1 M). The solid was collected by filtration and dried in an oven under reduced pressure to afford the title compound **I-3.** LC-MS 282 (M+1).

### Intermediate 1-4 (R)-7-(1-methylcyclopropyl)-5,6,7,8-tetrahydroacridine-2-carboxylic acid (I-4)

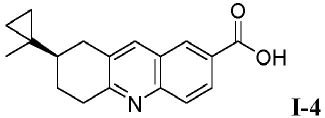

Intermediate, **I-4,** was prepared from compound B using analogous methodology to that outlined for Intermediate **I-3.** LC-MS 282 (M+1).

### Intermediate I-5 (S)-7-(tert-butyl)-5,6,7,8-tetrahydroacridine-2-carboxylic acid (I-5)

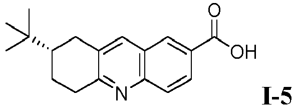

Intermediate, **I-5,** was prepared from compound A using analogous methodology to that outlined for Intermediate **I-3.** LC-MS 284 (M+1).

Following analogous methodology to that outlined for Intermediate **I-3** above, the following intermediates, **I-6, I-7, I-8, I-9, I-10,** and **I-11** were synthesized.

### Intermediate I-6: (R)-7-(1-methylcyclopropyl)-5,6,7,8-tetrahydroacridine-2-carboxylic acid (I-6)

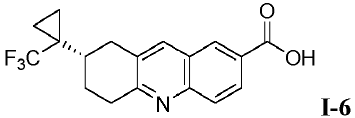

Intermediate **I-1** was used as starting material for making Intermediate, **I-6.** LC-MS 336 (M+1).

### Intermediate I-7 (S)-7-(1-methoxycyclopropyl)-5,6,7,8-tetrahydroacridine-2-carboxylic acid (I-7)

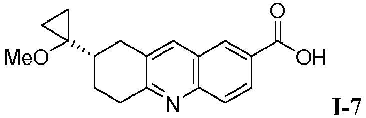

LC-MS 298 (M+1).

### Intermediate I-8 (S)-7-(1-(difluoromethyl)cyclopropyl)-5,6,7,8-tetrahydroacridine-2-carboxylic acid (I-8)

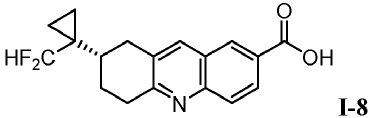

LC-MS 318 (M+1).

### Intermediate I-9 2-(tert-butyl)-1,2,3,4-tetrahydrobenzo[b][1,6]naphthyridine-8-carboxylic acid (I-9)

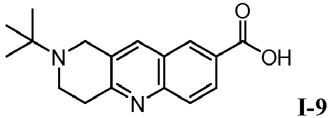

LC-MS 285 (M+1).

### Intermediate I-10 7-fluoro-7-isopropyl-5,6,7,8-tetrahydroacridine-2-carboxylic acid (I-10)

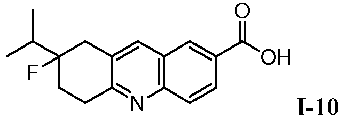

LC-MS 288 (M+1).

Intermediate **I-2** was used as starting material for making Intermediate, **I-10.**

### Intermediate I-11 (R)-7-fluoro-7-isopropyl-5,6,7,8-tetrahydroacridine-2-carboxylic acid (I-11)

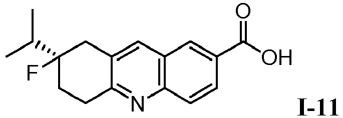

LC-MS 288 (M+1).

Intermediate **I-2** was used as starting material for making Intermediate, **I-11.**

### Scheme D: Synthesis of intermediate I-12

### 2-amino-5-bromo-3-fluorobenzaldehyde (I-12)

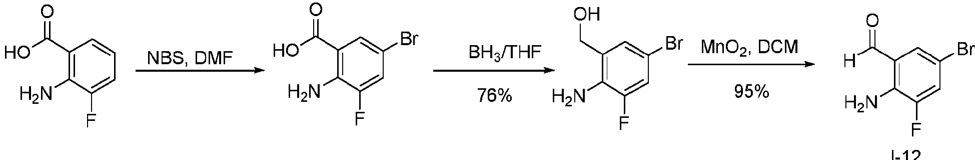

### Step 1 2-amino-5-bromo-3-fluorobenzoic acid

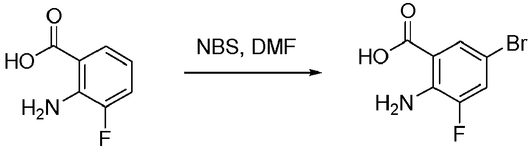

Into a 2-L 3-necked round-bottom flask purged and maintained with an inert atmosphere of nitrogen, was placed dichloromethane (1200 mL, 12.00 equiv), 2-amino-3-fluorobenzoic acid (100 g, 644.64 mmol, 1.00 equiv), NBS (120 g, 674.23 mmol, 1.00 equiv). The resulting solution was stirred for 3 h at 25°C. The solid was collected by filtration. This resulted in 2-amino-5-bromo-3-fluorobenzoic acid. LC-MS 236 (M+1).

### Step 2 (2-amino-5-bromo-3-fluorophenyl)methanol

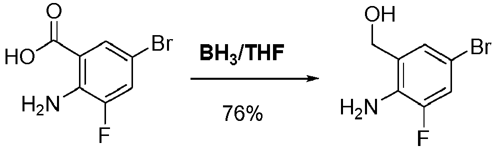

Into a 3-L 3-necked round-bottom flask purged and maintained with an inert atmosphere of nitrogen, was placed tetrahydrofuran (1587 mL), 2-amino-5-bromo-3-fluorobenzoic acid (158.7 g, 678.14 mmol, 1.00 equiv), BH₃-THF (1763 mL, 5.00 equiv) at 0°C. The resulting solution was stirred overnigt at 0°C. The reaction was then quenched by the addition of 200 mL of MeOH. The resulting mixture was concentrated under vacuum. The residue was dissolved in 500 mL of sat. aq. NaHCO₃. The resulting solution was extracted with 3x200 mL of ethyl acetate and the organic layers combined. The resulting mixture was washed with 3x200 mL of water and 2x200 mL of brine. The mixture was dried over anhydrous sodium sulfate and concentrated under vacuum. This resulted in (2-amino-5-bromo-3-fluorophenyl)methanol. LC-MS 223 (M+1).

### Step 3 2-amino-5-bromo-3-fluorobenzaldehyde (I-12)

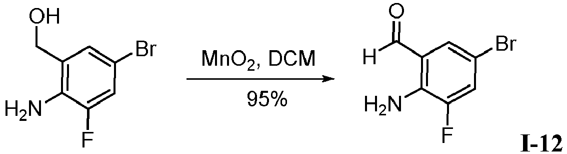

Into a 5-L 4-necked round-bottom flask purged and maintained with an inert atmosphere of nitrogen, was placed dichloromethane (3400 mL), (2-amino-5-bromo-3-fluorophenyl)methanol (96 g, 436.29 mmol, 1.00 equiv), MnO₂ (379 g, 4.36 mol, 10.00 equiv). The resulting mixture was stirred overnight at 75°C. The solid was filtered out. The filtrate was concentrated under vacuum. This resulted in 2-amino-5-bromo-3-fluorobenzaldehyde, **I-12**. LC-MS 220 (M+1).

Following analogous methodology to that outlined for Intermediate **I-12** above, the following intermediates, **I-13** and **I-14,** were synthesized.

### Intermediate I-13 2-amino-5-bromo-3-chlorobenzaldehyde (I-13)

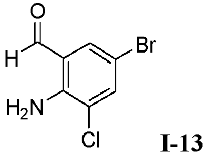

LC-MS 234 (M+1).

### Intermediate I-14 2-amino-5-bromo-3-methylbenzaldehyde (I-14)

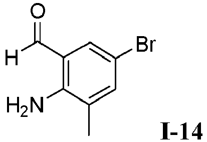

LC-MS 214 (M+1).

### Scheme E: Synthesis of intermediate I-15 ethyl (S)-4,7-difluoro-7-isopropyl-5,6,7,8-tetrahydroacridine-2-carboxylate (I-15)

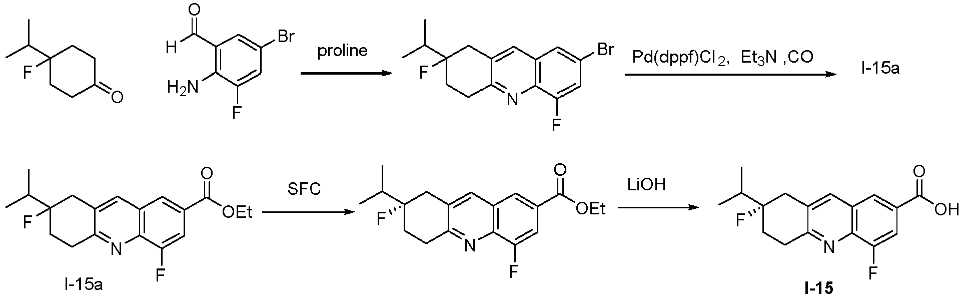

### Step 1 7-bromo-2,5-difluoro-2-isopropyl-1,2,3,4-tetrahydroacridine

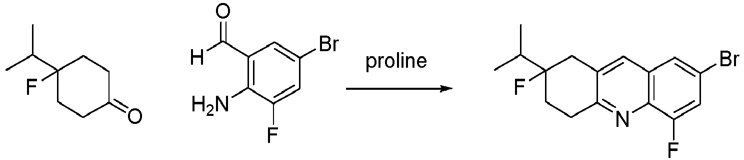

Into a 2-L 4-necked round-bottom flask purged and maintained with an inert atmosphere of nitrogen, was placed DMSO (1280 mL), 2-amino-5-bromo-3-fluorobenzaldehyde (64 g, 293.55 mmol, 1.20 equiv), 4-fluoro-4-(propan-2-yl)cyclohexan-1-one (**I-2**, 40 g, 252.82 mmol, 1.00 equiv), D-proline (30 g, 1.00 equiv). The resulting solution was stirred overnight at 25°C. The reaction was then quenched by the addition of 2000 mL of sat. aq. NaHCOs. The resulting solution was extracted with 3x500 mL of ethyl acetate and the organic layers combined. The resulting mixture was washed with 4x200 mL of H₂O and 2x200 mL of brine. The mixture was dried over anhydrous sodium sulfate and concentrated under vacuum. This resulted in 7-bromo-2,5-difluoro-2-(propan-2-yl)-1,2,3,4-tetrahydroacridine. LC-MS 340 (M+1).

### Step 2 ethyl 4,7-difluoro-7-isopropyl-5,6,7,8-tetrahydroacridine-2-carboxylate (I-15a)

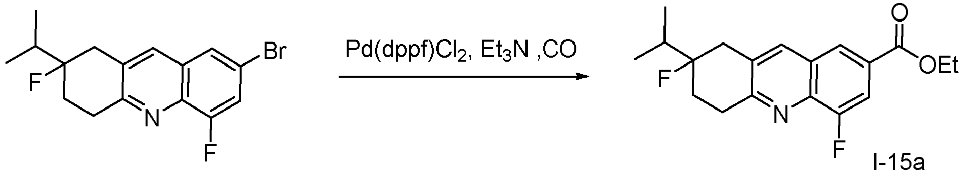

Into a 2-L round-bottom flask (1 atm) purged and maintained with an inert atmosphere of nitrogen, was placed N,N-dimethylformamide (960 mL), 7-bromo-2,5-difluoro-2-(propan-2-yl)-1,2,3,4-tetrahydroacridine (64 g, 188.12 mmol, 1.00 equiv), triethylamine (76.16 g, 752.64 mmol, 4.00 equiv), ethanol (173.44 g, 3.76 mol, 20.00 equiv), Pd(dppf)Cl₂ (15.36 g, 20.99 mmol, 0.10 equiv). To the above mixture CO (g) was introduced in. The resulting solution was stirred for 4 h at 90°C. The reaction was then quenched by the addition of 1000 mL of sat. aq. NaHCO₃ solution. The resulting solution was extracted with 4x200 mL of ethyl acetate and the organic layers combined. The resulting mixture was washed with 5x200 mL of H₂O and 3x200 mL of brine. The mixture was dried over anhydrous sodium sulfate and concentrated under vacuum. This resulted in ethyl 4,7-difluoro-7-(propan-2-yl)-5,6,7,8-tetrahydroacridine-2-carboxylate (**I-15a**). LC-MS 335 (M+1).

### Step 3 ethyl (S)-4,7-difluoro-7-isopropyl-5,6,7,8-tetrahydroacridine-2-carboxylate

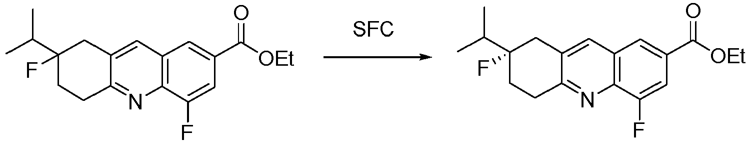

Ethyl 4,7-difluoro-7-(propan-2-yl)-5,6,7,8-tetrahydroacridine-2-carboxylate (57.7 g) was purified by Prep-SFC with the following conditions (prep SFC 350-2): Column, CHIRALPAK^{®} AD-H SFC, (Daicel Corporation, Fort Lee, NJ USA) 5*25cm,5um; mobile phase, CO₂(50%), ethanol(50%); Detector, uv 220 nm. This resulted in ethyl (S)-4,7-difluoro-7-isopropyl-5,6,7,8-tetrahydroacridine-2-carboxylate as the second eluting peak. LC-MS 335 (M+1).

### Step 4 ethyl (S)-4,7-difluoro-7-isopropyl-5,6,7,8-tetrahydroacridine-2-carboxylate (I-15)

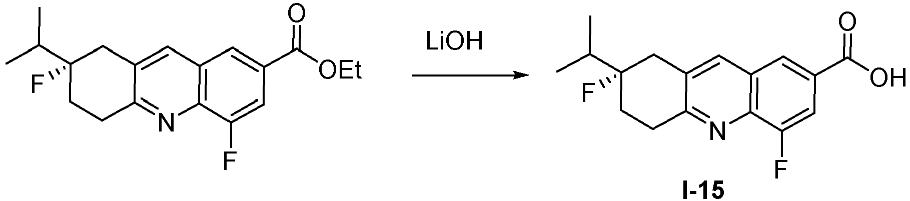

Into a 1-L 3-necked round-bottom flask purged and maintained with an inert atmosphere of nitrogen, was placed tetrahydrofuran (450 mL), ethyl (7R)-4,7-difluoro-7-(propan-2-yl)-5,6,7,8-tetrahydroacridine-2-carboxylate (30 g, 89.99 mmol, 1.00 equiv), aq. LiOH solution (1.0 M, 161.7 mL, 1.80 equiv). The resulting solution was stirred overnight at 30°C. The resulting mixture was concentrated under vacuum. The resulting solution was extracted with 200 mL of MTBE and the aqueous layers combined. The pH value of the solution was adjusted to 6 with aq. HCl (12 N). The solids were collected by filtration and washed with n-hexane. This resulted in (7R)-4,7-difluoro-7-(propan-2-yl)-5,6,7,8-tetrahydroacridine-2-carboxylic acid, **I-15.** LC-MS 305 (M+1).

Following analogous methodology to that outlined for Intermediate **I-15** above, the following intermediates, **I-16** through **I-20,** were synthesized.

### Intermediate I-16: (S)-7-(tert-butyl)-4-fluoro-5,6,7,8-tetrahydroacridine-2-carboxylic acid (I-16)

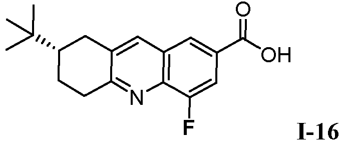

LC-MS 302 (M+1).

### Intermediate I-17 (S)-7-(tert-butyl)-4-chloro-5,6,7,8-tetrahydroacridine-2-carboxylic acid (I-17)

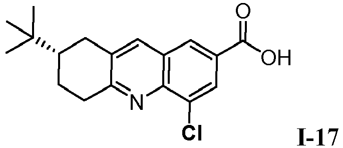

LC-MS 318 (M+1).

### Intermediate I-18 7-(tert-butyl)-4-methyl-5,6,7,8-tetrahydroacridine-2-carboxylic acid (I-18)

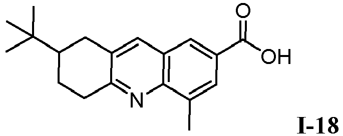

LC-MS 298 (M+1).

### Intermediate I-19 (S)-7-(tert-butyl)-3-fluoro-5,6,7,8-tetrahydroacridine-2-carboxylic acid (I-19)

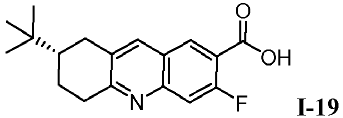

LC-MS 302 (M+1).

### Intermediate I-20 (S)-3,4,7-trifluoro-7-isopropyl-5,6,7,8-tetrahydroacridine-2-carboxylic acid (I-20)

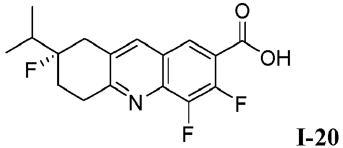

LC-MS 324 (M+1).

### Scheme F: Synthesis of intermediate I-21 (R)-3-(4-(1-amino-3-(4-hydroxypiperidin-1-yl)propyl)phenyl)oxazolidin-2-one (I-21)

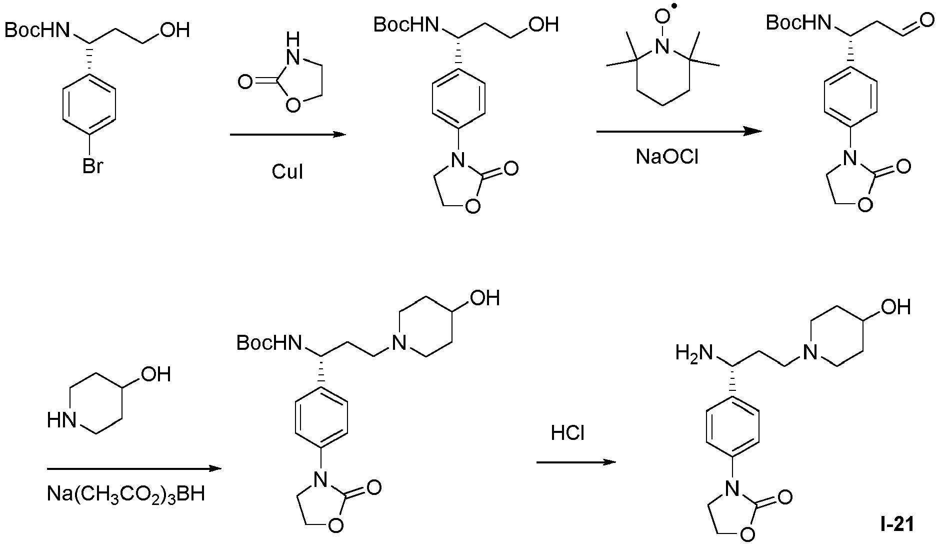

### Step 1 tert-butyl (R)-(3-hydroxy-1-(4-(2-oxooxazolidin-3-yl)phenyl)propyl)carbamate

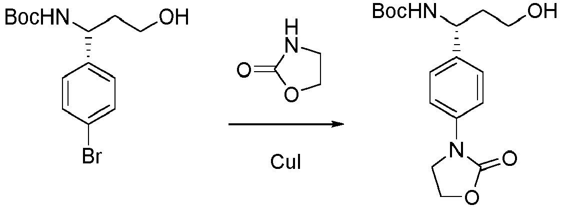

*tert*-butyl (R)-(1-(4-bromophenyl)-3-hydroxypropyl)carbamate (5.76 g, 17.4 mmol), potassium carbonate (7.23 g, 52.3 mmol), and copper iodide (4.9 g, 26.2 mmol) were all put into a round bottom flask. Dioxane (174 mL) was added and the reaction was stirred at RT while N₂ was bubbled through for 5min. Trans-N,N'-dimethylcyclohexane-1,2-diamine (8.3 mL, 52.6 mmol) was then added to the reaction and the reaction was stirred at 90°C overnight. The reaction was cooled to RT and diluted with aqueous NaHCO₃ solution and EtOAc. The layers were separated, and the organic layer was washed with brine. The organic phase was dried with anhydrous sodium sulfate, filtered, concentrated and purified by silica chromatography (0-10% MeOH/DCM) to provide the title compound. M: 337 (M+1).

### Step 2 tert-butyl (R)-(3-oxo-1-(4-(2-oxooxazolidin-3-yl)phenyl)propyl)carbamate

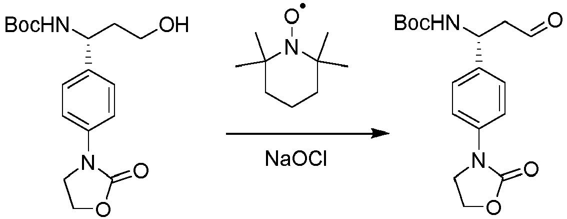

*tert*-butyl (R)-(3-hydroxy-1-(4-(2-oxooxazolidin-3-yl)phenyl)propyl)carbamate (5.92 g, 17.6 mmol) was dissolved in DCM (175 mL), TEMPO (0.287 g, 1.8 mmol) was added to the flask and the reaction was cooled to 0°C. Once cooled, bleach (6% aq., 50 mL) was added to the reaction. The reaction mixture was stirred for 3h. The layers were separated. The organic layer was washed with 10% sodium thiosulfate (aq.). The organic phase was dried with anhydrous sodium sulfate and concentrated under reduced pressure to afford the title compound. MS:335 (M+1).

### Step 3 tert-butyl (R)-(3-(4-hydroxypiperidin-1-yl)-1-(4-(2-oxooxazolidin-3-yl)phenyl)propyl)carbamate

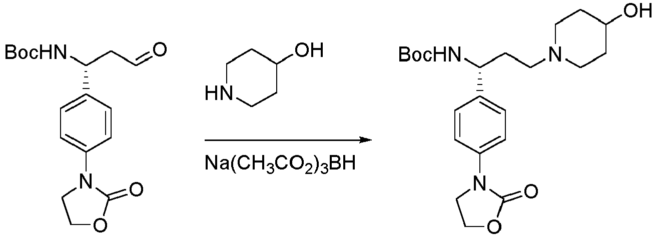

*tert*-butyl (R)-(3-oxo-1-(4-(2-oxooxazolidin-3-yl)phenyl)propyl)carbamate (3.72 g, 11.1 mmol) was dissolved in DCE (200 mL) and piperidin-4-ol (1.81 g, 17.9 mmol) was added. The mixture was stirred at RT for 5 min. Sodium triacetoxyborohydride (7.0 g, 33.1 mmol) was added to the reaction and the reaction mixture was stirred at RT over the weekend. The reaction was quenched with the addition of sat. NaHCO₃ (aq.) and diluted with DCM. The layers were separated and the organic layer was washed with brine. The organic layer was dried with anhydrous sodium sulfate, filtered, concentrated and purified by silica chromatography (0-30% MeOH/DCM) to provide the title compound. MS: 420 (M+1).

### Step 4 (R)-3-(4-(1-amino-3-(4-hydroxypiperidin-1-yl)propyl)phenyl)oxazolidin-2-one (I-21)

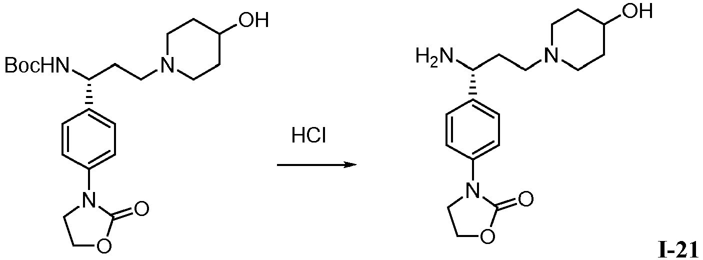

*tert*-butyl (R)-(3-(4-hydroxypiperidin-1-yl)-1-(4-(2-oxooxazolidin-3-yl)phenyl)propyl)carbamate (1.09 g, 2.6 mmol) was dissolved in THF (12 mL) and MeOH (12 mL). 4M HCl in dioxane (12 mL) was then added to the flask and the reaction was stirred at 75°C for 1h. The reaction mixture was concentrated under reduced pressure. The residue was dissolved in small amount of MeOH, and then diethyl ether was added. A solid crashed out, and the mixture was filtered. The solid was collected to give title compound as HCl salt. MS: 320 (M+1).

### Intermediate I-22

### (S)-N-((R)-1-(4-bromophenyl)-3-hydroxypropyl)-7-(1-methylcyclopropyl)-5,6,7,8-tetrahydroacridine-2-carboxamide (I-22)

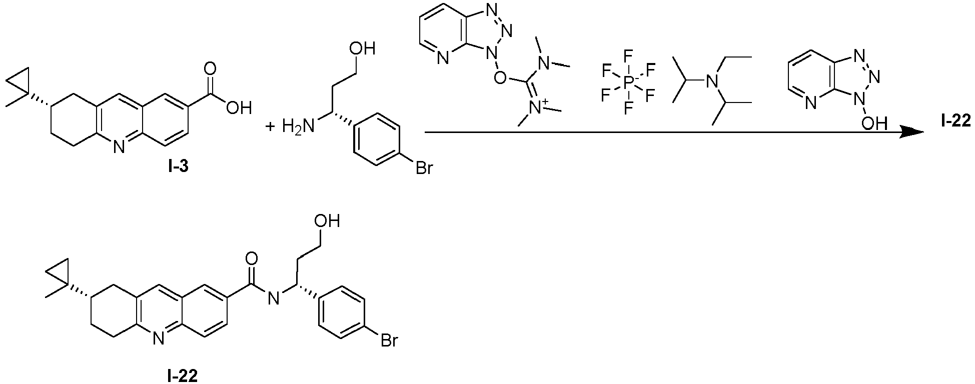

To a flask with (S)-7-(1-methylcyclopropyl)-5,6,7,8-tetrahydroacridine-2-carboxylic acid (I-3, 4.5 g, 15.99 mmol), (R)-3-amino-3-(4-bromophenyl)propan-1-ol (3.86 g, 16.79 mmol), HATU (8.51 g, 22.39 mmol), and HOAT (3.05 g, 22.39 mmol) was added DMF (60 ml) followed by DIEA (8.38 ml, 48.0 mmol)) at 0°C. The reaction was stirred at room temperature for 60 h. The reaction was quenched with sat. aq. NaHCO₃ solution, extracted with ethyl acetate (400 mL). The organic layer was washed with water (50 mLX 2), brine, dried over sodium sulfate, filtered and concentrated. The residue was purified by silica gel flash chromatography eluting with hexane grading to acetone to give the title compound, **I-22.** MS: 493 (M+1).

### Intermediate I-23 (S)-7-(1-methylcyclopropyl)-N-((R)-3-oxo-1-(4-(2-oxooxazolidin-3-yl)phenyl)propyl)-5,6,7,8-tetrahydroacridine-2-carboxamide (I-23)

### Step 1 (S)-N-((R)-3-hydroxy-1-(4-(2-oxooxazolidin-3-yl)phenyl)propyl)-7-(1-methylcyclopropyl)-5,6,7,8-tetrahydroacridine-2-carboxamide

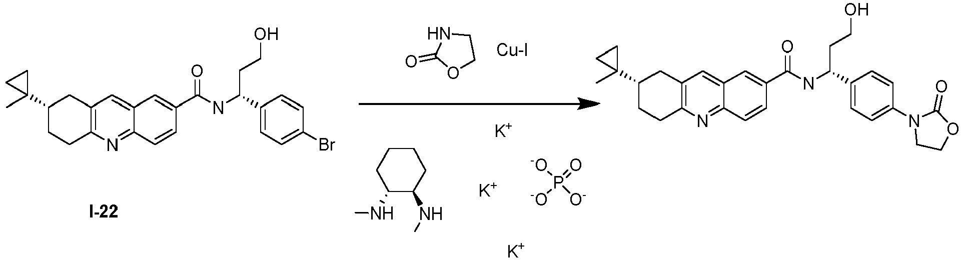

A mixture of (S)-N-((R)-1-(4-bromophenyl)-3-hydroxypropyl)-7-(1-methylcyclopropyl)-5,6,7,8-tetrahydroacridine-2-carboxamide **(I-22,** 750 mg, 1.520 mmol), copper(I) iodide (434 mg, 2.280 mmol), potassium phosphate tribasic (5 M aq. Solution, 922 µl, 4.56 mmol) was flushed with N₂ three times. To it were added DMSO (15 mL) and trans-N,N'-Dimethylcyclohexane-1,2-diamine (649 mg, 4.56 mmol). The reaction was stirred at 90°C for 2h. The reaction mixture was diluted with ethyl acetate, quenched with aq. NH₄Cl solution. The organic layer was washed with water, brine, dried over sodium sulfate, filtered and concentrated. The residue was purified by silica gel chromatography (MeOH in DCM) to afford the title compound. MS: 500 (M+1).

### Step 2 (S)-7-(1-methylcyclopropyl)-N-((R)-3-oxo-1-(4-(2-oxooxazolidin-3-yl)phenyl)propyl)-5,6,7,8-tetrahydroacridine-2-carboxamide

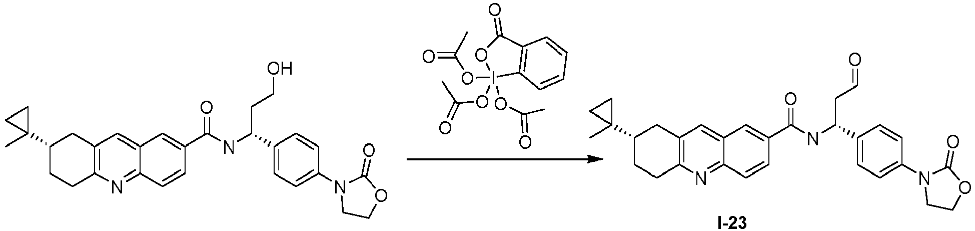

To a solution of (S)-N-((R)-3-hydroxy-1-(4-(2-oxooxazolidin-3-yl)phenyl)propyl)-7-(1-methylcyclopropyl)-5,6,7,8-tetrahydroacridine-2-carboxamide (700 mg, 1.401 mmol) in DCM (60 mL) at 0 °C was added DMP (832 mg, 1.962 mmol). The reaction was slowly warmed to RT. After stirring at RT for 2h, the reaction was quenched with DCM, sat. aq. NaHCO₃ and sat. aq. Na₂SO₃ solution. The layers were separated and then the aqueous layer was extracted with DCM (2 x 100 mL). The combined organic layers were washed with brine, dried over sodium sulfate, filtered and concentrated to give the title compound, **I-23.** MS: 498 (M+1).

### Intermediate I-24 S)-N-((R)-1-(6-chloropyridin-3-yl)-3-hydroxypropyl)-4,7-difluoro-7-isopropyl-5,6,7,8-tetrahydroacridine-2-carboxamide (I-24)

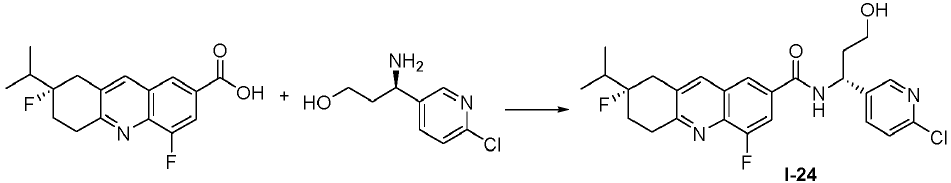

To a flask with (S)-4,7-difluoro-7-isopropyl-5,6,7,8-tetrahydroacridine-2-carboxylic acid **(I-15,** 3500 mg, 11.46 mmol), HOAt (1872 mg, 13.76 mmol), and HATU (5230 mg, 13.76 mmol) was added DMF (30 mL). The mixture was stirred for 10min, then (R)-3-amino-3-(6-chloropyridin-3-yl)propan-1-ol (HCl salt, 2813 mg, 12.61 mmol) was added, followed by DIEA (8.01 mL, 45.9 mmol). The reaction was stirred at room temperature overnight. The reaction mixture was added dropwise to aqueous NaHCO₃ solution (400 mL). The mixture was stirred for 10 min, then filtered. The filter cake was dried to afford the title compound. MS [M+H] 505.

### Intermediate I-25 (S)-4,7-difluoro-7-isopropyl-N-((R)-3-oxo-1-(6-(pyridazin-4-yl)pyridin-3-yl)propyl)-5,6,7,8-tetrahydroacridine-2-carboxamide (I-25)

### Step 1 (S)-4,7-difluoro-N-((R)-3-hydroxy-1-(6-(pyridazin-4-yl)pyridin-3-yl)propyl)-7-isopropyl-5,6,7,8-tetrahydroacridine-2-carboxamide

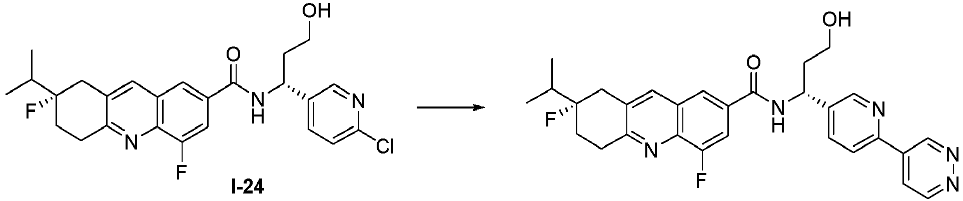

A mixture of (S)-N-((R)-1-(6-chloropyridin-3-yl)-3-hydroxypropyl)-4,7-difluoro-7-isopropyl-5,6,7,8-tetrahydroacridine-2-carboxamide (**I-24**, 3000 mg, 6.33 mmol), Pd(dppf)Cl₂ (1098 mg, 0.949 mmol) and 4-(4,4,5,5-tetramethyl-1,3,2-dioxaborolan-2-yl)pyridazine (2609 mg, 12.66 mmol) was flushed with N₂ three times. Then dioxane (20 mL) and aqueous Na₂CO₃ solution (2.5 M, 6.33 ml, 15.82 mmol), and water (2 mL) was added. The mixture was flushed with N₂ three times. Then the mixture was stirred at 100 ^{O}C for 5 hrs. The mixture was cooled to rt, poured into DCM. Na₂SO₄ was added. The mixture was then stirred for 20 min, filtered and evaporated. The residue was purified by flash chromatography (Teledyne Isco CombiFlash^{®} Rf, RediSep^{®} Silica 125g, Teledyne, Lincoln, NE USA) eluting with hexane grading to acetone to give product. MS [M+H] 518.

### Step 2 (S)-4,7-difluoro-7-isopropyl-N-((R)-3-oxo-1-(6-(pyridazin-4-yl)pyridin-3-yl)propyl)-5,6,7,8-tetrahydroacridine-2-carboxamide (I-25)

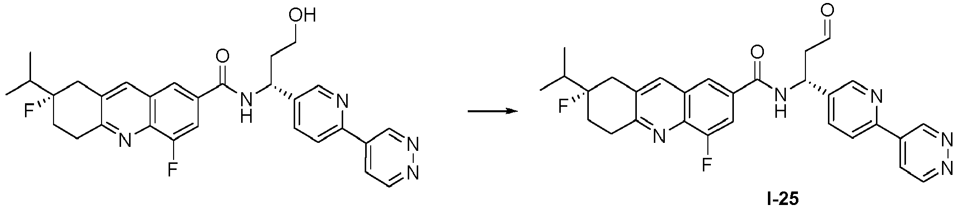

(S)-4,7-difluoro-N-((R)-3-hydroxy-1-(6-(pyridazin-4-yl)pyridin-3-yl)propyl)-7-isopropyl-5,6,7,8-tetrahydroacridine-2-carboxamide (305 mg, 0.589 mmol) was dissolved in CH₂Cl₂ (3929 µL). To it was slowly added Dess-Martin periodinane (325 mg, 0.766 mmol). The reaction was stirred for 1h at rt. The reaction was diluted in DCM and quenched with sat. aqueous NaHCO₃ and sat. aqueous Na₂S₂O₃ solution. The mixture was stirred for 30 min. Two layers were separated. The organic layer was collected, dried over Na₂SO₄, filtered, and concentrated to afford the title compound, I-25. MS [M+H] 516.

### Intermediate I-26 (S)-N-((R)-1-(6-chloropyridin-3-yl)-3-oxopropyl)-4,7-difluoro-7-isopropyl-5,6,7,8-tetrahydroacridine-2-carboxamide (I-26)

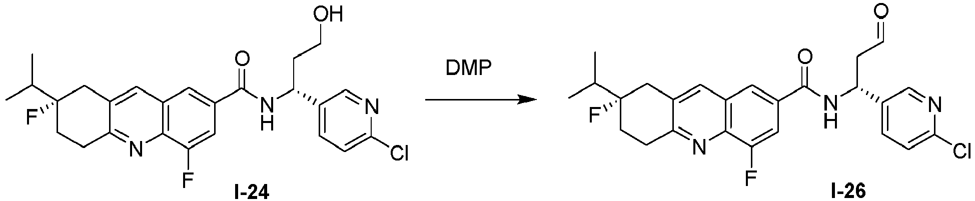

(S)-N-((R)-1-(6-chloropyridin-3-yl)-3 -hydroxypropyl)-4,7-difluoro-7-isopropyl-5, 6,7, 8-tetrahydroacridine-2-carboxamide **(I-24,** 2.5 g, 5.27 mmol) was dissolved in DCM (105 mL). To it was slowly added DMP (2.68 g, 6.33 mmol). The reaction was stirred at RT for 1h. The reaction was diluted in DCM and quenched with sat. aqueous NaHCO₃ and sat. aqueous Na₂S₂O₃ solution. The mixture was stirred for 30 min. Two layers were separated. The organic layer was collected, dried over Na₂SO₄, filtered, and concentrated to afford the title compound, **I-26.** MS [M+H] 503.

### Intermediate I-27 (S)-N-((R)-1-(6-chloropyridin-3-yl)-3-(4-hydroxypiperidin-1-yl)propyl)-4,7-difluoro-7-isopropyl-5,6,7,8-tetrahydroacridine-2-carboxamide (I-27)

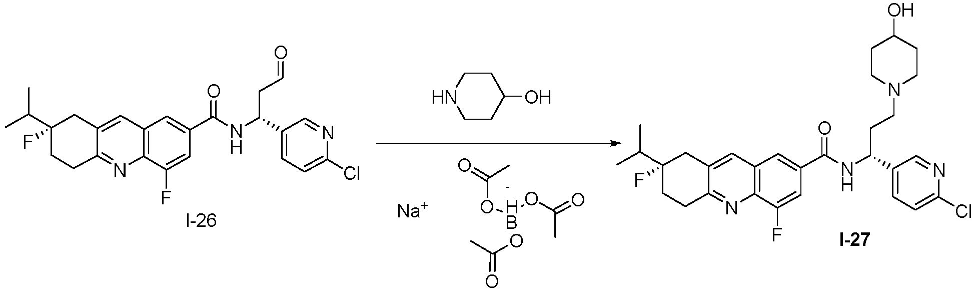

To (S)-N-((R)-1-(6-chloropyridin-3-yl)-3-oxopropyl)-4,7-difluoro-7-isopropyl-5,6,7,8-tetrahydroacridine-2-carboxamide **(I-26,** 1100 mg, 2.331 mmol) and 4-hydroxypiperidine (707 mg, 6.99 mmol) was added 5% AcOH in DCM (50 mL). The mixture was stirred at RT for 20 mins, and then was added sodium triacetoxyborohydride (988 mg, 4.66 mmol) slowly. The reaction was stirred at RT for another 30mins, diluted with DCM and quenched with sat. aq. NaHCO₃. The organic layer was washed with water, brine, dried over sodium sulfate, filtered and concentrated. The residue was purified by silica gel chromatography (acetone in hexanes) to afford the title compound, **(I-27).** MS [M+H] 557.

### Intermediate I-28 methyl 1-((R)-3-(6-chloropyridin-3-yl)-3-((S)-4,7-difluoro-7-isopropyl-5,6,7,8-tetrahydroacridine-2-carboxamido)propyl)piperidine-4-carboxylate (I-28)

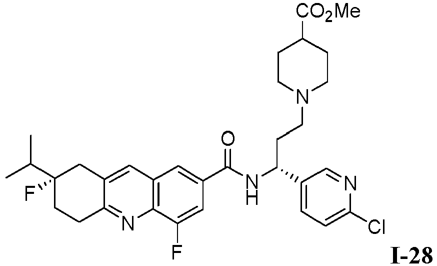

MS [M+H] 599.

Intermediate **I-28** was prepared following analogous methodology to that outlined for Intermediate **I-27** above.

### Intermediate I-29 (S)-9-(hydroxymethyl)-7-(1-methylcyclopropyl)-5,6,7,8-tetrahydroacridine-2-carboxylic acid (I-29)

### Step 1 methyl (S)-9-(hydroxymethyl)-7-(1-methylcyclopropyl)-5,6,7,8-tetrahydroacridine-2-carboxylate

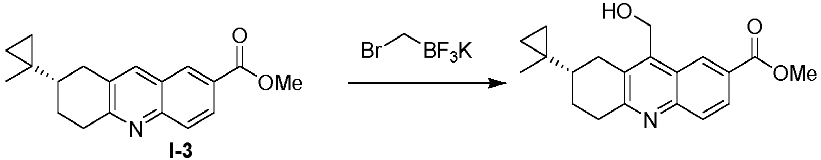

In a dry microwave vessel (S)-methyl 7-(1-methylcyclopropyl)-5,6,7,8-tetrahydroacridine-2-carboxylate **(I-3,** 1000 mg, 3.39 mmol), (bromomethyl)trifluoro-l4-borane, potassium salt (1360 mg, 6.77 mmol), silver phosphate (1134 mg, 2.71 mmol) and potassium persulfate (2196 mg, 8.13 mmol) were combined and evacuated followed by N₂ backfill three times. Degassed 3:1 HOAc:H₂O (bubbled with N₂ for 5 min.) was added and stirred at 50°C for 20 hrs. The reaction was cooled to RT and partitioned between EtOAc and H₂O. The organic layer was washed with water, brine; dried over sodium sulfate, filtered and concentrated. The residue was purified by silica gel chromatography (EtOAc in hexanes) to afford the title compound. MS [M+H] 326.

### Step 2 (S)-9-(hydroxymethyl)-7-(1-methylcyclopropyl)-5,6,7,8-tetrahydroacridine-2-carboxylic acid (I-29)

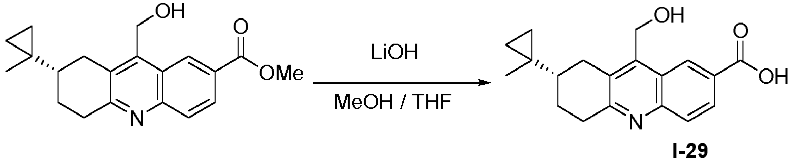

Intermediate I-29 was prepared following analogous methodology to that outlined for intermediate I-15 above. MS [M+H] 312.

### Scheme G: Synthesis of intermediate I-30

### methyl (R)-1-(3-amino-3-(4-(5-fluoro-6-hydroxypyridin-3-yl)phenyl)propyl)piperidine-4-carboxylate (I-30)

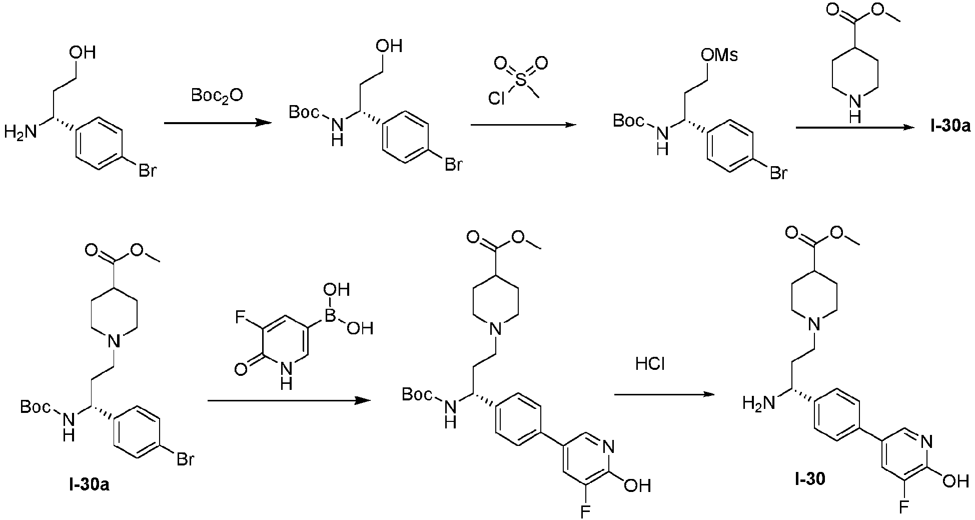

### Step 1 (R)-tert-butyl (1-(4-bromophenyl)-3-hydroxypropyl)carbamate

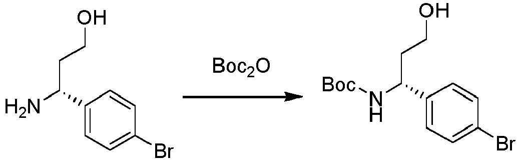

(R)-3-amino-3-(4-bromophenyl)propan-1-ol (2.48 g, 10.79 mmol) was put into a round bottom flask, dissolved in DCM (75 mL), and cooled to 0°C. Et₃N (3.80 mL, 27.3 mmol) was added to the flask followed by di-*tert*-butyl dicarbonate (3.57 g, 16.40 mmol). The reaction was warmed to RT overnight. To the reaction was added sat. NaHCO₃(aq.) and diluted with DCM. The layers were separated and the aqueous layer was extracted with DCM. The organic layers were combined, washed with brine, dried over Na₂SO₄, filtered and concentrated under reduced pressure. The residue was purified by silica gel chromatography (MeOH/DCM) to afford the title compound. MS: 332 (M+1).

### Step 2 (R)-3-(4-bromophenyl)-3-((tert-butoxycarbonyl)amino)propyl methane sulfonate

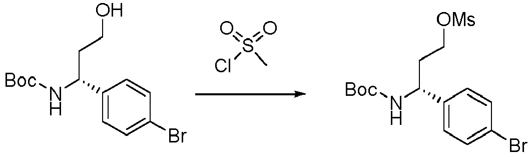

To a stirred solution of (R)-tert-butyl (1-(4-bromophenyl)-3-hydroxypropyl)carbamate (12.5 g, 37.9 mmol) in DCM (100 mL), TEA (15.83 mL, 114 mmol) and Ms-Cl (5.90 mL, 76 mmol) were added at 0°C. The reaction mixture was stirred at room temperature for 16 h. The reaction mixture was diluted with dichloromethane (50 mL) and H₂O (50 mL). The organic layer was washed with aqueous saturated NH₄Cl solution (50 mL), aqueous saturated NaHCO₃ solution (50 mL), brine solution (50 mL). The organic layer was dried over Na₂SO₄, filtered and concentrated to afford the title compound, which was used in next step without further purification. MS: 408 (M+1).

### Step 3 methyl (R)-1-(3-(4-bromophenyl)-3-((tert-butoxycarbonyl)amino)propyl)piperidine-4-carboxylate

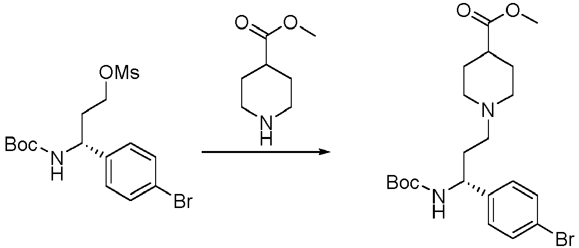

To a stirred solution of (R)-3-(4-bromophenyl)-3-((tert-butoxycarbonyl)amino)propyl methanesulfonate (15 g, 36.7 mmol)in acetonitrile (200 mL), methyl piperidine-4-carboxylate (19.85 mL, 147 mmol) was added at room temperature. The reaction mixture was stirred at 100°C for 2 hours. The reaction mixture was concentrated. The residue was diluted in dichloromethane (100 mL) and H₂0 (50 mL). The organic layer was washed with brine, dried over Na₂SO₄, filtered and concentrated under reduced pressure. The residue was purified by silica gel chromatography (MeOH/DCM) to afford the title compound. MS: 455 (M+1).

### Step 4 methyl (R)-1 -(3 -((tert-butoxy carbonyl)amino)-3 -(4-(5 -fluoro-6-hy droxypyridin-3 - yl)phenyl)propyl)piperidine-4-carboxylate

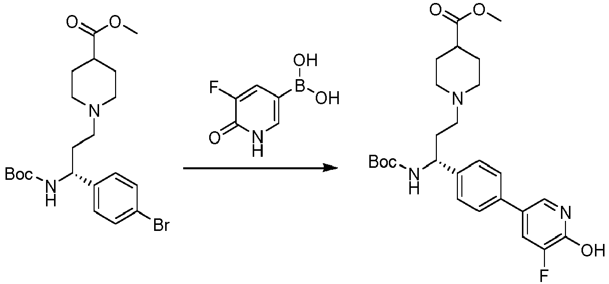

A mixture of Na₂CO₃ (4.05 g, 38.2 mmol) and PdCl₂(dppf)-CH₂Cl₂ adduct (1.041 g, 1.275 mmol) and (5-fluoro-6-oxo-1,6-dihydropyridin-3-yl)boronic acid (2 g, 12.75 mmol) and (R)-methyl 1-(3-(4-bromophenyl)-3-((tert-butoxycarbonyl)amino)propyl)piperidine-4-carboxylate (6.97 g, 15.30 mmol), 1,4-dioxane (40 mL) and water (4 mL) was stirred at 100°C for 1h. The reaction was cooled to RT, diluted with EtOAc and water. The organic layer was washed with brine, dried over Na₂SO₄, filtered and concentrated under reduced pressure. The residue was purified by silica gel chromatography (MeOH/DCM) to afford the title compound. MS: 488 (M+1).

### Step 5 methyl (R)-1-(3-amino-3-(-4-(5-fluoro-6-hydroxypyridin-3-yl)phenyl)propyl)piperidine 4-carboxylate (I-30)

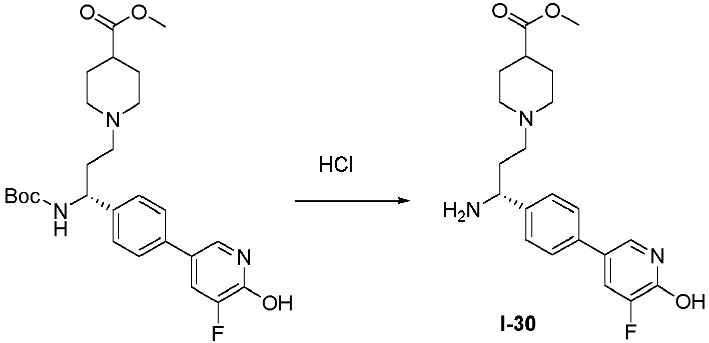

A stirred solution of (R)-methyl 1-(3-((tert-butoxycarbonyl)amino)-3-(4-(5-fluoro-6-hydroxypyridin-3-yl)phenyl)propyl)piperidine-4-carboxylate (500 mg, 1.026 mmol) in MeOH and DCM was added 4M HCl in 1,4-dioxane (8 mL). The mixture was stirred at RT under N₂ for 3 h, then concentrated to afford the title compound, **I-30.** MS: 388 (M+1).

### Synthesis of intermediate I-31 (S)-N-((R)-1-(3-((1-methylazetidin-3-yl)carbamoyl)phenyl)-3-oxopropyl)-7-(1-methylcyclopropyl)-5,6,7,8-tetrahydroacridine-2-carboxamide (I-31)

### Step 1 ethyl 3-((R)-3-hydroxy-1-((S)-7-(1-methylcyclopropyl)-5,6,7,8-tetrahydroacridine-2-carboxamido)propyl)benzoate

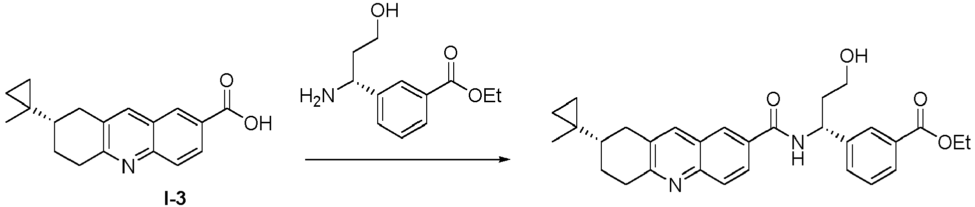

A mixture of (S)-7-(1-methylcyclopropyl)-5,6,7,8-tetrahydroacridine-2-carboxylic acid (**I-3**, 2.0 g, 7.11 mmol), (R)-ethyl 3-(1-amino-3-hydroxypropyl)benzoate (HCl salt, 2.4 g, 9.24 mmol), HATU (3.78 g, 9.95 mmol), was added anhydrous DMF (47.4 mL) and DIEA (4.97 mL, 28.4 mmol). The mixture was stirred at room temperature for 4 hs. Sat. aq. NaHCO₃ solution (200 mL) was added dropwise and the mixture was stirred for 10 min. The mixture was filtered and the filter cake was dried to afford the crude product, which was used directly in the next step without further purification. LC-MS 487 (M+1).

### Step 2 3-((R)-3-hydroxy-1-((S)-7-(1-methylcyclopropyl)-5,6,7,8-tetrahydroacridine-2-carboxamido)propyl)benzoic acid

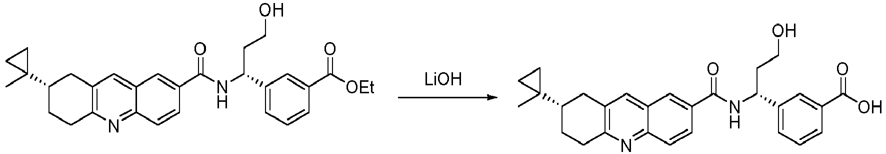

The title comopund (LC-MS 459 (M+1)) was prepared following analogous methodology to that outlined for intermediate **I-15** above.

### Step 3 (S)-N-((R)-3-hydroxy-1-(3-((1-methylazetidin-3-yl)carbamoyl)phenyl)propyl)-7-(1-methylcyclopropyl)-5,6,7,8-tetrahydroacridine-2-carboxamide

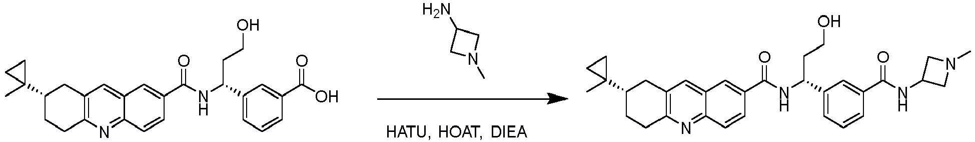

3-((R)-3-hydroxy-1-((S)-7-(1-methylcyclopropyl)-5,6,7,8-tetrahydroacridine-2-carboxamido)propyl)benzoic acid (1000 mg, 2.181 mmol), 1-methylazetidin-3-amine (oxalic acid salt, 960 mg, 5.45 mmol), HOAT (445 mg, 3.27 mmol) and HATU (1244 mg, 3.27 mmol) was added DMF (10 mL) followed by DIEA (1524 µL, 8.72 mmol). The reaction was stirred at RT overnight. The mixture was quenched with sat. aq. NaHCO₃ solution (100 mL), extracted with ethyl acetate (400 mL). The organic layer was washed with water (40 mL) twice, brine, dried over Na₂SO₄, filtered and concentrated to afford the title compound, which was used in the next step without further purification. MS: 527 (M+1).

### Step 4 (S)-N-((R)-1-(3-((1-methylazetidin-3-yl)carbamoyl)phenyl)-3-oxopropyl)-7-(1-methylcyclopropyl)-5,6,7,8-tetrahydroacridine-2-carboxamide (I-31)

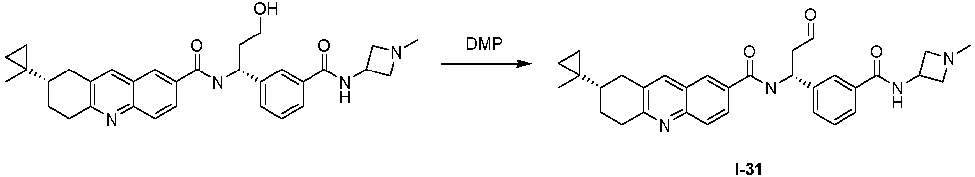

To a solution of (S)-N-((R)-3-hydroxy-1-(3-((1-methylazetidin-3-yl)carbamoyl)phenyl)propyl)-7-(1-methylcyclopropyl)-5,6,7,8-tetrahydroacridine-2-carboxamide (1100 mg, 2.089 mmol) in DCM (60 mL) at 0 °C was added DMP (1240 mg, 2.92 mmol). The reaction was stirred at RT for 2h. The reaction was diluted in DCM and quenched with sat. aqueous NaHCO₃ and sat. aqueous Na₂S₂O₃ solution. The mixture was stirred for 30 min. Two layers were separated. The organic layer was collected, dried over Na₂SO₄, filtered, and concentrated to afford the title compound, **I-31.** MS: 525 (M+1).

### Example 1

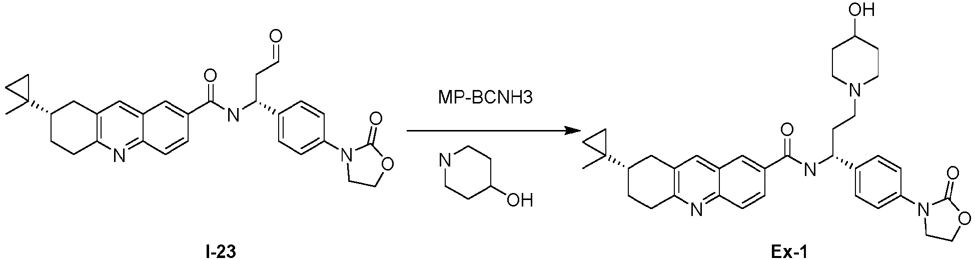

A solution of ((S)-7-(1-methylcyclopropyl)-N-((R)-3-oxo-1-(4-(2-oxooxazolidin-3-yl)phenyl)propyl)-5,6,7,8-tetrahydroacridine-2-carboxamide **(I-23,** 65 mg, 0.131 mmol), piperidin-4-ol (33.0 mg, 0.327 mmol) in MeOH (1306 µL) with 5% AcOH was stirred at RT for 0.5 h. Polymer-bound BH₃CN (2 equiv) was added. The mixture was stirred at RT overnight. The reaction was diluted with DMSO, neutralized with TFA, filtered and purified by reverse-phase HPLC (C18 column, MeCN/water with 0.1% TFA) to afford the product, **Ex. 1.**

| Ex. No. | Structure | Chemical Name | Mass [M+H] + | hNPRA EC50 (nM) |
|---|---|---|---|---|
| 1 | 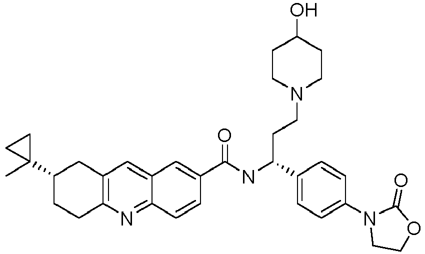 | (S)-N-((R)-3-(4-hydroxypiperidin-1-yl)-1-(4-(2-oxooxazolidin-3- yl)phenyl)propyl)-7-(1-methylcyclopropyl)-5,6,7,8-tetrahydroacridine-2-carboxamide | 583 | 701 |

Compound, Example 2 was prepared by following a analogous procedure as described for Example 1.

| Ex. No. | Structure | Chemical Name | Mass [M+H] + | hNPR A EC50 (nM) |
|---|---|---|---|---|
| 2 | 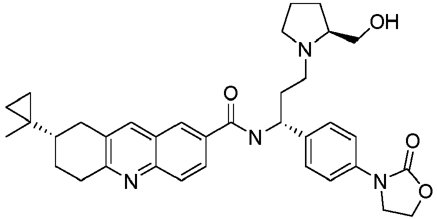 | (S)-N-((R)-3-((S)-2-(hydroxymethyl)pyrrolidin-1-yl)-1-(4-(2-oxooxazolidin-3-yl)phenyl)propyl)-7-(1-methylcyclopropyl)-5,6,7,8-etrahydroacridine-2-carboxamide | 583 | 1718 |

### Example 3

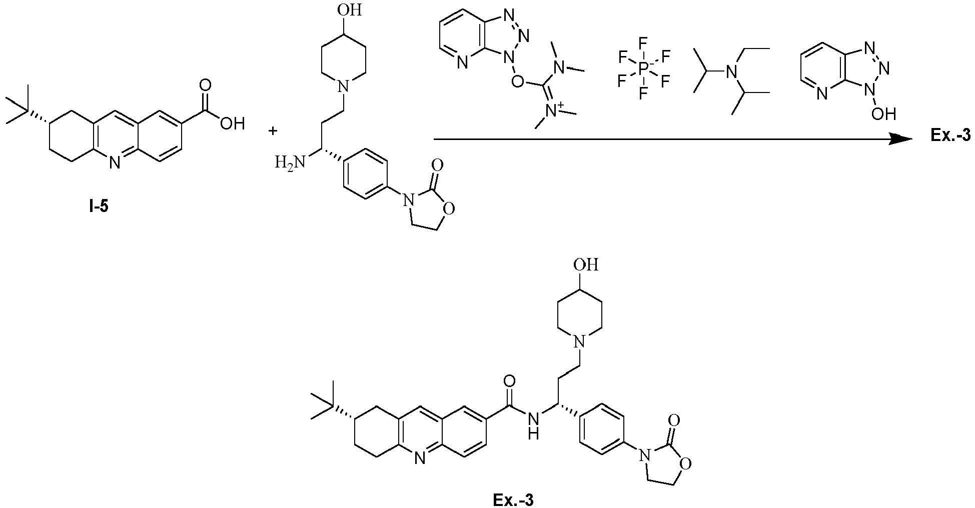

To a flask with (S)-7-(tert-butyl)-5,6,7,8-tetrahydroacridine-2-carboxylic acid **(I-5,** 18.06 mg, 0.064 mmol), (R)-3-(4-(1-amino-3-(4-hydroxypiperidin-1-yl)propyl)phenyl)oxazolidin-2-one (HCl salt, 25 mg, 0.064 mmol), HOAT (13.88 mg, 0.102 mmol) and HATU (38.8 mg, 0.102 mmol) was added DMF (1 mL) followed by DIEA (0.056 mL, 0.319 mmol). The reaction was stirred at RT overnight. The reaction mixture was filtered and purified by reverse-phase HPLC (C18 column, MeCN/water with 0.1% TFA) to afford the product, **Ex. 3.**

| Ex. No. | Structure | Chemical Name | Mass [M+H] + | hNPRA EC50 (nM) |
|---|---|---|---|---|
| 3 | 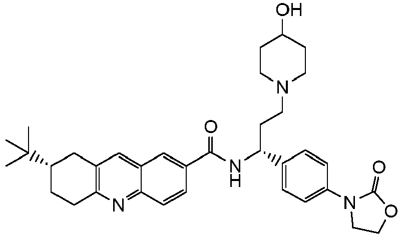 | (7S)-7-tert-butyl-N-{(1R)-3-(4-hydroxypiperidin-1-yl)-1-[4-(2-oxo-1,3-oxazoli din-3- yl)phenyl]propyl}-5,6,7,8-tetrahydroacridine-2-carboxamide | 585 | 753 |

**Examples 4-9** were prepared by following a similar procedure as is disclosed for **Example 3** by utilizing the appropriate tricyclic intermediate.

| Ex. No. | Structure | Chemical Name | Mass [M+H] + | hNPRA EC50 (nM) |
|---|---|---|---|---|
| 4 | 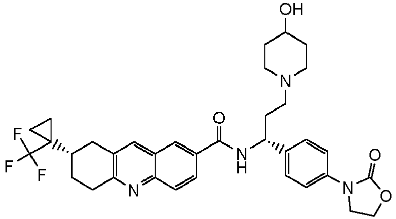 | (7S)-N-{(1R)-3-(4-hydroxypiperidin-1-yl)-1-[4-(2-oxo-1,3-oxazolidin-3-yl)phenyl]propyl}-7-[1-(trifluoromethyl)cyclopropyl ]-5,6,7,8-tetrahydroacridine-2-carboxamide | 637 | 2218 |
| 5 | 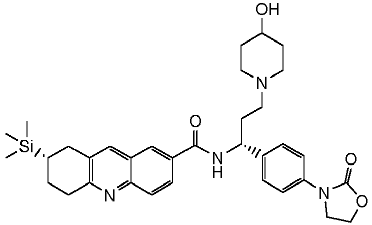 | (7S)-N-{(1R)-3-(4-hydroxypiperidin-1-yl)-1-[4-(2-oxo-1,3-oxazolidin-3-yl)phenyl]propyl}-7-(trimethylsilyl)-5,6,7,8-tetrahydroacridine-2-carboxamide | 601 | 1977 |
| 6 | 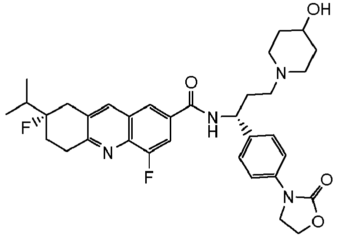 | (7S)-4,7-difluoro-N-{(1R)-3-(4-hydroxypiperidin-1-yl)-1-[4-(2-oxo-1,3-oxazoli din-3- yl)phenyl]propyl}-7-(1-methylethyl)-5,6,7,8-tetrahydroacridine-2-carboxamide | 607 | 114 |
| 7 | 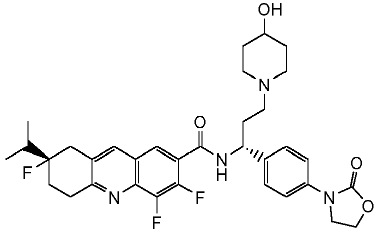 | (7S)-3,4,7-trifluoro-N-{(1R)-3-(4-hydroxypiperidin-1-yl)-1-[4-(2-oxo-1,3-oxazolidin-3-yl)phenyl]propyl}-7-(1-methylethyl)-5,6,7,8-tetrahydroacridine-2-carboxamide | 625 | 251 |
| 8 | 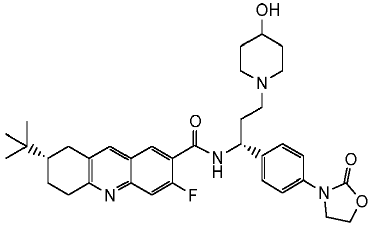 | 7-tert-butyl-3-fluoro-N-{(1R)-3-(4-hydroxypiperidin-1-yl)-1-[4-(2-oxo-1,3-oxazolidin-3-yl)phenyl]propyl}-5,6,7,8-tetrahydroacridine-2-carboxamide | 603 | 729 |
| 8 | 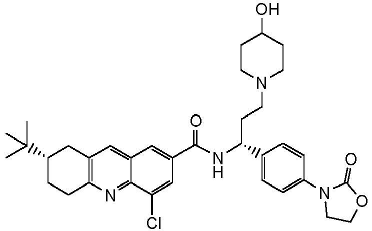 | (S)-7-(tert-butyl)-4-chloro-N-((R)-3-(4-hydroxypiperidin-1-yl)-1-(4-(2-oxooxazolidin-3-yl)phenyl)propyl)-5,6,7,8-tetrahydroacridine-2-carboxamide | 619 | 1271 |
| 9 | 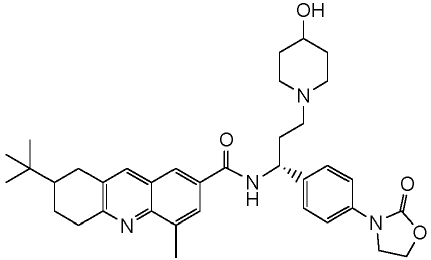 | 7-(tert-butyl)-N-((R)-3 -(4-hydroxypiperidin-1-yl)-1-(4-(2-oxooxazolidin-3-yl)phenyl)propyl)-4-methyl-5,6,7,8-tetrahydroacridine-2-carboxamide+ | 599 | 2552 |

### Example 10

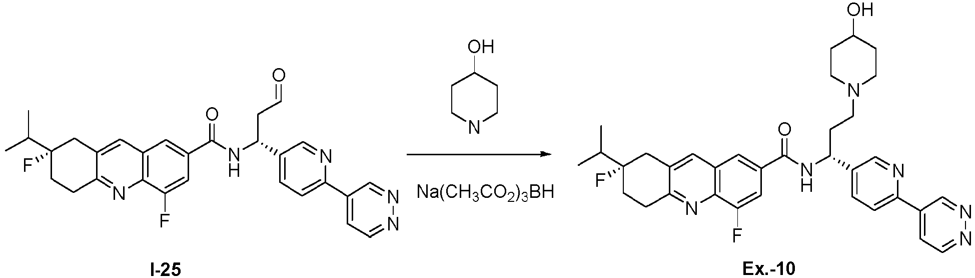

Piperidin-4-ol (8.83 mg, 0.087 mmol) and (S)-4,7-difluoro-7-isopropyl-N-((R)-3-oxo-1-(6-(pyridazin-4-yl)pyridin-3-yl)propyl)-5,6,7,8-tetrahydroacridine-2-carboxamide **(I-25,** 15 mg, 0.029 mmol) was dissolved in 5% acetic acid in DCE (2 mL). The mixture was stirred at RT for 30 min and then sodium triacetoxyborohydride (18.50 mg, 0.087 mmol) was added. The reaction mixture was stirred at RT overnight. The reaction is evaporated. The residue was diluted with DMSO, filtered and purified by purified by reverse-phase HPLC (C18 column, MeCN/water with 0.1% TFA) to afford the product, **Example 10.**

| Ex. No. | Structure | Chemical Name | Mass [M+H] + | hNPRA EC50 (nM) |
|---|---|---|---|---|
| 10 | 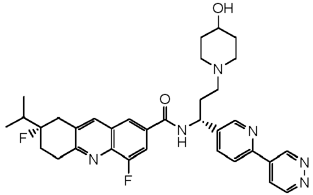 | (7S)-4,7-difluoro-N-[(1R)-3-(4-hydroxypiperidin-1-yl)-1-(6-pyridazin-4-ylpyridin-3-yl)propyl]-7-(1-methylethyl)-5,6,7,8-tetrahydroacridine-2-carboxamide | 601 | 112 |

### Examples 11-20

**Examples 11-20** were prepared by following a similar procedure of **Example 10** and by utilizing the appropriate tricyclic intermediate.

| Ex. No. | Structure | Chemical Name | Mass [M+H] + | hNPRA EC50 (nM) |
|---|---|---|---|---|
| 11 | 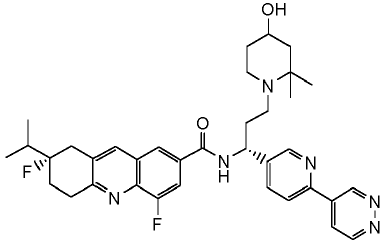 | (7S)-4,7-difluoro-N-[(1R)-3-(4-hydroxy-2,2-dimethylpiperidin-1-yl)-1-(6-pyridazin-4-ylpyridin-3-yl)propyl]-7-(1-methylethyl)-5,6,7,8-tetrahydroacridine-2-carboxamide | 629 | 219 |
| 12 | 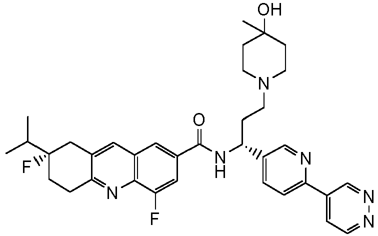 | (7S)-4,7-difluoro-N-[(1R)-3-(4-hydroxy-4-methylpiperidin-1-yl)-1- (6-pyridazin-4-ylpyridin-3-yl)propyl]-7-(1-methylethyl)-5,6,7,8-tetrahydroacridine-2-carboxamide | 615 | 205 |
| 13 | 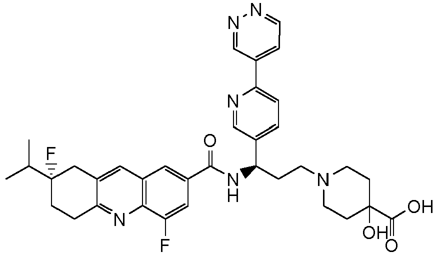 | 1-[(3R)-3-({[(7S)-4,7-difluoro-7-(1-methylethyl)-5,6,7,8-tetrahydroacridin-2-yl]carbonyl}amino)-3-(6-pyridazin-4-ylpyridin-3- yl)propyl]-4-hydroxypiperidine-4-carboxylic acid | 645 | 855 |
| 14 | 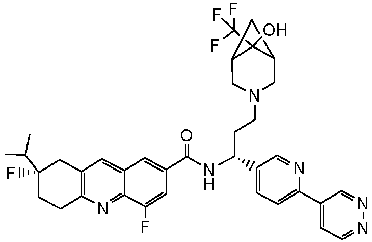 | (7S)-4,7-difluoro-N-[(1R)-3-[6-hydroxy-6-(trifluoromethyl)-3-azabicyclo[3.1.1]hept-3-yl]-1-(6-pyridazin-4-ylpyridin-3-yl)propyl]-7-(1-methylethyl)-5,6,7,8-tetrahydroacridine-2-carboxamide | 681 | 2090 |
| 15 | 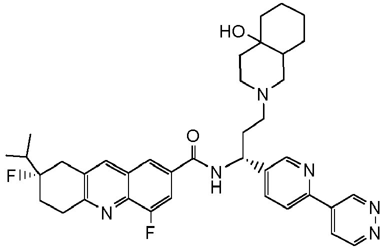 | (7S)-4,7-difluoro-N-[(1R)-3-(4a-hydroxyoctahydroisoquin olin-2(1H)-yl)-1-(6-pyridazin-4-ylpyridin-3- yl)propyl]-7-(1-methylethyl)-5,6,7,8-tetrahydroacridine-2-carboxamide | 655 | 257 |
| 16 | 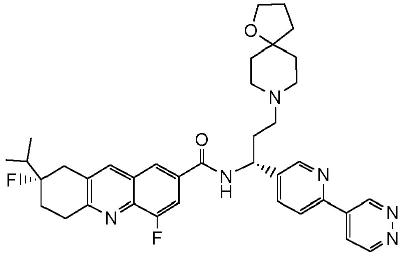 | (7S)-4,7-difluoro-7-(1-methylethyl)-N-[(1R)-3-(1-oxa-8-azaspiro[4.5]dec-8-yl)-1-(6-pyridazin-4-ylpyridin-3-yl)propyl]-5,6,7,8-tetrahydroacridine-2-carboxamide | 641 | 343 |
| 17 | 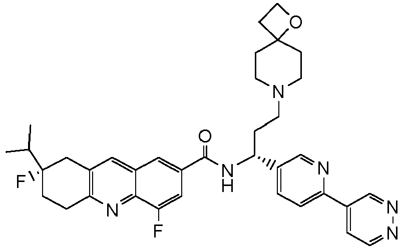 | (7S)-4,7-difluoro-7-(1-methylethyl)-N-[(1R)-3-(1-oxa-7-azaspiro[3.5]non-7-yl)-1-(6-pyridazin-4-ylpyridin-3-yl)propyl]-5,6,7,8-tetrahydroacridine-2-carboxamide | 627 | 217 |
| 18 | 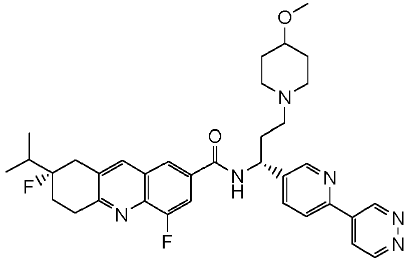 | (7S)-4,7-difluoro-N-[(1R)-3-(4-methoxypiperidin-1-yl)-1-(6-pyridazin-4-ylpyridin-3-yl)propyl]-7-(1-methylethyl)-5,6,7,8-tetrahydroacridine-2-carboxamide | 615 | 147 |
| 19 | 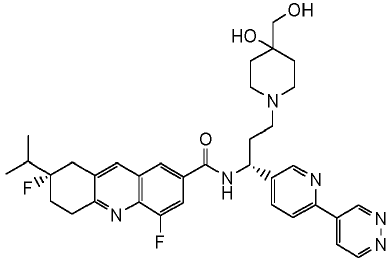 | (7S)-4,7-difluoro-N-[(1R)-3-[4-hydroxy-4-(hydroxymethyl)piperidin-1-yl]-1-(6-pyridazin-4-ylpyridin-3-yl)propyl]-7-(1-methylethyl)-5,6,7,8-tetrahydroacridine-2-carboxamide | 631 | 203 |
| 20 | 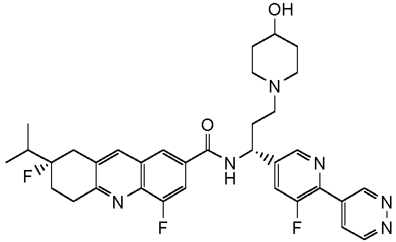 | (7S)-4,7-difluoro-N-[(1R)-1-(5-fluoro-6-pyridazin-4-ylpyridin-3- yl)-3-(4-hydroxypiperidin-1-yl)propyl]-7-(1-methylethyl)-5,6,7,8-tetrahydroacridine-2-carboxamide | 619 | 125 |

### Example 21

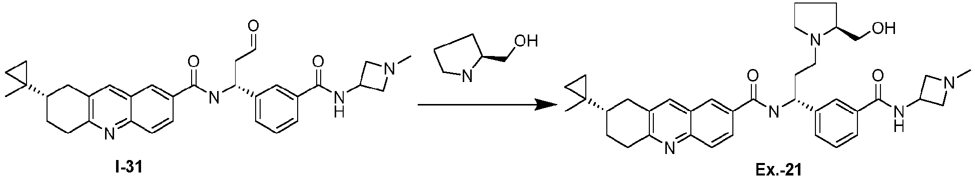

A solution of ((S)-N-((R)-1-(3-((1-methylazetidin-3-yl)carbamoyl)phenyl)-3-oxopropyl)-7-(1-methylcyclopropyl)-5,6,7,8-tetrahydroacridine-2-carboxamid (I-31, 60 mg, 0.114 mmol), (S)-pyrrolidin-2-ylmethanol (34.7 mg, 0.343 mmol) in MeOH (1144 µL) with 5% AcOH was stirred at RT for 0.5 h. Polymer-Bound BH₃CN (2 equiv.) was added, and the reaction mixture was stirred at RT overnight. The residue was diluted with DMSO, filtered and purified by purified by reverse-phase HPLC (C18 column, MeCN/water with 0.1% formic acid) to afford the product, **Ex. 21.**

| Ex. No. | Structure | Chemical Name | Mass [M+H] + | hNPRA EC50 (nM) |
|---|---|---|---|---|
| 21 | 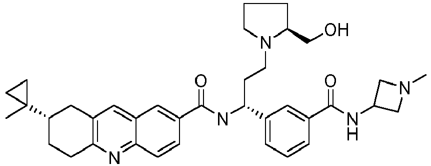 | (S)-N-((R)-3-((S)-2-(hydroxymethyl)pyrroli din-1-yl)-1-(3-((1-methylazetidin-3-yl)carbamoyl)phenyl)pr opyl)-7-(1-methylcyclopropyl)-5,6,7,8-tetrahydroacridine-2-carboxamide | 610 | 158 |

### Examples 22 and 23

**Examples 22 and 23** were prepared by following a similar procedure of **Example 21** and by utilizing the appropriate tricyclic intermediate.

| Ex. No. | Structure | Chemical Name | Mass [M+H] + | hNPRA EC50 (nM) |
|---|---|---|---|---|
| 22 | 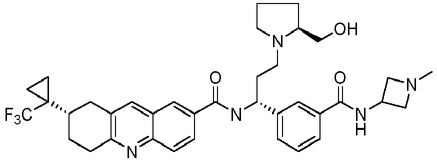 | (S)-N-((R)-3-((S)-2-(hydroxymethyl)pyrrol idin-1-yl)-1-(3-((1-methylazetidin-3-yl)carbamoyl)phenyl)p ropyl)-7-(1-(trifluoromethyl)cyclo propyl)-5,6,7,8-tetrahydroacridine-2-carboxamide | 664 | 278 |
| 23 | 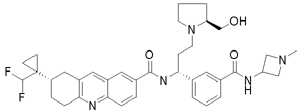 | ((S)-7-(1-(difluoromethyl)cyclo propyl)-N-((R)-3-((S)-2-(hydroxymethyl)pyrrol idin-1-yl)-1-(3-((1-methylazetidin-3-yl)carbamoyl)phenyl)p ropyl)-5,6,7,8-tetrahydroacridine-2-carboxamide | 646 | 405 |

### Example 24

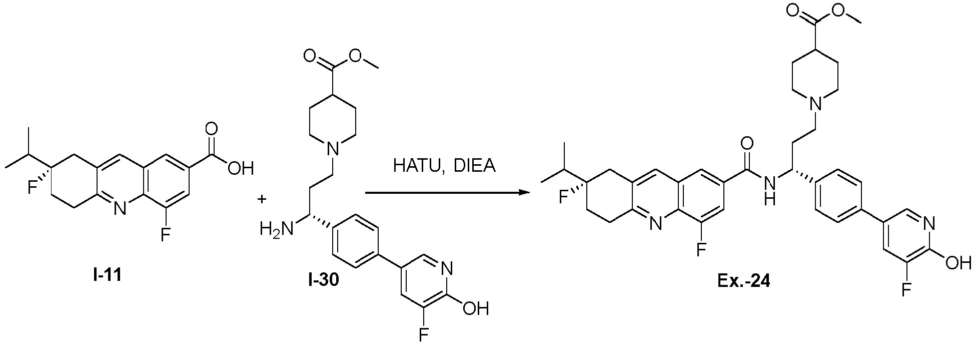

A mixture of HATU (74.7 mg, 0.197 mmol) and (S)-4,7-difluoro-7-isopropyl-5,6,7,8-tetrahydroacridine-2-carboxylic acid **(I-11,** 50 mg, 0.164 mmol) and methyl (R)-1-(3-amino-3-(4-(5-fluoro-6-hydroxypyridin-3-yl)phenyl)propyl)piperidine-4-carboxylate **(I-30,** 63 mg, 0.164 mmol) was added DMF (1 mL), followed by N-ethyl-N-isopropylpropan-2-amine (0.086 mL, 0.491 mmol). The mixture was stirred at rt for 30 mins, neutralized with TFA, filtered and purified by reverse-phase HPLC (C18 column, MeCN/water with 0.1% TFA) to afford the title compound, **Example 24.**

| Ex. No. | Structure | Chemical Name | Mass [M+H]+ | hNPRA EC50 (nM) |
|---|---|---|---|---|
| 24 | 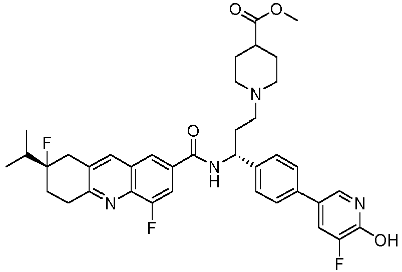 | methyl 1-((R)-3-((S)-4,7-difluoro-7-isopropyl-5,6,7,8-tetrahydroacridine-2-carboxamido)-3-(4-(5-fluoro-6-hydroxypyridin-3-yl)phenyl)propyl)piperidine-4-carboxylate | 675 | 237 |

### Example 25

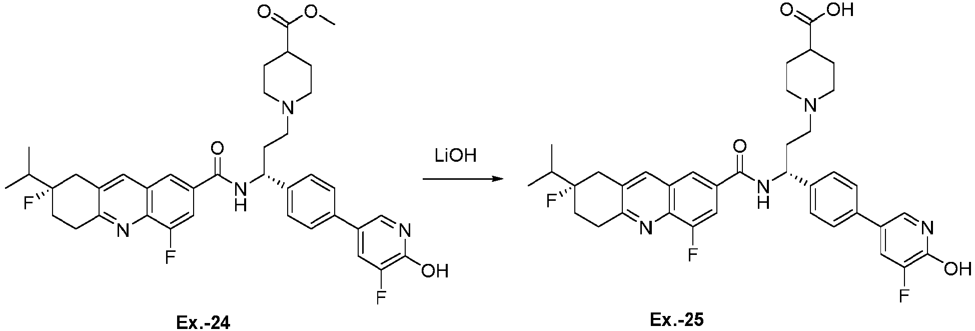

A mixture of LiOH (2.84 mg, 0.119 mmol) and methyl 1-((R)-3-((S)-4,7-difluoro-7-isopropyl-5,6,7,8-tetrahydroacridine-2-carboxamido)-3-(4-(5-fluoro-6-hydroxypyridin-3-yl)phenyl)propyl)piperidine-4-carboxylate **(Ex. 24,** 40 mg, 0.059 mmol) was added MeOH (1 mL), followed by water (0.2 mL). The mixture was stirred at rt for 30 mins, neutralized with TFA, filtered and purified by reverse-phase HPLC (C18 column, MeCN/water with 0.1% TFA) to afford the title compound, **Example 25.**

| Ex. No. | Structure | Chemical Name | Mass [M+H] + | hNPRA EC50 (nM) |
|---|---|---|---|---|
| 25 | 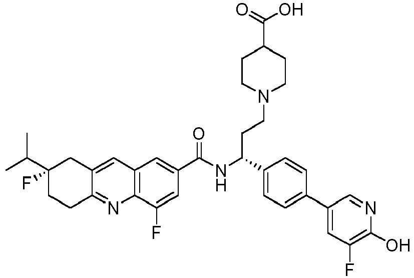 | 1-((R)-3-((S)-4,7-difluoro-7-isopropyl-5,6,7,8-tetrahydroacridine-2-carboxamido)-3-(4-(5-fluoro-6-hydroxypyridin-3-yl)phenyl)propyl)piperidin e-4-carboxylic acid | 661 | 167 |

### Example 26

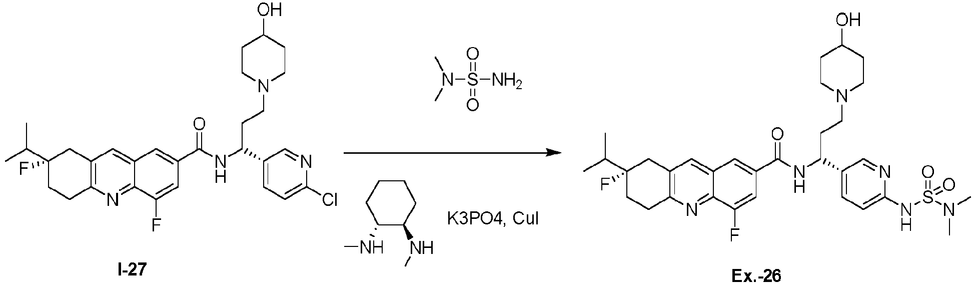

(S)-N-((R)-1-(6-chloropyridin-3-yl)-3-(4-hydroxypiperidin-1-yl)propyl)-4,7-difluoro-7-isopropyl-5,6,7,8-tetrahydroacridine-2-carboxamide **(I-27,** 70 mg, 0.126 mmol), N,N-dimethylsulfamide (62.4 mg, 0.503 mmol), potassium phosphate (107 mg, 0.503 mmol), CuI (23.93 mg, 0.126 mmol), and (1R,2R)-N1,N2-dimethylcyclohexane-1,2-diamine (39.6 µL, 0.251 mmol) were added together in dioxane (1257 µL). The mixture was flushed under N₂ for 2 min. The reaction was stirred at 100°C for 2 days then cooled down to RT and filtered through a Celite^{®} pad. The filtrate was concentrated. The residue was purified by reverse-phase HPLC (C18 column, MeCN/water with 0.1% TFA) to afford the title compound, **Example 26.**

| Ex. No. | Structure | Chemical Name | Mass [M+H] + | hNPRA EC50 (nM) |
|---|---|---|---|---|
| 26 | 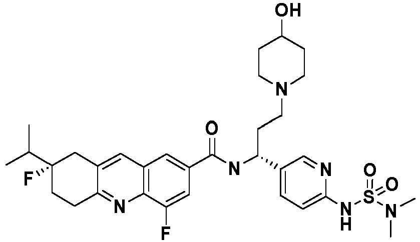 | (S)-N-((R)-1-(6-((N,N-dimethylsulfamoyl)amin o)pyridin-3-yl)-3-(4-hydroxypiperidin-1-yl)propyl)-4,7-difluoro-7-isopropyl-5,6,7,8-tetrahydroacridine-2-carboxamide | 645 | 246 |

### Example 27

**Example 27** was prepared by following a similar procedure to that shown in **Example 26.**

| Ex. No. | Structure | Chemical Name | Mass [M+H] + | hNPRA EC50 (nM) |
|---|---|---|---|---|
| 27 | 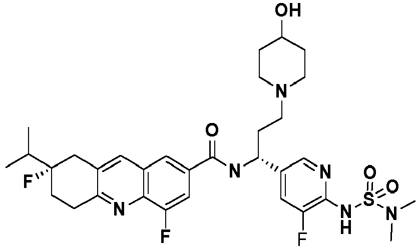 | (S)-N-((R)-1-(6-((N,N-dimethylsulfamoyl)amin o)-5-fluoropyridin-3-yl)-3-(4-hydroxypiperidin-1-yl)propyl)-4,7-difluoro-7-isopropyl-5,6,7,8-tetrahydroacridine-2-carboxamide | 662 | 2450 |

### Example 28

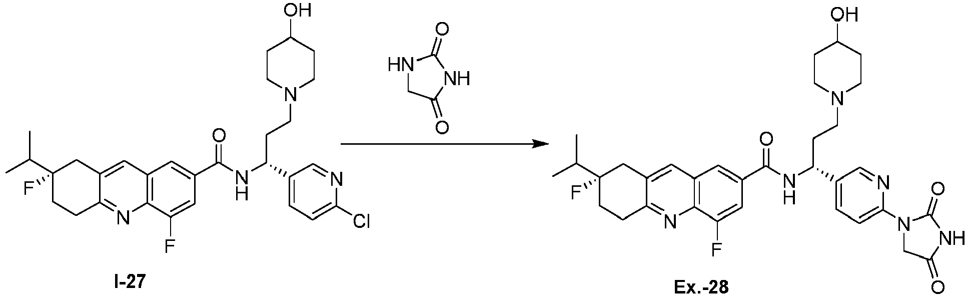

A mixture of (S)-N-((R)-1-(6-chloropyridin-3-yl)-3-(4-hydroxypiperidin-1-yl)propyl)-4,7-difluoro-7-isopropyl-5,6,7,8-tetrahydroacridine-2-carboxamide **(I-27,** 50.1 mg, 0.090 mmol), imidazolidine-2,4-dione (18 mg, 0.180 mmol), xantphos (20.81 mg, 0.036 mmol), Pd(OAc)₂ (4.04 mg, 0.018 mmol) and Cs₂CO₃ (58.6 mg, 0.180 mmol) in dioxane (500 µL) was flushed with N₂ three times. The mixture was stirred at 100 °C in a sealed vial for 5 hs. The reaction mixture was cooled to RT, diluted with DMSO, filtered and purified by reverse-phase HPLC (C18 column, MeCN/water with 0.1% TFA) to afford the title compound, **Example 28.**

| Ex. No. | Structure | Chemical Name | Mass [M+H] + | hNPRA EC50 (nM) |
|---|---|---|---|---|
| 28 | 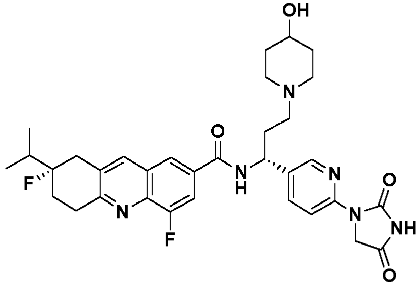 | (S)-N-((R)-1-(6-(2,4-dioxoimidazolidin-1-yl)pyridin-3-yl)-3-(4-hydroxypiperidin-1-yl)propyl)-4,7-difluoro-7-isopropyl-5,6,7,8-tetrahydroacridine-2-carboxamide | 621 | 321 |

### Example 29

Example 29 was prepared by following a similar procedure of of that disclosed in **Example 28**.

| Ex. No. | Structure | Chemical Name | Mass [M+H] + | hNPRA EC50 (nM) |
|---|---|---|---|---|
| 29 | 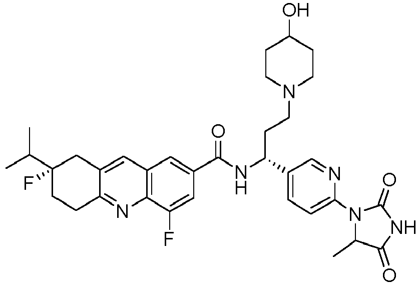 | (7S)-4,7-difluoro-N-((1R)-3-(4-hydroxypiperidin-1-yl)-1-(6-(5-methyl-2,4-dioxoimidazolidin-1-yl)pyridin-3-yl)propyl)-7-isopropyl-5,6,7,8-tetrahydroacridine-2-carboxamide | 635 | 275 |

### Example 30

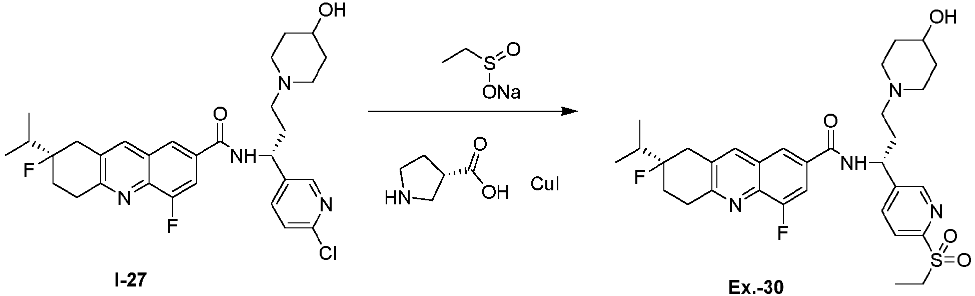

A mixture of (S)-N-((R)-1-(6-chloropyridin-3-yl)-3-(4-hydroxypiperidin-1-yl)propyl)-4,7-difluoro-7-isopropyl-5,6,7,8-tetrahydroacridine-2-carboxamide (I-27, 60 mg, 0.108 mmol), (S)-pyrrolidine-3-carboxylic acid (20 mg, 0.174 mmol), CuI (14 mg, 0.074 mmol), sodium ethanesulfinate (40 mg, 0.344 mmol) was added DMSO (1077 µL). After flushing with N₂, the reaction mixture was stirred at 120°C for 24h. The reaction was cooled to RT, and filtered through a celite. The filter cake was washed with 5 mL EtOAc and 5 mL MeOH. The filtrate was concentrated. The residue was purified by reverse-phase HPLC (C18 column, MeCN/water with 0.1% TFA) to afford the title compound, **Example 30.**

| Ex. No. | Structure | Chemical Name | Mass [M+H] + | hNPRA EC50 (nM) |
|---|---|---|---|---|
| 30 | 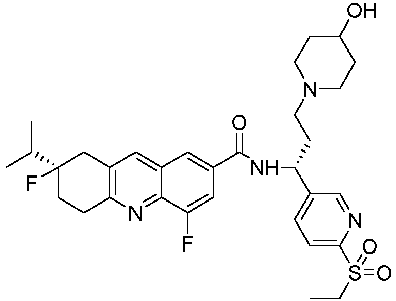 | (S)-N-((R)-1-(6-(ethylsulfonyl)pyridin-3-yl)-3-(4-hydroxypiperidin-1-yl)propyl)-4,7-difluoro-7-isopropyl-5,6,7,8-tetrahydroacridine-2-carboxamide | 615 | 970 |

### Example 31

Example 31 was prepared by following a similar procedure to that disclosed in Example 3.

| Ex. No. | Structure | Chemical Name | Mass [M+H] + | hNPRA EC50 (nM) |
|---|---|---|---|---|
| 31 | 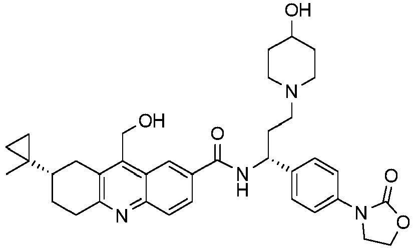 | (S)-9-(hydroxymethyl)-N-((R)-3-(4-hydroxypiperidin-1-yl)-1-(4-(2-oxooxazolidin-3-yl)phenyl)propyl)-7-(1-methylcyclopropyl)-5,6,7,8-tetrahydroacridine-2-carboxamide | 613 | 7188 |

### Example 32

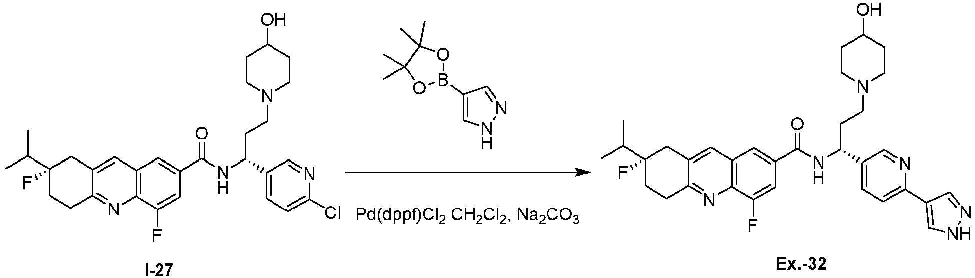

A mixture of (S)-N-((R)-1-(6-chloropyridin-3-yl)-3-(4-hydroxypiperidin-1-yl)propyl)-4,7-difluoro-7-isopropyl-5,6,7,8-tetrahydroacridine-2-carboxamide (I-27, 30 mg, 0.054 mmol), Pd(dppf)Cl₂ (8.80 mg, 10.77 µmol), 4-(4,4,5,5-tetramethyl-1,3,2-dioxaborolan-2-yl)-1H-pyrazole (20.90 mg, 0.108 mmol) was evacuated under vacuum and backfill with nitrogen (3x). Then dioxane (979 µL) and water (98 µL), 2M aq. Na₂CO₃ (64.6 µL, 0.162 mmol) was added. The mixture was evacuated under vacuum and backfill with nitrogen (3x). The reaction was stirred at 100°C overnight. The mixture was then cooled to rt, diluted with DMSO, neutralized with formic acid, filtered and purified by reverse-phase HPLC (C18 column, MeCN/water with 0.1% TFA) to afford the title compound, **Example 32.**

| Ex. No. | Structure | Chemical Name | Mass [M+H] + | hNPRA EC50 (nM) |
|---|---|---|---|---|
| 32 | 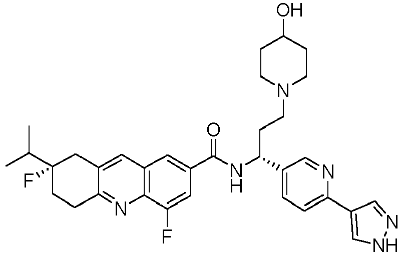 | (S)-N-((R)-1-(6-(1H-pyrazol-4-yl)pyridin-3-yl)-3-(4-hydroxypiperidin-1-yl)propyl)-4,7-difluoro-7-isopropyl-5,6,7,8-tetrahydroacridine-2-carboxamide | 589 | 217 |

### Example 33

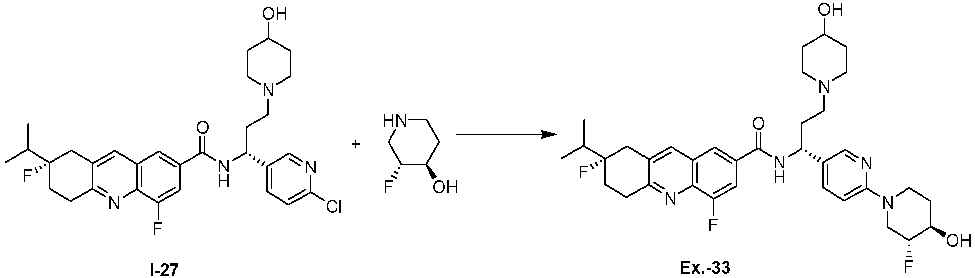

A mixture of Pd(dba)2 (8.83 mg, 9.64 µmol), Xantphos (11.14 mg, 0.019 mmol), (S)-N-((R)-1-(6-chloropyridin-3-yl)-3-(4-hydroxypiperidin-1-yl)propyl)-4,7-difluoro-7-isopropyl-5,6,7,8-tetrahydroacridine-2-carboxamide **(I-27,** 35.8 mg, 0.064 mmol), (3R,4R)-3-fluoropiperidin-4-ol (HCl salt, 30 mg, 0.193 mmol), Cs₂CO₃ (104 mg, 0.321 mmol) was flushed with N₂ three times. Then Dixoane (2 mL) was added. The mixture was flushed with N₂ three times again and then stirred at 100 ^{O}C for 16 hrs. The reaction mixture was filtered and purified by reverse-phase HPLC (C18 column, MeCN/water with 0.1% TFA) to afford the title compound, **Example 33.**

| Ex. No. | Structure | Chemical Name | Mass [M+H] + | hNPRA EC50 (nM) |
|---|---|---|---|---|
| 33 | 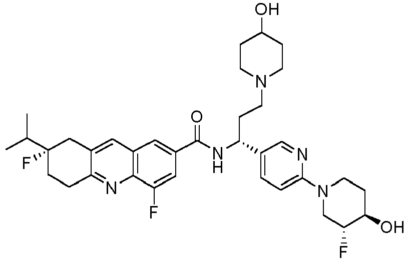 | (S)-4,7-difluoro-N-((R)-1-(6-((3R,4R)-3-fluoro-4-hydroxypiperidin-1-yl)pyridin-3-yl)-3-(4-hydroxypiperidin-1-yl)propyl)-7-isopropyl-5,6,7,8-tetrahydroacridine-2-carboxamide | 640 | 871 |

### Example 34

**Example 34** was prepared by following a similar procedure of that disclosed in **Example 33.**

| Ex. No. | Structure | Chemical Name | Mass [M+ H]+ | hNPRA EC50 (nM) |
|---|---|---|---|---|
| 34 | 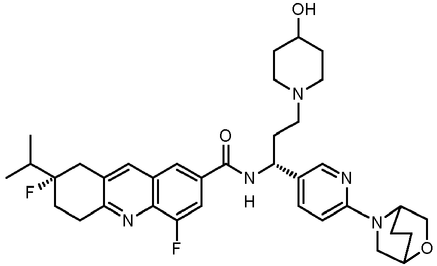 | (7S)-N-((1R)-1-(6-(2-oxa-5-azabicyclo[2.2.2]octan-5-yl)pyridin-3-yl)-3 -(4-hydroxypiperidin-1-yl)propyl)-4,7-difluoro-7-isopropyl-5,6,7,8-tetrahydroacridine-2-carboxamide | 634 | 2838 |

### Example 35 through Example 64

**Example 35** through **Example 64** were synthesized by a similar procedure as below:

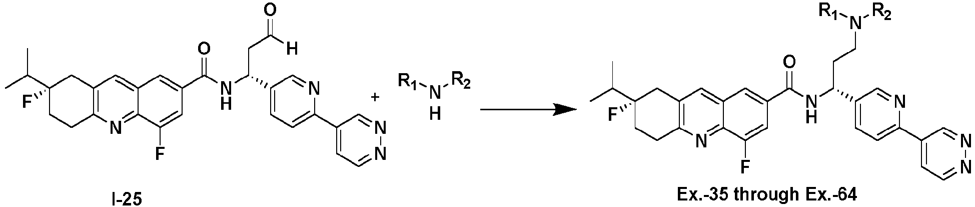

To a solution of (S)-4,7-difluoro-7-isopropyl-N-((R)-3-oxo-1-(6-(pyridazin-4-yl)pyridin-3-yl)propyl)-5,6,7,8-tetrahydroacridine-2-carboxamide **(I-25,** 17 mg, 0.033 mmol) and amino substrate (0.033 mmol) in 5% HOAc / MeOH (1 mL) was added polystyrene supported BH₃CN (70 mg, 2.45 mmol/g). The resulting reaction mixture was shaken at ambient temperature for 16 hours. The reaction was filtered and the solvent was evaporated in vacuum. The crude product was purified by reverse-phase HPLC (MeCN / water with 0.1% TFA as modifier) to give the product as a TFA salt.

| Ex. No. | Structure | Chemical Name | Mass [M+H] ⁺ | hNPRA EC₅₀ (nM) |
|---|---|---|---|---|
| **35** | 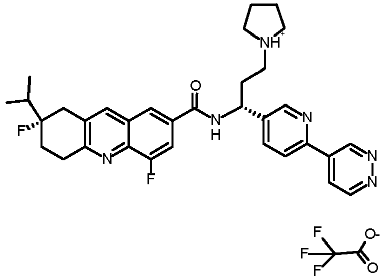 | rac-(7S)-4,7-difluoro-7-isopropyl-N-[rac-(1R)-1-(6-pyridazin-4-yl-3-pyridyl)-3-pyrrolidin-1-ium-1-yl-propyl]-6,8-dihydro-5H-acridine-2-carboxamide;2,2,2-trifluoroacetate | 571.08 | 285 |
| **36** | 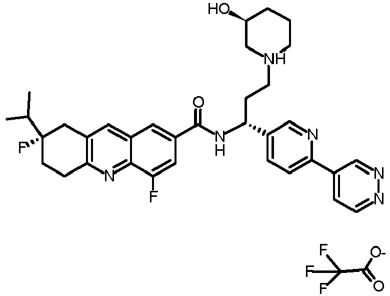 | rac-(7S)-4,7-difluoro-7-isopropyl-N-[rac-(1R)-1-(6-pyridazin-4-yl-3-pyridyl)-3-[rac-(3S)-3-hydroxypiperidin-1-ium-1-yl]propyl]-6,8-dihydro-5H-acridine-2-carboxamide;2,2,2-trifluoroacetate | 601.06 | 260.2 |
| **37** | 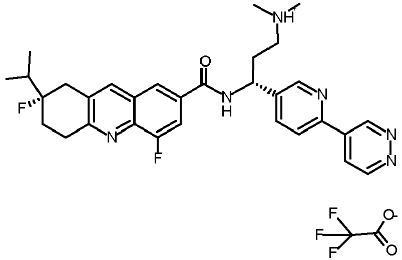 | dimethyl-[rac-(3R)-3- (6-pyridazin-4-yl-3-pyridyl)-3-[[rac-(7S)-4,7-difluoro-7-isopropyl-6,8-dihydro-5H-acridine-2-carbonyl]amino]propy l]ammonium;2,2,2-trifluoroacetate | 545.11 | 269.9 |
| **38** | 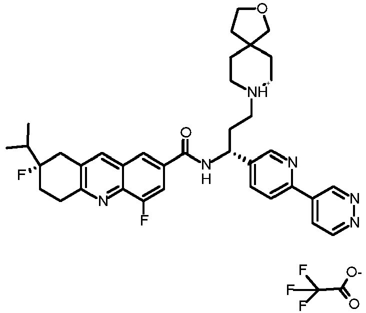 | rac-(7S)-4,7-difluoro-7-isopropyl-N-[rac-(1R)-3-(2-oxa-8-azoniaspiro[4.5]decan-8-yl)-1-(6-pyridazin-4-yl-3-pyridyl)propyl]-6,8-dihydro-5H-acridine-2-carboxamide;2,2,2-trifluoroacetate | 641.10 | 317 |
| **39** | 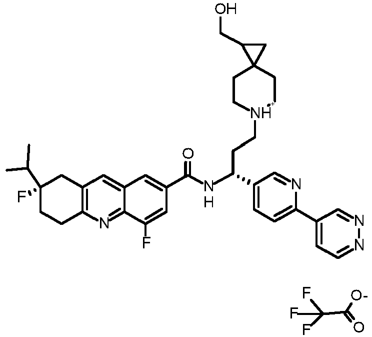 | rac-(7S)-4,7-difluoro-7-isopropyl-N-[rac-(1R)-3-[2-(hydroxymethyl)-6-azoniaspiro[2.5]octan-6-yl]-1-(6-pyridazin-4-yl-3-pyridyl)propyl]-6,8-dihydro-5H-acridine-2-carboxamide;2,2,2-trifluoroacetate | 641.20 | 258.3 |
| **40** | 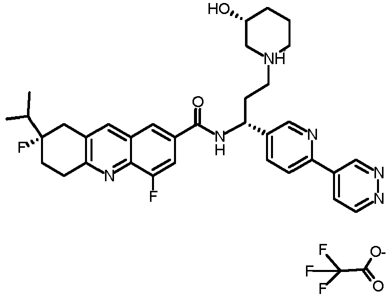 | rac-(7S)-4,7-difluoro-7-isopropyl-N-[rac-(1R)-1-(6-pyridazin-4-yl-3-pyridyl)-3-[rac-(3R)-3-hydroxypiperidin-1-ium-1-yl]propyl]-6,8-dihydro-5H-acridine-2-carboxamide;2,2,2-trifluoroacetate | 601.33 | 338.3 |
| **41** | 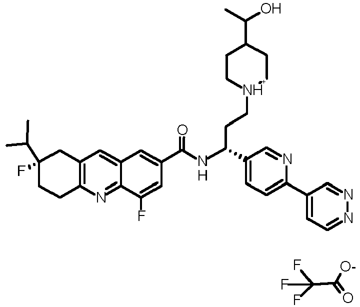 | rac-(7S)-4,7-difluoro-7-isopropyl-N-[rac-(1R)-3-[4-(1-hydroxyethyl)piperidi n-1-ium-1-yl]-1-(6-pyridazin-4-yl-3-pyridyl)propyl]-6,8-dihydro-5H-acridine-2-carboxamide;2,2,2-trifluoroacetate | 629.12 | 235.9 |
| **42** | 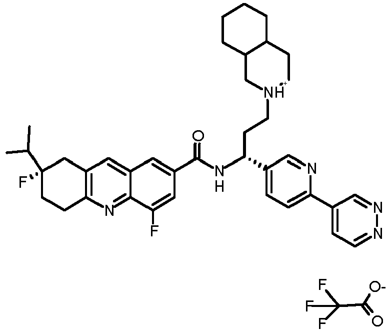 | rac-(7S)-4,7-difluoro-7-isopropyl-N-[rac-(1R)-3-(1,2,3,4,4a,5,6,7,8,8a-decahydroisoquinolin-2-ium-2-yl)-1-(6-pyridazin-4-yl-3-pyridyl)propyl]-6,8-dihydro-5H-acridine-2-carboxamide;2,2,2-trifluoroacetate | 639.30 | 375 |
| **43** | 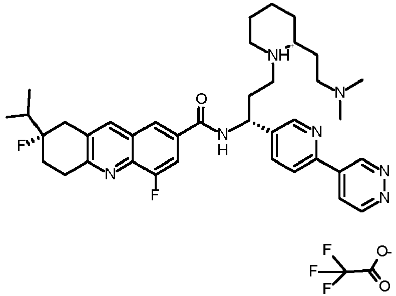 | rac-(7S)-4,7-difluoro-7-isopropyl-N-[rac-(1R)-3-[2-[2-(dimethylamino)ethyl] piperidin-1-ium-1-yl]-1-(6-pyridazin-4-yl-3-pyridyl)propyl]-6,8-dihydro-5H-acridine-2-carboxamide;2,2,2-trifluoroacetate | 656.34 | 215.9 |
| **44** | 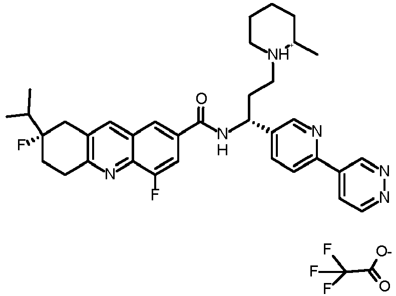 | rac-(7S)-4,7-difluoro-7-isopropyl-N-[rac-(1R)-3-(2-methylpiperidin-1-ium-1-yl)-1-(6-pyridazin-4-yl-3-pyridyl)propyl]-6,8-dihydro-5H-acridine-2-carboxamide;2,2,2-trifluoroacetate | 599.10 | 150 |
| **45** | 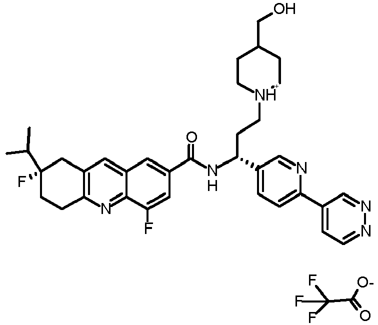 | rac-(7S)-4,7-difluoro-7-isopropyl-N-[rac-(1R)-3-[4-(hydroxymethyl)piperi din-1-ium-1-yl]-1-(6-pyridazin-4-yl-3-pyridyl)propyl]-6,8-dihydro-5H-acridine-2-carboxamide;2,2,2-trifluoroacetate | 615.26 | 136.5 |
| **46** | 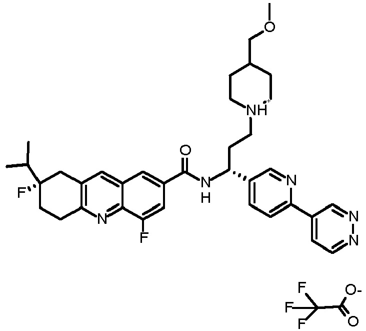 | rac-(7S)-4,7-difluoro-7-isopropyl-N-[rac-(1R)-3-[4-(methoxymethyl)piper idin-1-ium-1-yl]-1-(6-pyridazin-4-yl-3-pyridyl)propyl]-6,8-dihydro-5H-acridine-2-carboxamide;2,2,2-trifluoroacetate | 629.31 | 204.2 |
| **47** | 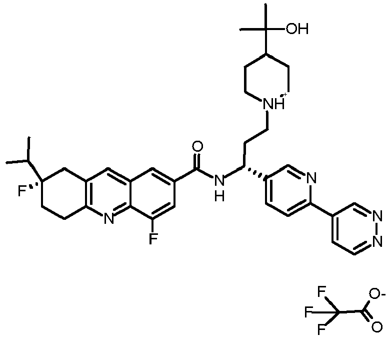 | rac-(7S)-4,7-difluoro-7-isopropyl-N-[rac-(1R)-3-[4-(1-hydroxy-1-methylethyl)piperidin-1-ium-1-yl]-1-(6-pyridazin-4-yl-3-pyridyl)propyl]-6,8-dihydro-5H-acridine-2-carboxamide;2,2,2-trifluoroacetate | 643.23 | 188.4 |
| **48** | 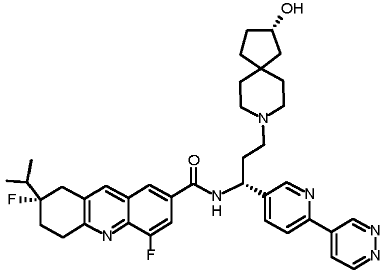 | rac-(7S)-4,7-difluoro-7-isopropyl-N-[rac-(1R)-1-(6-pyridazin-4-yl-3-pyridyl)-3 -[rac-(3S)-3-hydroxy-8-azaspiro[4.5]decan-8-yl]propyl]-6,8-dihydro-5H-acridine-2-carboxamide | 655.20 | 105.5 |
| **49** | 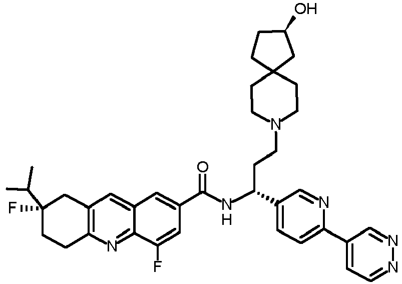 | rac-(7S)-4,7-difluoro-7-isopropyl-N-[rac-(1R)-1-(6-pyridazin-4-yl-3-pyridyl)-3 -[rac-(3R)-3-hydroxy-8-azaspiro[4.5]decan-8-yl]propyl]-6,8-dihydro-5H-acridine-2-carboxamide | 655.20 | 98.51 |
| **50** | 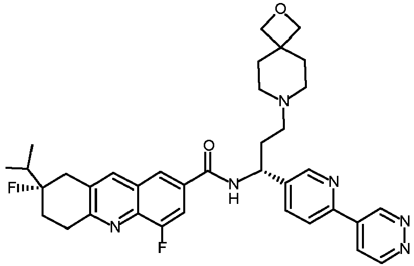 | rac-(7S)-4,7-difluoro-7-isopropyl-N-[rac-(1R)-3-(2-oxa-7-azaspiro[3.5]nonan-7-yl)-1-(6-pyridazin-4-yl-3-pyridyl)propyl]-6,8-dihydro-5H-acridine-2-carboxamide | 627.17 | 240 |
| **51** | 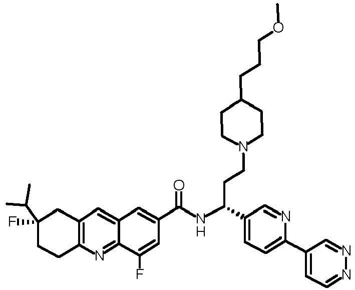 | rac-(7S)-4,7-difluoro-7-isopropyl-N-[rac-(1R)-3-[4-(3-methoxypropyl)-1-piperidyl]-1-(6-pyridazin-4-yl-3-pyridyl)propyl]-6,8-dihydro-5H-acridine-2-carboxamide | 657.26 | 174.6 |
| **52** | 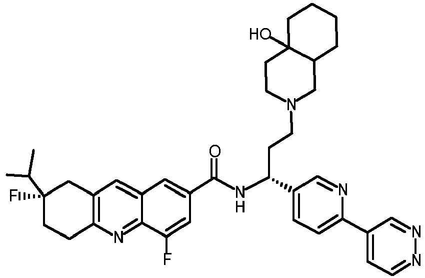 | rac-(7S)-4,7-difluoro-7-isopropyl-N-[rac-(1R)-3-(4a-hydroxy-1,3,4,5,6,7,8,8a-octahydroisoquinolin-2-yl)-1-(6-pyridazin-4-yl-3-pyridyl)propyl]-6,8-dihydro-5H-acridine-2-carboxamide | 655.13 | 257.7 |
| **53** | 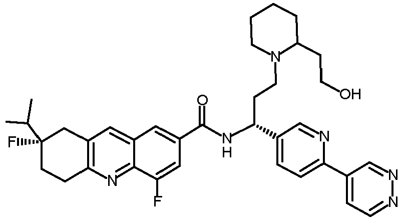 | rac-(7S)-4,7-difluoro-7-isopropyl-N-[rac-(1R)-3-[2-(2-hydroxyethyl)-1-piperidyl]-1-(6-pyridazin-4-yl-3-pyridyl)propyl]-6,8-dihydro-5H-acridine-2-carboxamide | 629.11 | 98.13 |
| **54** | 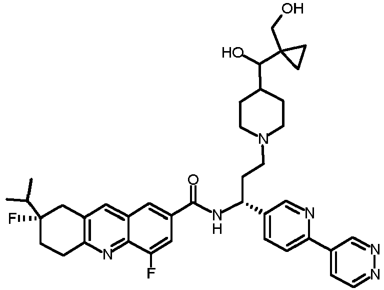 | rac-(7S)-4,7-difluoro-7-isopropyl-N-[rac-(1R)-3-[4-[hydroxy-[1-(hydroxymethyl)cyclo propyl]methyl]-1-piperidyl]-1-(6-pyridazin-4-yl-3-pyridyl)propyl]-6,8-dihydro-5H-acridine-2-carboxamide | 685.30 | 91.14 |
| **55** | 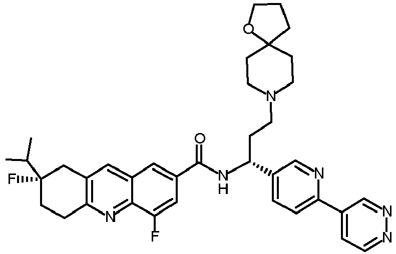 | rac-(7S)-4,7-difluoro-7-isopropyl-N-[rac-(1R)-3-(1-oxa-8-azaspiro[4.5]decan-8-yl)-1-(6-pyridazin-4-yl-3-pyridyl)propyl]-6,8-dihydro-5H-acridine-2-carboxamide | 641.16 | 343.9 |
| **56** | 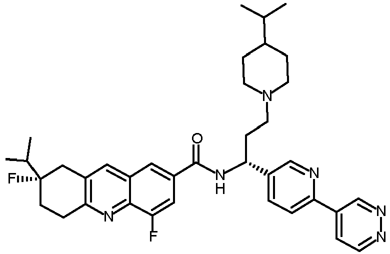 | rac-(7S)-4,7-difluoro-7-isopropyl-N-[rac-(1R)-3-(4-isopropyl-1-piperidyl)-1-(6-pyridazin-4-yl-3-pyridyl)propyl]-6,8-dihydro-5H-acridine-2-carboxamide | 627.30 | 257.7 |
| **57** | 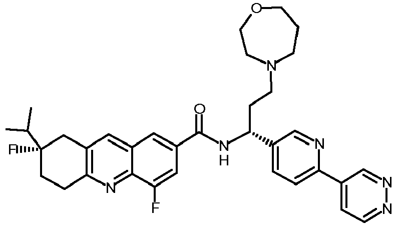 | rac-(7S)-4,7-difluoro-7-isopropyl-N-[rac-(1R)-3-(1,4-oxazepan-4-yl)-1-(6-pyridazin-4-yl-3-pyridyl)propyl]-6,8-dihydro-5H-acridine-2-carboxamide | 601.12 | 142.3 |
| **58** | 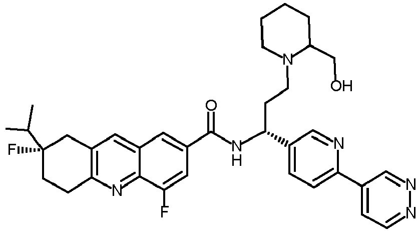 | rac-(7S)-4,7-difluoro-7-isopropyl-N-[rac-(1R)-3-[2-(hydroxymethyl)-1-piperidyl]-1-(6-pyridazin-4-yl-3-pyridyl)propyl]-6,8-dihydro-5H-acridine-2-carboxamide | 615.18 | 79.7 |
| **59** | 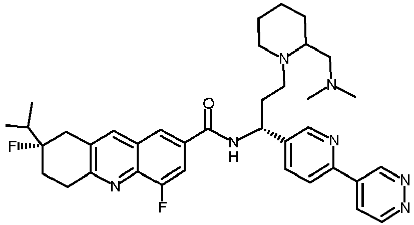 | rac-(7S)-4,7-difluoro-7-isopropyl-N-[rac-(1R)-3-[2-[(dimethylamino)meth yl]-1-piperidyl]-1-(6-pyridazin-4-yl-3-pyridyl)propyl]-6,8-dihydro-5H-acridine-2-carboxamide | 642.14 | 434 |
| **60** | 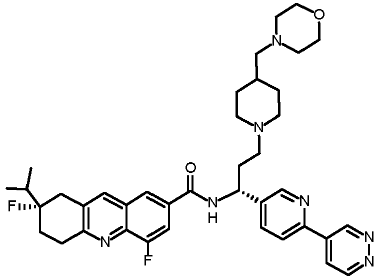 | rac-(7S)-4,7-difluoro-7-isopropyl-N-[rac-(1R)-3-[4-(morpholinomethyl)-1-piperidyl]-1-(6-pyridazin-4-yl-3-pyridyl)propyl]-6,8-dihydro-5H-acridine-2-carboxamide | 684.24 | 182.3 |
| **61** | 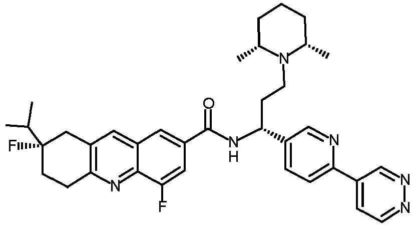 | rac-(7S)-4,7-difluoro-7-isopropyl-N-[rac-(1R)-1-(6-pyridazin-4-yl-3-pyridyl)-3-[rac-(2R,6S)-2,6-dimethyl-1-piperidyl]propyl]-6,8-dihydro-5H-acridine-2-carboxamide | 613.24 | 103.2 |
| **62** | 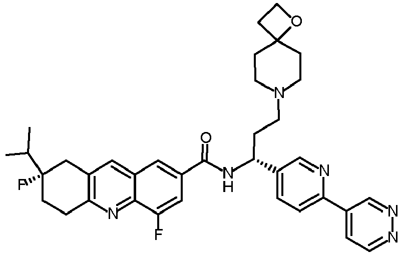 | rac-(7S)-4,7-difluoro-7-isopropyl-N-[rac-(1R)-3-(1-oxa-7-azaspiro[3.5]nonan-7-yl)-1-(6-pyridazin-4-yl-3-pyridyl)propyl]-6,8-dihydro-5H-acridine-2-carboxamide | 627.19 | 60.27 |
| **63** | 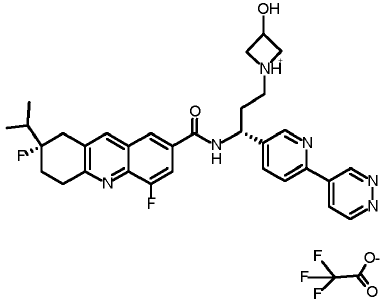 | rac-(7S)-4,7-difluoro-7-isopropyl-N-[rac-(1R)-3-(3-hydroxyazetidin-1-ium-1-yl)-1-(6-pyridazin-4-yl-3-pyridyl)propyl]-6,8-dihydro-5H-acridine-2-carboxamide;2,2,2-trifluoroacetate | 573.18 | 1193 |
| **64** | 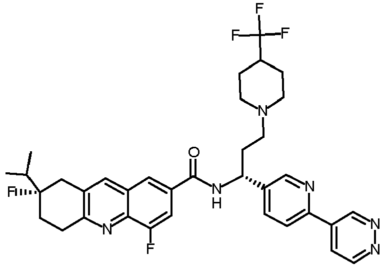 | rac-(7S)-4,7-difluoro-7-isopropyl-N-[rac-(1R)-1-(6-pyridazin-4-yl-3-pyridyl)-3-[4-(trifluoromethyl)-1-piperidyl]propyl]-6,8-dihydro-5H-acridine-2-carboxamide | 653.18 | 1056 |

### Example 65 through Example 68

**Example 65 through 68** were synthesized by a similar procedure as outlined below.

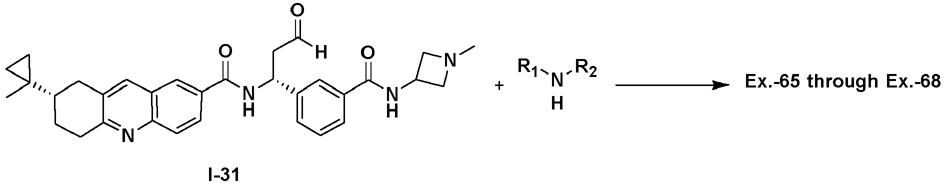

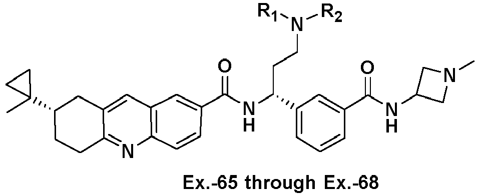

To a solution of (S)-N-((R)-1-(3-((1-methylazetidin-3-yl)carbamoyl)phenyl)-3-oxopropyl)-7-(1-methylcyclopropyl)-5,6,7,8-tetrahydroacridine-2-carboxamide **(I-31,** 25 mg, 0.048 mmol) and amino substrate (0.050 mmol) in anhydrous 5% HOAc/MeOH (1.5 mL) was added polystyrene supported BH₃CN (100 mg, 2.45 mmol/g) at ambient temperature. The resulting reaction mixture was shaken at ambient temperature for 16 hours. The solution was filtered and the solvent was evaporated in vacuum. The crude product was purified by reverse-phase HPLC (MeCN / water with 0.1% TFA as modifier) to give the product as a TFA salt.

| Ex. No. | Structure | Chemical Name | Mass [M+H] ⁺ | hNPRA EC₅₀ (nM) |
|---|---|---|---|---|
| **65** | 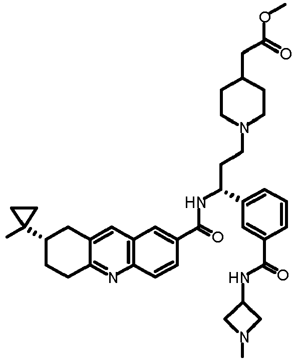 | methyl 2-[1-[rac-(3R)-3-[3-[(1-methylazetidin-3-yl)carbamoyl]phenyl]-3-[[rac-(7S)-7-(1-methylcyclopropyl)-5,6,7,8-tetrahydroacridine-2-carbonyl]amino]propyl]-4-piperidyl]acetate | 666.42 | 359.3 |
| **66** | 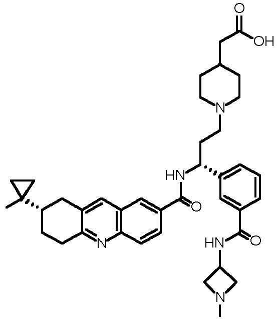 | 2-[1-[rac-(3R)-3-[3-[(1-methylazetidin-3-yl)carbamoyl]phenyl]-3-[[rac-(7S)-7-(1-methylcyclopropyl)-5,6,7,8-tetrahydroacridine-2-carbonyl]amino]propyl]-4-piperidyl]acetic acid | 652.30 | 398.5 |
| **67** | 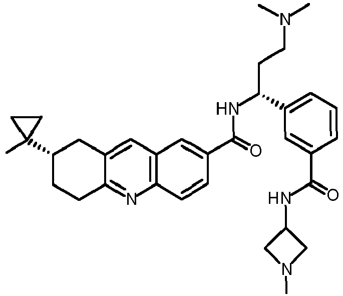 | rac-(7S)-7-(1-methylcyclopropyl)-N-[rac-(1R)-3-(dimethylamino)-1-[3-[(1-methylazetidin-3-yl)carbamoyl]phenyl]propy l]-5,6,7,8-tetrahydroacridine-2-carboxamide | 554.34 | 377.8 |
| **68** | 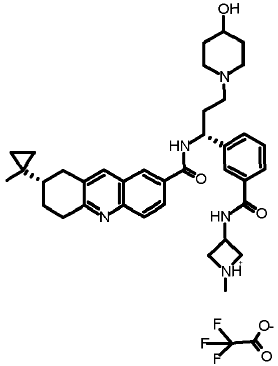 | rac-(7S)-7-(1-methylcyclopropyl)-N-[rac-(1R)-3-(4-hydroxy-1-piperidyl)-1-[3-[(1-methylazetidin-1-ium-3-yl)carbamoyl]phenyl]propy l]-5,6,7,8-tetrahydroacridine-2-carboxamide;2,2,2-trifluoroacetate | 610.26 | 368.6 |

## Claims

1. A compound of the formula I, or a pharmaceutically acceptable salt thereof: 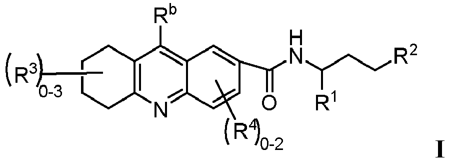
**R^{b}** is hydrogen, halogen, C1-6 alkyl, hydroxy, -(C1-10 alkyl)OH, or C1-6haloalkyl;
**R¹** is selected from phenyl, pyridyl, thiazolyl, imidazolyl, pyrazinyl, and oxadiazolyl, wherein **R¹** is substituted by 0, 1, 2, or 3, **R⁶;**
**R²** is independently selected from:
C₁₋₁₀ alkyl,
arylC₀₋₁₀ alkyl,
C₃₋₁₂ cycloalkylC₀₋₁₀ alkyl,
heteroarylC₀₋₁₀ alkyl,
heterocyclylC₀₋₁₀ alkyl,
C1-10alkylaminoC₀₋₁₀ alkyl,
heteroarylC₀₋₁₀alkylaminoC₀₋₁₀ alkyl,
heterocyclylC₀₋₁₀alkylaminoC₀₋₁₀ alkyl,
C₁₋₁₀ heteroalkyl aminoC₀₋₁₀ alkyl,
C₃₋₁₂ cycloalkyl C₀₋₁₀ alkylaminoC₀₋₁₀ alkyl,
aryl C₀₋₁₀ alkylaminoC₀₋₁₀ alkyl,
amino, and
(C₁₋₁₀ alkyl)₁₋₂ aminoC₀₋₁₀ alkyl, wherein **R²** is each substituted with 0,1, 2, 3, or 4 **R⁵** substituents;
each **R3** is independently selected from halogen, C₁₋₆ alkyl, C₃₋₁₂ cycloalkyl,
-Si(CH₃)₃ and C₁₋₆haloalkyl, wherein each **R³** is independently substituted with 0,1, or 2, **R⁸** substituents and each **R⁸** is independently selected from: C₁₋₆ alkyl, C₁₋₆ alkoxy, halogen, and C₁₋₆haloalkyl;
each **R⁴** is independently selected from halogen, C₁₋₆ alkyl, and C₁₋₆haloalkyl;
each **R⁵** is independently selected from:
halogen,
C₁₋₁₀ alkyl,
C₁₋₁₀ heteroalkyl,
aryl C₀₋₁₀ alkyl,
C₃₋₁₂ cycloalkyl C₀₋₁₀ alkyl,
heteroaryl C₀₋₁₀ alkyl,
heterocyclylC₀₋₁₀ alkyl,
amino C₀₋₁₀ alkyl,
((C₁₋₁₀alkyl)₁₋₂amino)C₀₋₁₀ alkyl,
-CO₂(C₁₋₁₀ alkyl),
-(C₀₋₁₀ alkyl)CO₂H,
-(C₀₋₁₀ alkyl)CO₂(C₁₋₁₀ alkyl),
Oxo (=O),
hydroxy,
-(C₁₋₁₀ alkyl)OH,
C₁₋₁₀ alkoxyC₀₋₁₀ alkyl,
cyano, and
C₁₋₆haloalkyl, wherein each **R⁵** is independently substituted with 0,1, 2, 3, or 4 **R⁹** and each **R⁹** is independently selected from: C₁₋₆ alkyl, hydroxy, C₁₋₆ alkoxy, halogen, and C₁₋₆haloalkyl;
**R⁶** is independently selected from:
halogen,
C₁₋₁₀ alkyl,
aryl C₀₋₁₀ alkyl,
C₃₋₁₂ cycloalkylC₀₋₁₀ alkyl,
heteroaryl C₀₋₁₀ alkyl,
heterocyclyl C₀₋₁₀ alkyl,
((C₀₋₁₀)alkyl)₁₋₂aminocarbonyl,
C₁₋₁₀ alkoxy(C₀₋₁₀)alkyl,
(C₀₋₁₀)alkylaminocarbonyl,
(C₁₋₁₀)heteroalkylaminocarbonyl,
aryl(C₀₋₁₀)alkylaminocarbonyl,
(C₃₋₁₂)cycloalkyl(C₀₋₁₀)alkylaminocarbonyl,
heteroaryl(C₀₋₁₀)alkylaminocarbonyl,
heterocyclyl(C₀₋₁₀)alkylaminocarbonyl,
-NHSO₂(C₀₋₆ alkyl),
-SO₂N(C₁₋₆ alkyl)₀₋₂,
-SO₂CF₃,
-SO₂CF₂H,
amino,
(C₀₋₁₀ alkyl)₁₋₂ amino,
hydroxy,
-(C₁₋₁₀ alkyl)OH,
cyano,
C₁₋₆haloalkyl,
-CO₂(C₁₋₁₀ alkyl),
Oxo (=O);
C₁₋₁₀ alkylS(O)₁₋₂,
C₁₋₁₀ heteroalkyl S(O)₁₋₂,
(C₃₋₁₂) cycloalkylS(O)₁₋₂,
heterocyclyl S(O)₁₋₂,
heteroarylS(O)₁₋₂, and
arylS(O)₁₋₂;
wherein **R⁶** is each substituted with 0,1, 2, 3, or 4 **R⁷;**
each **R⁷** is independently selected from:
halogen,
C₁₋₁₀ alkyl,
C₁₋₆ haloalkyl,
C₁₋₁₀ heteroalkyl,
aryl C₀₋₁₀ alkyl,
C₃₋₁₂ cycloalkyl C₀₋₁₀ alkyl,
heteroaryl C₀₋₁₀ alkyl,
heterocyclyl C₀₋₁₀ alkyl,
amino C₀₋₁₀ alkyl,
((C₁₋₁₀)alkyl)₁₋₂amino,
-CO₂(C₁₋₁₀ alkyl),
-(C₀₋₁₀ alkyl)CO₂H,
Oxo (=O),
hydroxy,
-(C₁₋₁₀ alkyl)OH,
C₁₋₁₀ alkoxy,
cyano, and
aminocarbonyl; wherein
the term "heteroalkyl" refers to an alkyl group where 1, 2, or 3 of the carbon atoms is substituted by a heteroatom independently chosen from N, O, or S; the term "heterocyclyl" means a monocyclic or bicyclic system of up to 10 atoms in each ring containing 1-4 heteroatoms selected from O,N,S,SO or SO₂;
the term "aryl" refers to a monocyclic, bicyclic or tricyclic carbocyclic aromatic ring or ring system containing 5-14 carbon atoms, wherein at least one of the rings is aromatic; and
the term "heteroaryl" refers to a stable monocyclic, bicyclic or tricyclic ring system containing 5-14 carbon atoms and containing at least one ring heteroatom selected from N, S (including SO and SO₂) and O, wherein at least one of the heteroatom containing rings is aromatic.

2. The compound of Claim 1 or a pharmaceutically acceptable salt thereof, wherein **R¹** is phenyl or pyridyl, wherein **R¹** is substituted by 0, 1, 2 or 3 **R⁶.**

3. The compound of any of Claims 1 or 2 or a pharmaceutically acceptable salt thereof, wherein each **R⁶** is independently selected from: halogen, heteroaryl C₀₋₁₀ alkyl, heterocyclyl C₀₋₁₀ alkyl, heterocyclyl(C₀₋₁₀)alkylaminocarbonyl, -NHSO₂(C₀₋₆ alkyl), -SO₂N(C1-6 alkyl)₀₋₂, C₁₋₁₀ alkylS(O)₁₋₂, C₁₋₁₀ heteroalkyl S(O)₁₋₂, (C₃₋₁₂) cycloalkylS(O)₁₋₂, heterocyclyl S(O)₁₋₂, heteroarylS(O)₁₋₂, and arylS(O)₁₋₂, wherein **R⁶** is each substituted with 0,1, 2, 3, or 4 **R⁷.**

4. The compound of any of Claims 1 or 2 or a pharmaceutically acceptable salt thereof, wherein each **R⁶** is independently selected from fluoro, chloro, bromo, iodo, oxazolidinyl, pyridazinyl, piperidinyl, pyridinyl, oxa-azabicyclo-[2.2.2]octanyl, 2-oxa-5-azabicyclo-[2.2.2]octanyl, imidazolinyl, ethylsulfonyl, pyrazolyl, azetidinylcarbamoyl, and - NS(O)₂H, wherein **R⁶** is each substituted with 0,1, or 2, **R⁷.**

5. The compound of any of Claims 1 to 4 or a pharmaceutically acceptable salt thereof, wherein each **R²** is independently selected from C₁₋₁₀ alkyl, C₃₋₁₂ cycloalkylC₀₋₁₀ alkyl, heteroarylC₀₋₁₀ alkyl, heterocyclylC₀₋₁₀ alkyl, C₃₋₁₂ cycloalkyl C₀₋₁₀ alkylaminoC⁰⁻¹⁰ alkyl, amino, and (C₁₋₁₀ alkyl)₁₋₂ aminoC₀₋₁₀ alkyl, wherein **R²** is each substituted with 0,1, 2, 3, or 4 **R⁵.**

6. The compound of any of Claims 1 to 4 or a pharmaceutically acceptable salt thereof, wherein each **R²** is independently selected from cyclopropylmethyl, dimethylamino, pyrrolidinyl, oxazepanyl, azetidinyl, azabicyclo[3.1.1]heptyl, decahydroisoquinolinyl, azaspiro[2.5]octanyl, 6-azaspiro[2.5]octanyl, azaspiro[4.5]decanyl, oxa-azaspiro[4.5]decyl, and oxa-azaspiro[3.5]nonyl, piperidinyl, wherein **R⁵** is each substituted with 0,1, 2, 3, or 4 **R⁵.**

7. The compound of any of Claims 1 to 6, or a pharmaceutically acceptable salt thereof, wherein **R⁵** is independently selected from C₁₋₁₀ alkyl, C₃₋₁₂ cycloalkyl C₀₋₁₀ alkyl, heteroaryl C₀₋₁₀ alkyl, heterocyclylC₀₋₁₀ alkyl, ((C₁₋₁₀alkyl)1-2amino)C₀₋₁₀ alkyl, -CO₂(C₁₋₁₀ alkyl), -(C₀₋₁₀ alkyl)CO₂H, -(C₀₋₁₀ alkyl)CO₂(C₁₋₁₀ alkyl), Oxo, hydroxy, -(C₁₋₁₀ alkyl)OH, C₁₋₁₀ alkoxyC₀₋₁₀ alkyl, and C₁₋₆haloalkyl, wherein each **R⁵** is independently substituted with 0,1, 2, 3, or 4 **R⁹.**

8. The compound of any of Claims 1 to 6 or a pharmaceutically acceptable salt thereof, wherein each **R⁵** is independently selected from methyl, isopropyl, ethyl, butyl, tert-butyl, (cyclopropyl)methyl, morpholinomethyl, methoxymethyl, methoxypropyl, methoxy, (dimethylamino)ethyl, (dimethylamino)methyl, hydroxymethyl, carboxy, hydroxy(methylethyl), trifluoromethyl, wherein each **R⁵** is independently substituted with 0,1, 2, 3, or 4 **R⁹.**

9. The compound of any of Claims 1 to 8 or a pharmaceutically acceptable salt thereof, wherein each **R3** is independently selected from halogen, isopropyl, methyl, ethyl *tert-*butyl, difluoromethyl, cyclopropyl, and -Si(CH₃)₃, wherein each **R³** is independently substituted with 0, 1, or 2 **R⁸.**

10. The compound of any of Claims 1 to 9 or a pharmaceutically acceptable salt thereof, wherein **R⁴** is independently selected from fluoro, chloro and methyl.

11. The compound of any of Claims 1 to 10 or a pharmaceutically acceptable salt thereof, wherein each **R⁷** is independently selected from halogen, C₁₋₁₀ alkyl, C₁₋₆ haloalkyl, amino C₀₋₁₀ alkyl, ((C₁₋₁₀)alkyl)₁₋₂amino, Oxo, hydroxy, -(C₁₋₁₀ alkyl)OH, and C₁₋₁₀ alkoxy.

12. The compound of any of Claims 1 to 11 or a pharmaceutically acceptable salt thereof, wherein each **R⁸** is independently selected from: methyl, ethyl, propyl, butyl, methoxy, difluoromethyl, fluoro, and chloro.

13. The compound of any of Claims 1 to 12 or a pharmaceutically acceptable salt thereof, wherein each **R⁹** is independently selected from: methyl, ethyl, hydroxy, methoxy, ethoxy, halogen, and trifluoromethyl.

14. The compound of claim 1, or a pharmaceutically acceptable salt thereof, selected from:
(S)-N-((R)-3-(4-hydroxypiperidin-1-yl)-1-(4-(2-oxooxazolidin-3-yl)phenyl)propyl)-7-(1-methylcyclopropyl)-5,6,7,8-tetrahydroacridine-2-carboxamide;
(S)-N-((R)-3-((S)-2-(hydroxymethyl)pyrrolidin-1-yl)-1-(4-(2-oxooxazolidin-3-yl)phenyl)propyl)-7-(1-methylcyclopropyl)-5,6,7,8-tetrahydroacridine-2-carboxamide;
(7S)-7-tert-butyl-N-{(1R)-3-(4-hydroxypiperidin-1-yl)-1-[4-(2-oxo-1,3-oxazolidin-3-yl)phenyl]propyl}-5,6,7,8-tetrahydroacridine-2-carboxamide;
(7S)-N-{(1R)-3-(4-hydroxypiperidin-1-yl)-1-[4-(2-oxo-1,3-oxazolidin-3-yl)phenyl]propyl}-7-(trimethylsilyl)-5,6,7,8-tetrahydroacridine-2-carboxamide;
(7S)-4,7-difluoro-N-{(1R)-3-(4-hydroxypiperidin-1-yl)-1-[4-(2-oxo-1,3-oxazolidin-3-yl)phenyl]propyl}-7-(1-methylethyl)-5,6,7,8-tetrahydroacridine-2-carboxamide;
(7S)-3,4,7-trifluoro-N-{(1R)-3-(4-hydroxypiperidin-1-yl)-1-[4-(2-oxo-1,3-oxazolidin-3-yl)phenyl]propyl}-7-(1-methylethyl)-5,6,7,8-tetrahydroacridine-2-carboxamide;
7-tert-butyl-3-fluoro-N-{(1R)-3-(4-hydroxypiperidin-1-yl)-1-[4-(2-oxo-1,3-oxazolidin-3-yl)phenyl]propyl}-5,6,7,8-tetrahydroacridine-2-carboxamide;
(S)-7-(tert-butyl)-4-chloro-N-((R)-3-(4-hydroxypiperidin-1-yl)-1-(4-(2-oxooxazolidin-3-yl)phenyl)propyl)-5, 6,7, 8-tetrahydroacridine-2-carboxamide;
7-(tert-butyl)-N-((R)-3-(4-hydroxypiperidin-1-yl)-1-(4-(2-oxooxazolidin-3-yl)phenyl)propyl)-4-methyl-5,6,7,8-tetrahydroacridine-2-carboxamide;
(7S)-4,7-difluoro-N-[(1R)-3-(4-hydroxypiperidin-1-yl)-1-(6-pyridazin-4-ylpyridin-3-yl)propyl]-7-(1-methylethyl)-5,6,7,8-tetrahydroacridine-2-carboxamide;
(7S)-4,7-difluoro-N-[(1R)-3-(4-hydroxy-2,2-dimethylpiperidin-1-yl)-1-(6-pyridazin-4-ylpyridin-3-yl)propyl]-7-(1-methylethyl)-5,6,7,8-tetrahydroacridine-2-carboxamide;
(7S)-4,7-difluoro-N-[(1R)-3-(4-hydroxy-4-methylpiperidin-1-yl)-1-(6-pyridazin-4-ylpyridin-3- yl)propyl]-7-(1-methylethyl)-5,6,7,8-tetrahydroacridine-2-carboxamide;
1-[(3R)-3-({[(7S)-4,7-difluoro-7-(1-methylethyl)-5,6,7,8-tetrahydroacridin-2-yl]carbonyl}amino)-3-(6-pyridazin-4-ylpyridin-3-yl)propyl]-4-hydroxypiperidine-4-carboxylic acid;
(7S)-4,7-difluoro-N-[(1R)-3-[6-hydroxy-6-(trifluoromethyl)-3-azabicyclo[3.1.1]hept-3-yl]-1-(6-pyridazin-4-ylpyridin-3-yl)propyl]-7-(1-methylethyl)-5,6,7,8-tetrahydroacridine-2-carboxamide;
(7S)-4,7-difluoro-N-[(1R)-3-(4a-hydroxyoctahydroisoquinolin-2(1H)-yl)-1-(6-pyridazin-4-ylpyridin-3-yl)propyl]-7-(1-methylethyl)-5,6,7,8-tetrahydroacridine-2-carboxamide;
(7S)-4,7-difluoro-7-(1-methylethyl)-N-[(1R)-3-(1-oxa-8-azaspiro[4.5]dec-8-yl)-1-(6-pyridazin-4-ylpyridin-3-yl)propyl]-5,6,7,8-tetrahydroacridine-2-carboxamide;
(7S)-4,7-difluoro-7-(1-methylethyl)-N-[(1R)-3-(1-oxa-7-azaspiro[3.5]non-7-yl)-1-(6-pyridazin-4-ylpyridin-3-yl)propyl]-5,6,7,8-tetrahydroacridine-2-carboxamide;
(7S)-4,7-difluoro-N-[(1R)-3-(4-methoxypiperidin-1-yl)-1-(6-pyridazin-4-ylpyridin-3-yl)propyl]-7-(1-methylethyl)-5,6,7,8-tetrahydroacridine-2-carboxamide;
(7S)-4,7-difluoro-N-[(1R)-3-[4-hydroxy-4-(hydroxymethyl)piperidin-1-yl]-1-(6-pyridazin-4-ylpyridin-3-yl)propyl]-7-(1-methylethyl)-5,6,7,8-tetrahydroacridine-2-carboxamide;
(7S)-4,7-difluoro-N-[(1R)-1-(5-fluoro-6-pyridazin-4-ylpyridin-3-yl)-3-(4-hydroxypiperidin-1-yl)propyl]-7-(1-methylethyl)-5,6,7,8-tetrahydroacridine-2-carboxamide;
(S)-N-((R)-3-((S)-2-(hydroxymethyl)pyrrolidin-1-yl)-1-(3-((1-methylazetidin-3-yl)carbamoyl)phenyl)propyl)-7-(1-methylcyclopropyl)-5,6,7,8-tetrahydroacridine-2-carboxamide;
(S)-N-((R)-3-((S)-2-(hydroxymethyl)pyrrolidin-1-yl)-1-(3-((1-methylazetidin-3-yl)carbamoyl)phenyl)propyl)-7-(1-(trifluoromethyl)cyclopropyl)-5,6,7,8-tetrahydroacridine-2-carboxamide;
((S)-7-(1-(difluoromethyl)cyclopropyl)-N-((R)-3-((S)-2-(hydroxymethyl)pyrrolidin-1-yl)-1-(3-((1-methylazetidin-3-yl)carbamoyl)phenyl)propyl)-5,6,7,8-tetrahydroacridine-2-carboxamide;
methyl 1-((R)-3-((S)-4,7-difluoro-7-isopropyl-5,6,7,8-tetrahydroacridine-2-carboxamido)-3-(4-(5-fluoro-6-hydroxypyridin-3-yl)phenyl)propyl)piperidine-4-carboxylate;
1-((R)-3-((S)-4,7-difluoro-7-isopropyl-5,6,7,8-tetrahydroacridine-2-carboxamido)-3-(4-(5-fluoro-6-hydroxypyridin-3-yl)phenyl)propyl)piperidine-4-carboxylic acid;
(S)-N-((R)-1-(6-((N,N-dimethylsulfamoyl)amino)pyridin-3-yl)-3-(4-hydroxypiperidin-1-yl)propyl)-4,7-difluoro-7-isopropyl-5,6,7,8-tetrahydroacridine-2-carboxamide;
(S)-N-((R)-1-(6-((N,N-dimethylsulfamoyl)amino)-5-fluoropyridin-3-yl)-3-(4-hydroxypiperidin-1-yl)propyl)-4,7-difluoro-7-isopropyl-5,6,7,8-tetrahydroacridine-2-carboxamide;
(S)-N-((R)-1-(6-(2,4-dioxoimidazolidin-1-yl)pyridin-3-yl)-3-(4-hydroxypiperidin-1-yl)propyl)-4,7-difluoro-7-isopropyl-5, 6,7, 8-tetrahydroacridine-2-carb oxamide;
(7S)-4,7-difluoro-N-((1R)-3-(4-hydroxypiperidin-1-yl)-1-(6-(5-methyl-2,4-dioxoimidazolidin-1-yl)pyridin-3-yl)propyl)-7-isopropyl-5,6,7,8-tetrahydroacridine-2-carboxamide;
(S)-N-((R)-1-(6-(ethylsulfonyl)pyridin-3-yl)-3-(4-hydroxypiperidin-1-yl)propyl)-4,7-difluoro-7-isopropyl-5, 6,7, 8-tetrahydroacridine-2-carboxamide;
(S)-9-(hydroxymethyl)-N-((R)-3-(4-hydroxypiperidin-1-yl)-1-(4-(2-oxooxazolidin-3-yl)phenyl)propyl)-7-(1-methylcyclopropyl)-5,6,7,8-tetrahydroacridine-2-carboxamide;
(S)-N-((R)-1-(6-(1H-pyrazol-4-yl)pyridin-3-yl)-3-(4-hydroxypiperidin-1-yl)propyl)-4,7-difluoro-7-isopropyl-5, 6,7, 8-tetrahydroacridine-2-carboxamide;
(S)-4,7-difluoro-N-((R)-1-(6-((3R,4R)-3-fluoro-4-hydroxypiperidin-1-yl)pyridin-3-yl)-3-(4-hydroxypiperidin-1-yl)propyl)-7-isopropyl-5, 6,7, 8-tetrahydroacridine-2-carboxamide;
(7S)-N-((1R)-1-(6-(2-oxa-5-azabicyclo[2.2.2]octan-5-yl)pyridin-3-yl)-3-(4-hydroxypiperidin-1-yl)propyl)-4,7-difluoro-7-isopropyl-5,6,7,8-tetrahydroacridine-2-carboxamide;
(7S)-4,7-difluoro-7-isopropyl-N-[(1R)-1-(6-pyridazin-4-yl-3-pyridyl)-3-pyrrolidin-1-ium-1-yl-propyl]-6,8-dihydro-5H-acridine-2-carboxamide;2,2,2-trifluoroacetate;
(7S)-4,7-difluoro-7-isopropyl-N-[(1R)-1-(6-pyridazin-4-yl-3-pyridyl)-3-[(3S)-3-hydroxypiperidin-1-ium-1-yl]propyl]-6,8-dihydro-5H-acridine-2-carboxamide;2,2,2-trifluoroacetate;
dimethyl-[(3R)-3-(6-pyridazin-4-yl-3-pyridyl)-3-[[(7S)-4,7-difluoro-7-isopropyl-6,8-dihydro-5H-acridine-2-carbonyl]amino]propyl]ammonium;2,2,2-trifluoroacetate;
(7S)-4,7-difluoro-7-isopropyl-N-[(1R)-3-(2-oxa-8-azoniaspiro[4.5]decan-8-yl)-1-(6-pyridazin-4-yl-3-pyridyl)propyl]-6,8-dihydro-5H-acridine-2-carboxamide;2,2,2-trifluoroacetate;
(7S)-4,7-difluoro-7-isopropyl-N-[(1R)-3-[2-(hydroxymethyl)-6-azoniaspiro[2.5]octan-6-yl]-1-(6-pyridazin-4-yl-3-pyridyl)propyl]-6,8-dihydro-5H-acridine-2-carboxamide;2,2,2-trifluoroacetate;
(7S)-4,7-difluoro-7-isopropyl-N-[(1R)-1-(6-pyridazin-4-yl-3-pyridyl)-3-[(3R)-3-hydroxypiperidin-1-ium-1-yl]propyl]-6,8-dihydro-5H-acridine-2-carboxamide;2,2,2-trifluoroacetate;
(7S)-4,7-difluoro-7-isopropyl-N-[(1R)-3-[4-(1-hydroxyethyl)piperidin-1-ium-1-yl]-1-(6-pyridazin-4-yl-3-pyridyl)propyl]-6,8-dihydro-5H-acridine-2-carboxamide;2,2,2-trifluoroacetate;
(7S)-4,7-difluoro-7-isopropyl-N-[(1R)-3-(1,2,3,4,4a,5,6,7,8,8a-decahydroisoquinolin-2-ium-2-yl)-1-(6-pyridazin-4-yl-3-pyridyl)propyl]-6,8-dihydro-5H-acridine-2-carboxamide;2,2,2-trifluoroacetate;
(7S)-4,7-difluoro-7-isopropyl-N-[(1R)-3-[2-[2-(dimethylamino)ethyl]piperidin-1-ium-1-yl]-1-(6-pyridazin-4-yl-3-pyridyl)propyl]-6, 8-dihydro-SH-acridine-2-carboxamide;2,2,2-trifluoroacetate;
(7S)-4,7-difluoro-7-isopropyl-N-[(1R)-3-(2-methylpiperidin-1-ium-1-yl)-1-(6-pyridazin-4-yl-3-pyridyl)propyl]-6,8-dihydro-5H-acridine-2-carboxamide;2,2,2-trifluoroacetate;
(7S)-4,7-difluoro-7-isopropyl-N-[(1R)-3-[4-(hydroxymethyl)piperidin-1-ium-1-yl]-1-(6-pyridazin-4-yl-3-pyridyl)propyl]-6,8-dihydro-5H-acridine-2-carboxamide;2,2,2-trifluoroacetate;
(7S)-4,7-difluoro-7-isopropyl-N-[(1R)-3-[4-(methoxymethyl)piperidin-1-ium-1-yl]-1-(6-pyridazin-4-yl-3-pyridyl)propyl]-6,8-dihydro-5H-acridine-2-carboxamide;2,2,2-trifluoroacetate;
(7S)-4,7-difluoro-7-isopropyl-N-[(1R)-3-[4-(1-hydroxy-1-methyl-ethyl)piperidin-1-ium-1-yl]-1-(6-pyridazin-4-yl-3-pyridyl)propyl]-6,8-dihydro-5H-acridine-2-carboxamide;2,2,2-trifluoroacetate;
(7S)-4,7-difluoro-7-isopropyl-N-[(1R)-1-(6-pyridazin-4-yl-3-pyridyl)-3-[(3S)-3-hydroxy-8-azaspiro[4.5]decan-8-yl]propyl]-6,8-dihydro-5H-acridine-2-carboxamide;
(7S)-4,7-difluoro-7-isopropyl-N-[(1R)-1-(6-pyridazin-4-yl-3-pyridyl)-3-[(3R)-3-hydroxy-8-azaspiro[4.5]decan-8-yl]propyl]-6,8-dihydro-5H-acridine-2-carboxamide;
(7S)-4,7-difluoro-7-isopropyl-N-[(1R)-3-(2-oxa-7-azaspiro[3.5]nonan-7-yl)-1-(6-pyridazin-4-yl-3-pyridyl)propyl]-6, 8-dihydro-SH-acridine-2-carboxamide;
(7S)-4,7-difluoro-7-isopropyl-N-[(1R)-3-[4-(3-methoxypropyl)-1-piperidyl]-1-(6-pyridazin-4-yl-3-pyridyl)propyl]-6,8-dihydro-5H-acridine-2-carboxamide;
(7S)-4,7-difluoro-7-isopropyl-N-[(1R)-3-(4a-hydroxy-1,3,4,5,6,7,8,8a-octahydroisoquinolin-2-yl)-1-(6-pyridazin-4-yl-3-pyridyl)propyl]-6,8-dihydro-5H-acridine-2-carboxamide;
(7S)-4,7-difluoro-7-isopropyl-N-[(1R)-3-[2-(2-hydroxyethyl)-1-piperidyl]-1-(6-pyridazin-4-yl-3-pyridyl)propyl]-6,8-dihydro-5H-acridine-2-carboxamide;
(7S)-4,7-difluoro-7-isopropyl-N-[(1R)-3-[4-[hydroxy-[1-(hydroxymethyl)cyclopropyl]methyl]-1-piperidyl]-1-(6-pyridazin-4-yl-3-pyridyl)propyl]-6,8-dihydro-5H-acridine-2-carboxamide;
(7S)-4,7-difluoro-7-isopropyl-N-[(1R)-3-(1-oxa-8-azaspiro[4.5]decan-8-yl)-1-(6-pyridazin-4-yl-3-pyridyl)propyl]-6,8-dihydro-5H-acridine-2-carboxamide;
(7S)-4,7-difluoro-7-isopropyl-N-[(1R)-3-(4-isopropyl-1-piperidyl)-1-(6-pyridazin-4-yl-3-pyridyl)propyl]-6,8-dihydro-5H-acridine-2-carboxamide;
(7S)-4,7-difluoro-7-isopropyl-N-[(1R)-3-(1,4-oxazepan-4-yl)-1-(6-pyridazin-4-yl-3-pyridyl)propyl]-6,8-dihydro-5H-acridine-2-carboxamide;
(7S)-4,7-difluoro-7-isopropyl-N-[(1R)-3-[2-(hydroxymethyl)-1-piperidyl]-1-(6-pyridazin-4-yl-3-pyridyl)propyl]-6,8-dihydro-5H-acridine-2-carboxamide;
(7S)-4,7-difluoro-7-isopropyl-N-[(1R)-3-[2-[(dimethylamino)methyl]-1-piperidyl]-1-(6-pyridazin-4-yl-3-pyridyl)propyl]-6,8-dihydro-5H-acridine-2-carboxamide;
(7S)-4,7-difluoro-7-isopropyl-N-[(1R)-3-[4-(morpholinomethyl)-1-piperidyl]-1-(6-pyridazin-4-yl-3-pyridyl)propyl]-6,8-dihydro-5H-acridine-2-carboxamide;
(7S)-4,7-difluoro-7-isopropyl-N-[(1R)-1-(6-pyridazin-4-yl-3-pyridyl)-3-[(2R,6S)-2,6-dimethyl-1-piperidyl]propyl]-6,8-dihydro-5H-acridine-2-carboxamide;
(7S)-4,7-difluoro-7-isopropyl-N-[(1R)-3-(1-oxa-7-azaspiro[3.5]nonan-7-yl)-1-(6-pyridazin-4-yl-3-pyridyl)propyl]-6,8-dihydro-5H-acridine-2-carboxamide;
(7S)-4,7-difluoro-7-isopropyl-N-[(1R)-3-(3-hydroxyazetidin-1-ium-1-yl)-1-(6-pyridazin-4-yl-3-pyridyl)propyl]-6,8-dihydro-5H-acridine-2-carboxamide;2,2,2-trifluoroacetate;
(7S)-4,7-difluoro-7-isopropyl-N-[(1R)-1-(6-pyridazin-4-yl-3-pyridyl)-3-[4-(trifluoromethyl)-1-piperidyl]propyl]-6,8-dihydro-5H-acridine-2-carboxamide;
methyl 2-[1-[(3R)-3-[3-[(1-methylazetidin-3-yl)carbamoyl]phenyl]-3-[[(7S)-7-(1-methylcyclopropyl)-5,6,7,8-tetrahydroacridine-2-carbonyl]amino]propyl]-4-piperidyl]acetate;
2-[1-[(3R)-3-[3-[(1-methylazetidin-3-yl)carbamoyl]phenyl]-3-[[(7S)-7-(1-methylcyclopropyl)-5,6,7,8-tetrahydroacridine-2-carbonyl]amino]propyl]-4-piperidyl]acetic acid;
(7S)-7-(1-methylcyclopropyl)-N-[(1R)-3-(dimethylamino)-1-[3-[(1-methylazetidin-3-yl)carbamoyl]phenyl]propyl]-5,6,7,8-tetrahydroacridine-2-carboxamide; and
(7S)-7-(1-methylcyclopropyl)-N-[(1R)-3-(4-hydroxy-1-piperidyl)-1-[3-[(1-methylazetidin-1-ium-3-yl)carbamoyl]phenyl]propyl]-5,6,7,8-tetrahydroacridine-2-carboxamide;2,2,2-trifluoroacetate.

15. A pharmaceutical composition comprising an effective amount of a compound of any of Claims 1 to 14, or a pharmaceutically acceptable salt thereof, and a pharmaceutically acceptable carrier.

16. The pharmaceutical composition of Claim 15, further comprising one or more additional therapeutic agents.

17. A compound of any one of claims 1-14, or a pharmaceutically acceptable salt thereof, for use in therapy.

18. A compound of any one of claims 1-14 for use in the treatment of cardiometabolic diseases, kidney disease, or diabetes.

## Patentansprüche

1. Eine Verbindung der Formel I oder ein pharmazeutisch annehmbares Salz davon: 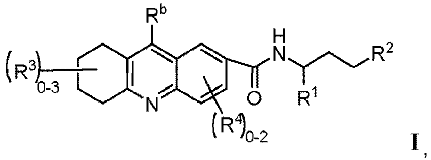 wobei
**R^{b}** Wasserstoff, Halogen, C1-6-Alkyl, Hydroxy, -(C1-10-Alkyl)OH oder C1-6-Halogenalkyl ist,
**R¹** ausgewählt ist aus Phenyl, Pyridyl, Thiazolyl, Imidazolyl, Pyrazinyl und Oxadiazolyl, wobei **R¹** durch 0, 1, 2 oder 3 **R⁶** substituiert ist,
**R²** unabhängig ausgewählt ist aus:
C₁₋₁₀-Alkyl,
Aryl-C₀₋₁₀-alkyl,
C₃₋₁₂-Cycloalkyl-C₀₋₁₀-alkyl,
Heteroaryl-C₀₋₁₀-alkyl,
Heterocyclyl-C₀₋₁₀-alkyl,
C1-10-Alkylamino-C₀₋₁₀-alkyl,
Heteroaryl-C₀₋₁₀-alkylamino-C₀₋₁₀-alkyl,
Heterocyclyl-C₀₋₁₀-alkylamino-C₀₋₁₀-alkyl,
C₁₋₁₀-Heteroalkylamino-C₀₋₁₀-alkyl,
C₃₋₁₂-Cycloalkyl-C₀₋₁₀-alkylamino-C₀₋₁₀-alkyl,
Aryl-C₀₋₁₀-alkylamino-C₀₋₁₀-alkyl,
Amino und
(C₁₋₁₀-Alkyl)₁₋₂-amino-C₀₋₁₀-alkyl, wobei **R²** jeweils mit 0, 1, 2, 3 oder 4 **R⁵**-Substituenten substituiert ist,
jedes **R³** unabhängig ausgewählt ist aus Halogen, C₁₋₆-Alkyl, C₃₋₁₂-Cycloalkyl, -Si(CH₃)₃ und C₁₋₆-Halogenalkyl, wobei jedes **R³** unabhängig mit 0, 1 oder 2 **R⁸**-Substituenten substituiert ist und jedes **R⁸** unabhängig ausgewählt ist aus: C₁₋₆-Alkyl, C₁₋₆-Alkoxy, Halogen und C₁₋₆-Halogenalkyl,
jedes **R⁴** unabhängig ausgewählt ist aus Halogen, C₁₋₆-Alkyl und C₁₋₆-Halogenalkyl,
jedes **R⁵** unabhängig ausgewählt ist aus:
Halogen,
C₁₋₁₀-Alkyl,
C₁₋₁₀-Heteroalkyl,
Aryl-C₀₋₁₀-alkyl,
C₃₋₁₂-Cycloalkyl-C₀₋₁₀-alkyl,
Heteroaryl-C₀₋₁₀-alkyl, Heterocyclyl-C₀₋₁₀-alkyl, Amino-C₀₋₁₀-alkyl,
((C₁₋₁₀-Alkyl)₁₋₂-amino)C₀₋₁₀-alkyl,
-CO₂(C₁₋₁₀-Alkyl),
-(C₀₋₁₀Alkyl)CO₂H,
-(C₀₋₁₀-Alkyl)CO₂(C₁₋₁₀-alkyl),
Oxo (=O),
Hydroxy,
-(C₁₋₁₀-Alkyl)OH,
C₁₋₁₀-Alkoxy-C₀₋₁₀-alkyl,
Cyano und
C₁₋₆-Halogenalkyl, wobei jedes **R⁵** unabhängig mit 0, 1, 2, 3 oder 4 **R⁹** substituiert ist und jedes **R⁹** unabhängig ausgewählt ist aus: C₁₋₆-Alkyl, Hydroxy, C₁₋₆-Alkoxy, Halogen und C₁₋₆-Halogenalkyl,
**R⁶** unabhängig ausgewählt ist aus:
Halogen,
C₁₋₁₀-Alkyl,
Aryl-C₀₋₁₀-alkyl,
C₃₋₁₂-Cycloalkyl-C₀₋₁₀-alkyl,
Heteroaryl-C₀₋₁₀-alkyl,
Heterocyclyl-C₀₋₁₀-alkyl,
((C₀₋₁₀)Alkyl)₁₋₂-aminocarbonyl,
C₁₋₁₀-Alkoxy(C₀₋₁₀)alkyl,
(C₀₋₁₀)Alkylaminocarbonyl,
(C₁₋₁₀)Heteroalkylaminocarbonyl,
Aryl(C₀₋₁₀)alkylaminocarbonyl,
(C₃₋₁₂)Cycloalkyl(C₀₋₁₀)alkylaminocarbonyl,
Heteroaryl(C₀₋₁₀)alkylaminocarbonyl,
Heterocyclyl(C₀₋₁₀)alkylaminocarbonyl,
-NHSO₂(C₀₋₆-Alkyl),
-SO₂N(C₁₋₆-Alkyl)₀₋₂,
-SO₂CF₃,
-SO₂CF₂H,
Amino,
(C₀₋₁₀-Alkyl)₁₋₂-amino,
Hydroxy,
-(C₁₋₁₀-Alkyl)OH,
Cyano,
C₁₋₆-Halogenalkyl,
-CO₂(C₁₋₁₀-Alkyl)
Oxo (=O),
C₁₋₁₀-Alkyl-S(O)₁₋₂,
C₁₋₁₀-Heteroalkyl-S(O)₁₋₂,
(C₃₋₁₂)Cycloalkyl-S(O)₁₋₂,
Heterocyclyl-S(O)₁₋₂,
Heteroaryl-S(O)₁₋₂ und
Aryl-S(O)₁₋₂,
wobei **R⁶** jeweils mit 0, 1, 2, 3 oder 4 **R⁷** substituiert ist,
jedes **R⁷** unabhängig ausgewählt ist aus:
Halogen,
C₁₋₁₀-Alkyl,
C₁₋₆-Halogenalkyl,
C₁₋₁₀-Heteroalkyl,
Aryl-C₀₋₁₀-alkyl,
C₃₋₁₂-Cycloalkyl-C₀₋₁₀-alkyl,
Heteroaryl-C₀₋₁₀-alkyl,
Heterocyclyl-C₀₋₁₀-alkyl,
Amino-C₀₋₁₀-alkyl,
((C₁₋₁₀)Alkyl)₁₋₂-amino,
-CO₂(C₁₋₁₀-Alkyl),
-(C₀₋₁₀-Alkyl)CO₂H,
Oxo (=O),
Hydroxy,
-(C₁₋₁₀-Alkyl)OH,
C₁₋₁₀-Alkoxy,
Cyano und
Aminocarbonyl, wobei
der Ausdruck "Heteroalkyl" eine Alkylgruppe bedeutet, bei der 1, 2 oder 3 der Kohlenstoffatome durch ein Heteroatom, unabhängig ausgewählt aus N, O und S, substituiert sind, der Ausdruck "Heterocyclyl" ein monocyclisches oder bicyclisches System mit bis zu 10 Atomen in jedem Ring, enthaltend 1-4 Heteroatome, ausgewählt aus O, N, S, SO oder SO₂, bedeutet,
der Ausdruck "Aryl" einen monocyclischen, bicyclischen oder tricyclischen carbocyclischen aromatischen Ring oder ein monocyclisches, bicyclisches oder tricyclisches carbocyclisches aromatisches Ringsystem, enthaltend 5-14 Kohlenstoffatome, wobei wenigstens einer der Ringe aromatisch ist, bedeutet, und der Ausdruck "Heteroaryl" ein stabiles monocyclisches, bicyclisches oder tricyclisches Ringsystem, das 5-14 Kohlenstoffatome enthält und wenigstens ein Ring-Heteroatom, ausgewählt aus N, S (einschließlich SO und SO₂) und O, enthält, wobei wenigstens einer der Heteroatom-enthaltenden Ringe aromatisch ist, bedeutet.

2. Die Verbindung nach Anspruch 1 oder ein pharmazeutisch annehmbares Salz davon, wobei **R¹** Phenyl oder Pyridyl ist, wobei **R¹** durch 0, 1, 2 oder 3 **R⁶** substituiert ist.

3. Die Verbindung nach einem der Ansprüche 1 oder 2 oder ein pharmazeutisch annehmbares Salz davon, wobei jedes **R⁶** unabhängig ausgewählt ist aus: Halogen, Heteroaryl-C₀₋₁₀-alkyl, Heterocyclyl-C₀₋₁₀-alkyl, Heterocyclyl(C₀₋₁₀)alkylaminocarbonyl, -NHSO₂(C₀₋₆-Alkyl), - SO₂N(C1-6-Alkyl)₀₋₂, C₁₋₁₀-Alkyl-S(O)₁₋₂, C₁₋₁₀-Heteroalkyl-S(O)₁₋₂, (C₃₋₁₂)Cycloalkyl-S(O)₁₋₂, Heterocyclyl-S(O)₁₋₂, Heteroaryl-S(O)₁₋₂ und Aryl-S(O)₁₋₂, wobei **R⁶** jeweils mit 0, 1, 2, 3 oder 4 **R⁷** substituiert ist.

4. Die Verbindung nach einem der Ansprüche 1 oder 2 oder ein pharmazeutisch annehmbares Salz davon, wobei jedes **R⁶** unabhängig ausgewählt ist aus Fluor, Chlor, Brom, lod, Oxazolidinyl, Pyridazinyl, Piperidinyl, Pyridinyl, Oxaazabicyclo-[2.2.2]octanyl, 2-Oxa-5-azabicyclo-[2.2.2]octanyl, Imidazolinyl, Ethylsulfonyl, Pyrazolyl, Azetidinylcarbamoyl und - NS(O)₂H, wobei **R⁶** jeweils mit 0, 1 oder 2 **R⁷** substituiert ist.

5. Die Verbindung nach einem der Ansprüche 1 bis 4 oder ein pharmazeutisch annehmbares Salz davon, wobei jedes **R²** unabhängig ausgewählt ist aus C₁₋₁₀-Alkyl, C₃₋₁₂-Cycloalkyl-C₀₋₁₀-alkyl, Heteroaryl-C₀₋₁₀-alkyl, Heterocyclyl-C₀₋₁₀-alkyl, C₃₋₁₂-Cycloalkyl-C₀₋₁₀-alkylamino-C₀₋₁₀-alkyl, Amino und (C₁₋₁₀-Alkyl)₁₋₂-amino-C₀₋₁₀-alkyl, wobei **R²** jeweils mit 0, 1, 2, 3 oder 4 **R⁵** substituiert ist.

6. Die Verbindung nach einem der Ansprüche 1 bis 4 oder ein pharmazeutisch annehmbares Salz davon, wobei jedes **R²** unabhängig ausgewählt ist aus Cyclopropylmethyl, Dimethylamino, Pyrrolidinyl, Oxazepanyl, Azetidinyl, Azabicyclo[3.1.1]heptyl, Decahydroisochinolinyl, Azaspiro[2.5]octanyl, 6-Azaspiro[2.5]octanyl, Azaspiro[4.5]decanyl, Oxaazaspiro[4.5]decyl und Oxaazaspiro[3.5]nonyl, Piperidinyl, wobei **R⁵** jeweils mit 0, 1, 2, 3 oder 4 **R⁵** substituiert ist.

7. Die Verbindung nach einem der Ansprüche 1 bis 6 oder ein pharmazeutisch annehmbares Salz davon, wobei **R⁵** unabhängig ausgewählt ist aus C₁₋₁₀-Alkyl, C₃₋₁₂-Cycloalkyl-C₀₋₁₀-alkyl, Heteroaryl-C₀₋₁₀-alkyl, Heterocyclyl-C₀₋₁₀-alkyl, ((C₁₋₁₀-Alkyl)₁₋₂-amino)C₀₋₁₀-alkyl, - CO₂(C₁₋₁₀-Alkyl), -(C₀₋₁₀-Alkyl)CO₂H, -(C₀₋₁₀-Alkyl)CO₂(C₁₋₁₀-alkyl), Oxo, Hydroxy, -(C₁₋₁₀-Alkyl)OH, C₁₋₁₀-Alkoxy-C₀₋₁₀-alkyl und C₁₋₆-Halogenalkyl, wobei jedes **R⁵** unabhängig mit 0, 1, 2, 3 oder 4 **R⁹** substituiert ist.

8. Die Verbindung nach einem der Ansprüche 1 bis 6 oder ein pharmazeutisch annehmbares Salz davon, wobei jedes **R⁵** unabhängig ausgewählt ist aus Methyl, Isopropyl, Ethyl, Butyl, *tert*-Butyl, (Cyclopropyl)methyl, Morpholinomethyl, Methoxymethyl, Methoxypropyl, Methoxy, (Dimethylamino)ethyl, (Dimethylamino)methyl, Hydroxymethyl, Carboxy, Hydroxy(methylethyl), Trifluormethyl, wobei jedes **R⁵** unabhängig mit 0, 1, 2, 3 oder 4 **R⁹** substituiert ist.

9. Die Verbindung nach einem der Ansprüche 1 bis 8 oder ein pharmazeutisch annehmbares Salz davon, wobei jedes **R³** unabhängig ausgewählt ist aus Halogen, Isopropyl, Methyl, Ethyl-*tert*-butyl, Difluormethyl, Cyclopropyl und -Si(CH₃)₃, wobei jedes **R³** unabhängig mit 0, 1 oder 2 **R⁸** substituiert ist.

10. Die Verbindung nach einem der Ansprüche 1 bis 9 oder ein pharmazeutisch annehmbares Salz davon, wobei **R⁴** unabhängig ausgewählt ist aus Fluor, Chlor und Methyl.

11. Die Verbindung nach einem der Ansprüche 1 bis 10 oder ein pharmazeutisch annehmbares Salz davon, wobei jedes **R⁷** unabhängig ausgewählt ist aus Halogen, C₁₋₁₀-Alkyl, C₁₋₆-Halogenalkyl, Amino-C₀₋₁₀-alkyl, ((C₁₋₁₀)Alkyl)₁₋₂-amino, Oxo, Hydroxy, -(C₁₋₁₀-Alkyl)OH und C₁₋₁₀-Alkoxy.

12. Die Verbindung nach einem der Ansprüche 1 bis 11 oder ein pharmazeutisch annehmbares Salz davon, wobei jedes **R⁸** unabhängig ausgewählt ist aus: Methyl, Ethyl, Propyl, Butyl, Methoxy, Difluormethyl, Fluor und Chlor.

13. Die Verbindung nach einem der Ansprüche 1 bis 12 oder ein pharmazeutisch annehmbares Salz davon, wobei jedes **R⁹** unabhängig ausgewählt ist aus: Methyl, Ethyl, Hydroxy, Methoxy, Ethoxy, Halogen und Trifluormethyl.

14. Die Verbindung nach Anspruch 1 oder ein pharmazeutisch annehmbares Salz davon, ausgewählt aus:
(S)-N-((R)-3-(4-Hydroxypiperidin-1-yl)-1-(4-(2-oxooxazolidin-3-yl)phenyl)propyl)-7-(1-methylyclopropyl)-5,6,7,8-tertrahydroacridin-2-carboxamid,
(S)-N-((R)-3-((S)-2-(Hydroxymethyl)pyrrolidin-1-yl)-1-(4-(2-oxooxazolidin-3-yl)phenyl)propyl)-7-(1-methylcyclopropyl)-5,6,7,8-tetrahydroacridin-2-carboxamid,
(7S)-7-tert-Butyl-N-{(1R)-3-(4-hydroxypiperidin-1-yl)-1-[4-(2-oxo-1,3-oxazolidin-3-yl)phenyl]propyl}-5,6,7,8-tetrahydroacridin-2-carboxamid,
(7S)-N-{(1R)-3-(4-Hydroxypiperidin-1-yl)-1-[4-(2-oxo-1,3-oxazolidin-3-yl)phenyl]propyl}-7-(trimethylsilyl)-5,6,7,8-tetrahydroacridin-2-carboxamid,
(7S)-4,7-Difluor-N-{(1R)-3-(4-hydroxypiperidin-1-yl)-1-[4-(2-oxo-1,3-oxazolidin-3-yl)phenyl]propyl}-7-(1-methylethyl)-5,6,7,8-tetrahydroacridin-2-carboxamid,
(7S)-3,4,7-Trifluor-N-{(1R)-3-(4-hydroxypiperidin-1-yl)-1-[4-(2-oxo-1,3-oxazolidin-3-yl)phenyl]propyl}-7-(1-methylethyl)-5,6,7,8-tetrahydroacridin-2-carboxamid,
7-tert-Butyl-3-fluor-N-{(1R)-3-(4-hydroxypiperidin-1-yl)-1-[4-(2-oxo-1,3-oxazolidin-3-yl)phenyl]propyl}-5,6,7,8-tetrahydroacridin-2-carboxamid,
(S)-7-(tert-Butyl)-4-chlor-N-((R)-3-(4-hydroxypiperidin-1-yl)-1-(4-(2-oxooxazolidin-3-yl)phenyl)propyl)-5,6,7,8-tetrahydroacridin-2-carboxamid,
7-(tert-Butyl)-N-((R)-3-(4-hydroxypiperidin-1-yl)-1-(4-(2-oxooxazolidin-3-yl)phenyl)propyl)-4-methyl-5,6,7,8-tetrahydroacridin-2-carboxamid,
(7S)-4,7-Difluor-N-[(1R)-3-(4-hydroxypiperidin-1-yl)-1-(6-pyridazin-4-ylpyridin-3-yl)propyl]-7-(1-methylethyl)-5,6,7,8-tetrahydroacridin-2-carboxamid,
(7S)-4,7-Difluor-N-[(1R)-3-(4-hydroxy-2,2-dimethylpiperidin-1-yl)-1-(6-pyridazin-4-ylpyridin-3-yl)propyl]-7-(1-methylethyl)-5,6,7,8-tetrahydroacridin-2-carboxamid,
(7S)-4,7-Difluor-N-[(1R)-3-(4-hydroxy-4-methylpipyridin-1-yl)-1-(6-pyridazin-4-ylpyridin-3-yl)propyl]-7-(1-methylethyl)-5,6,7,8-tetrahydroacridin-2-carboxamid,
1-[(3R)-3-({[(7S)-4,7-Difluor-7-(1-methylethyl)-5,6,7,8-tetrahydroacridin-2-yl]carbonyl}amino)-3-(6-pyridazin-4-ylpyridin-3-yl)propyl]-4-hydroxypiperidin-4-carbonsäure,
(7S)-4,7-Difluor-N-[(1R)-3-[6-hydroxy-6-(trifluormethyl)-3-azabicyclo[3.1.1]hept-3-yl]-1-(6-pyridazin-4-ylpyridin-3-yl)propyl]-7-(1-methylethyl)-5,6,7,8-tetrahydroacridin-2-carboxamid,
(7S)-4,7-Difluor-N-[(1R)-3-(4a-hydroxyoctahydroisochinolin-2(1H)-yl)-1-(6-pyridazin-4-ylpyridin-3-yl)propyl]-7-(1-methylethyl)-5,6,7,8-tetrahydroacridin-2-carboxamid,
(7S)-4,7-Difluor-7-(1-methylethyl)-N-[(1R)-3-(1-oxa-8-azaspiro[4.5]dec-8-yl)-1-(6-pyridazin-4-ylpyridin-3-yl)propyl]-5,6,7,8-tetrahydroacridin-2-carboxamid,
(7S)-4,7-Difluor-7-(1-methylethyl)-N-[(1R)-3-(1-oxa-7-azaspiro[3.5]non-7-yl)-1-(6-pyridazin-4-ylpyridin-3-yl)propyl]-5,6,7,8-tetrahydroacridin-2-carboxamid,
(7S)-4,7-Difluor-N-[(1R)-3-(4-methoxypiperidin-1-yl)-1-(6-pyridazin-4-ylpyridin-3-yl)propyl]-7-(1-methylethyl)-5,6,7,8-tetrahydroacridin-2-carboxamid,
(7S)-4,7-Difluor-N-[(1R)-3-[4-hydroxy-4-(hydroxymethyl)piperidin-1-yl]-1-(6-pyridazin-4-ylpyridin-2-yl)propyl]-7-(1-methylethyl)-5,6,7,8-tetrahydroacridin-2-carboxamid,
(7S)-4,7-Difluor-N-[(1R)-1-(5-fluor-6-pyridazin-4-ylpyridin-3-yl)-3-(4-hydroxypiperidin--yl)propyl]-7-(1-methylethyl)-5,6,7,8-tetrahydroacridin-2-carboxamid,
(S)-N-((R)-3-((S)-2-(Hydroxymethyl)pyrrolidin-1-yl)-1-(3-((1-methylazetidin-3-yl)carbamoyl)-phenyl)propyl)-7-(1-methylcyclopropyl)-5,6,7,8-tetrahydroacridin-2-carboxamid,
(S)-N-((R)-3-((S)-2-(Hydroxymethyl)pyrrolidin-1-yl)-1-(3-((1-methylazetidin-3-yl)carbamoyl)-phenyl)propyl)-7-(1-(trifluormethyl)cyclopropyl)-5,6,7,8-tetrahydroacridin-2-carboxamid,
((S)-7-(1-(Difluormethyl)cyclopropyl)-N-((R)-3-((S)-2-(hydroxymethyl)pyrrolidin-1-yl)-1-(3-((1-methylazetidin-3-yl)carbamoyl)phenyl)propyl)-5,6,7,8-tetrahydroacridin-2-carboxamid,
Methyl-1-((R)-3-((S)-4,7-difluor-7-isopropyl-5,6,7,8-tetrahydroacridin-2-carboxamido)-3-(4-(5-fluor-6-hydroxypridin-3-yl)phenyl)propyl)piperidin-4-carboxylat,
1-((R)-3-((S)-4,7-Difluor-7-isopropyl-5,6,7,8-tetrahydroacridin-2-carboxamido)-3-(4-(5-fluor-6-hydroxypyridin-3-yl)phenyl)propyl)piperidin-4-carbonsäure,
(S)-N-((R)-1-(6-((N,N-Dimethylsulfamoyl)amino)pyridin-3-yl)-3-(4-hydroxypiperidin-1-yl)propyl)-4,7-difluor-7-isopropyl-5,6,7,8-tetrahydroacridin-2-carboxamid,
(S)-N-((R)-1-(6-((N,N-Dimethylsulfamoyl)amino)-5-fluorpyridin-3-yl)-3-(4-hydroxypiperidin-1-yl)propyl)-4,7-difluor-7-isopropyl-5,6,7,8-tetrahydroacridin-2-carboxamid,
(S)-N-((R)-1-(6-(2,4-Dioxoimidazolidin-1-yl)pyridin-3-yl)-3-(4-hydroxypiperidin-1-yl)propyl)-4,7-difluor-7-isopropyl-5,6,7,8-tetrahydroacridin-2-carboxamid,
(7S)-4,7-Difluor-N-((1R)-3-(4-hydroxypiperidin-1-yl)-1-(6-(5-methyl-2,4-dioxoimidazolidin-1-yl)pyridin-3-yl)propyl)-7-isopropyl-5,6,7,8-tetrahydroacridin-2-carboxamid,
(S)-N-((R)-1-(6-(Ethylsulfonyl)pyridin-3-yl)-3-(4-hydroxypiperidin-1-yl)propyl)-4,7-difluor-7-isopropyl-5,6,7,8-tetrahydroacridin-2-carboxamid,
(S)-9-(Hydroxymethyl)-N-((R)-3-(4-hydroxypiperidin-1-yl)-1-(4-(2-oxooxazolidin-3-yl)phenyl)propyl)-7-(1-methylcyclopropyl)-5,6,7,8-tetrahydroacridin-2-carboxamid,
(S)-N-((R)-1-(6-(1H-Pyrazol-4-yl)pyridin-3-yl)-3-(4-hydroxypiperidin-1-yl)propyl)-4,7-difluor-7-isopropyl-5,6,7,8-tetrahydroacridin-2-carboxamid,
(S)-4,7-Difluor-N-((R)-1-(6-((3R,4R)-3-fluor-4-hydroxypiperidin-1-yl)pyridin-3-yl)-3-(4-hydroxypiperidin-1-yl)propyl)-7-isopropyl-5,6,7,8-tetrahydroacridin-2-carboxamid,
(7S)-N-((1R)-1-(6-(2-Oxa-5-azabicyclo[2.2.2]octan-5-yl)pyridin-3-yl)-3-(4-hydroxypiperidin-1-yl)propyl)-4,7-difluor-7-isopropyl-5,6,7,8-tetrahydroacridin-2-carboxamid,
(7S)-4,7-Difluor-7-isopropyl-N-[(1R)-1-(6-pyridazin-4-yl-3-pyridyl)-3-pyrrolidin-1-ium-1-ylpropyl]-6,8-dihydro-5H-acridin-2-carboxamid-2,2,2-trifluoracetat,
(7S)-4,7-Difluor-7-isopropyl-N-[(1R)-1-(6-pyridazin-4-yl-3-pyridyl)-3-[(3S)-3-hydroxypiperidin-1-ium-1-yl]propyl]-6,8-dihydro-5H-acridin-2-carboxamid-2,2,2-trifluoracetat,
Dimethyl-[(3R)-3-(6-pyridazin-4-yl-3-pyridyl)-3-[[(7S)-4,7-difluor-7-isopropyl-6,8-dihydro-5H-acridin-2-carbonyl]amino]propyl]ammonium-2,2,2-trifluoracetat,
(7S)-4,7-Difluor-7-isopropyl-N-[(1R)-3-(2-oxa-8-azoniaspiro[4.5]decan-8-yl)-1-(6-pyridazin-4-yl-3-pyridyl)propyl]-6,8-dihydro-5H-acridin-2-carboxamid-2,2,2-trifluoracetat,
(7S)-4,7-Difluor-7-isopropyl-N-[(1R)-3-[2-(hydroxymethyl)-6-azoniaspiro[2.5]octan-6-yl]-1-(6-pyridazin-4-yl-3-pyridyl)propyl]-6,8-dihydro-5H-acridin-2-carboxamid-2,2,2-trifluoracetat,
(7S)-4,7-Difluor-7-isopropyl-N-[(1R)-1-(6-pyridazin-4-yl-3-pyridyl)-3-[(3R)-3-hydroxypiperidin-1-ium-1-yl]propyl]-6,8-dihydro-5H-acridin-2-carboxamid-2,2,2-trifluoracetat,
(7S)-4,7-Difluor-7-isopropyl-N-[(1R)-3-[4-(1-hydroxyethyl)piperidin-1-ium-1-yl]-1-(6-pyridazin-4-yl-3-pyridyl)propyl]-6,8-dihydro-5H-acridin-2-carboxamid-2,2,2-trifluoracetat,
(7S)-4,7-Difluor-7-isopropyl-N-[(1R)-3-(1,2,3,4,4a,5,6,7,8,8a-decahydroisochinolin-2-ium-2-yl)-1-(6-pyridazin-4-yl-3-pyridyl)propyl]-6,8-dihydro-5H-acridin-2-carboxamid-2,2,2-trifluoracetat,
(7S)-4,7-Difluor-7-isopropyl-N-[(1R)-3-[2-[2-(dimethylamino)ethyl]piperidin-1-ium-1-yl]-1-(6-pyridazin-4-yl-3-pyridyl)propyl]-6,8-dihydro-5H-acridin-2-carboxamid-2,2,2-trifluoracetat,
(7S)-4,7-Difluor-7-isopropyl-N-[(1R)-3-(2-methylpiperidin-1-ium-1-yl)-1-(6-pyridazin-4-yl-3-pyridyl)propyl]-6,8-dihydro-5H-acridin-2-carboxamid-2,2,2-trifluoracetat,
(7S)-4,7-Difluor-7-isopropyl-N-[(1R)-3-[4-(hydroxymethyl)piperidin-1-ium-1-yl]-1-(6-pyridazin-4-yl-3-pyridyl)propyl]-6,8-dihydro-5H-acridin-2-carboxamid-2,2,2-trifluoracetat,
(7S)-4,7-Difluor-7-isopropyl-N-[(1R)-3-[4-(methoxymethyl)piperidin-1-ium-1-yl]-1-(6-pyridazin-4-yl-3-pyridyl)propyl]-6,8-dihydro-5H-acridin-2-carboxamid-2,2,2-trifluoracetat,
(7S)-4,7-Difluor-7-isopropyl-N-[(1R)-3-[4-(1-hydroxy-1-methylethyl)piperidin-1-ium-1-yl]-1-(6-pyridazin-4-yl-3-pyridyl)propyl]-6,8-dihydro-5H-acridin-2-carboxamid-2,2,2-trifluoracetat,
(7S)-4,7-Difluor-7-isopropyl-N-[(1R)-1-(6-pyridazin-4-yl-3-pyridyl)-3-[(3S)-3-hydroxy-8-azaspiro[4,5]decan-8-yl]propyl]-6,8-dihydro-5H-acridin-2-carboxamid,
(7S)-4,7-Difluor-7-isopropyl-N-[(1R)-1-(6-pyridazin-4-yl-3-pyridyl)-3-[(3R)-3-hydroxy-8-azaspiro[4.5]decan-8-yl]propyl]-6,8-dihydro-5H-acridin-2-carboxamid,
(7S)-4,7-Difluor-7-isopropyl-N-[(1R)-3-(2-oxa-7-azaspiro[3.5]nonan-7-yl)-1-(6-pyridazin-4-yl-3-pyridyl)propyl]-6,8-dihydro-5H-acridin-2-carboxamid,
(7S)-4,7-Difluor-7-isopropyl-N-[(1R)-3-[4-(3-methoxypropyl)-1-piperidyl]-1-(6-pyridazin-4-yl-3-pyridyl)propyl]-6,8-dihydro-5H-acridin-2-carboxamid,
(7S)-4,7-Difluor-7-isopropyl-N-[(1R)-3-(4a-hydroxy-1,3,4,5,6,7,8,8a-octahydroisochinolin-2-yl)1-(6-pyridazin-4-yl-3-pyridyl)propyl]-6,8-dihydro-5H-acridin-2-carboxamid,
(7S)-4,7-Difluor-7-isopropyl-N-[(1R)-3-[2-(2-hydroxyethyl)-1-piperidyl]-1-(6-pyridazin-4-yl-3-pyridyl)propyl]-6,8-dihydro-5H-acridin-2-carboxamid,
(7S)-4,7-Difluor-7-isopropyl-N-[(1R)-3-[4-[hydroxy-[1-(hydroxymethyl)cyclopropyl]methyl]-1-piperidyl]-1-(6-pyridazin-4-yl-3-pyridyl)propyl]-6,8-dihydro-5H-acridin-2-carboxamid,
(7S)-4,7-Difluor-7-isopropyl-N-[(1R)-3-(1-oxa-8-azaspiro[4.5]decan-8-yl)-1-(6-pyridazin-4-yl-3-pyridyl)propyl]-6,8-dihydro-5H-acridin-2-carboxamid,
(7S)-4,7-Difluor-7-isopropyl-N-[(1R)-3-(4-isopropyl-1-piperidyl)-1-(6-pyridazin-4-yl-3-pyridyl)propyl]-6,8-dihydro-5H-acridin-2-carboxamid,
(7S)-4,7-Difluor-7-isopropyl-N-[(1R)-3-(1,4-oxazepan-4-yl)-1-(6-pyridazin-4-yl-3-pyridyl)propyl]-6,8-dihydro-5H-acridin-2-carboxamid,
(7S)-4,7-Difluor-7-isopropyl-N-[(1R)-3-[2-(hydroxymethyl)-1-piperidyl]-1-(6-pyridazin-4-yl-3-pyridyl)propyl]-6,8-dihydro-5H-acridin-2-carboxamid,
(7S)-4,7-Difluor-7-isopropyl-N-[(1R)-3-[2-[(dimethylamino)methyl]-1-piperidyl]-1-(6-pyridazin-4-yl-3-pyridyl)propyl]-6,8-dihydro-5H-acridin-2-carboxamid,
(7S)-4,7-Difluor-7-isopropyl-N-[(1R)-3-[4-(morpholinomethyl)-1-piperidyl]-1-(6-pyridazin-4-yl-3-pyridyl)propyl]-6,8-dihydro-5H-acridin-2-carboxamid,
(7S)-4,7-Difluor-7-isopropyl-N-[(1R)-1-(6-pyridazin-4-yl-3-pyridyl)-3-[(2R,6S)-2,6-dimethyl-1-piperidyl]propyl]-6,8-dihydro-5H-acridin-2-carboxamid,
(7S)-4,7-Difluor-7-isopropyl-N-[(1R)-3-(1-oxa-7-azaspiro[3.5]nonan-7-yl)-1-(6-pyridazin-4-yl-3-pyridyl)propyl]-6,8-dihydro-5H-acridin-2-carboxamid,
(7S)-4,7-Difluor-7-isopropyl-N-[(1R)-3-(3-hydroxyazetidin-1-ium-1-yl)-1-(6-pyridazin-4-yl-3-pyridyl)propyl]-6,8-dihydro-5H-acridin-2-carboxamid-2,2,2-trifluoracetat,
(7S)-4,7-Difluor-7-isopropyl-N-[(1R)-1-(6-pyridazin-4-yl-3-pyridyl)-3-[4-(trifluormethyl)-1-piperidyl]propyl]-6,8-dihydro-5H-acridin-2-carboxamid,
Methyl-2-[1-[(3R)-3-[3-[(1-methylazetidin-3-yl)carbamoyl]phenyl]-3-[[(7S)-7-(1-methyl-cyclopropyl)-5,6,7,8-tetrahydroacridin-2-carbonyl]amino]propyl]-4-piperidyl]acetat,
2-[1-[(3R)-3-[3-[(1-Methylazetidin-3-yl)carbamoyl]phenyl]-3-[[(7S)-7-(1-methylcyclopropyl)-5,6,7,8-tetrahydroacridin-2-carbonyl]amino]propyl]-4-piperidyl]essigsäure,
(7S)-7-(1-Methylcyclopropyl)-N-[(1R)-3-(dimethylamino)-1-[3-[(1-methylazetidin-3-yl)carbamoyl]phenyl]propyl]-5,6,7,8-tetrahydroacridin-2-carboxamid und
(7S)-7-(1-Methylcyclopropyl)-N-[(1R)-3-(4-hydroxy-1-piperidyl)-1-[3-[(1-methylazetidin-1-ium-3-yl)carbamoyl]phenyl]propyl]-5,6,7,8-tetrahydroacridin-2-carboxamid-2,2,2-trifluoracetat.

15. Eine pharmazeutische Zusammensetzung, die eine wirksame Menge einer Verbindung nach einem der Ansprüche 1 bis 14 oder eines pharmazeutisch annehmbaren Salzes davon und einen pharmazeutisch annehmbaren Träger umfasst.

16. Die pharmazeutische Zusammensetzung nach Anspruch 15, die ferner ein oder mehrere zusätzliche therapeutische Mittel umfasst.

17. Eine Verbindung nach einem der Ansprüche 1-14 oder ein pharmazeutisch annehmbares Salz davon zur Verwendung in der Therapie.

18. Eine Verbindung nach einem der Ansprüche 1-14 zur Verwendung bei der Behandlung von kardiometabolischen Erkrankungen, Nierenerkrankung oder Diabetes.

## Revendications

1. Composé de formule 1, ou un sel pharmaceutiquement acceptable de celui-ci : 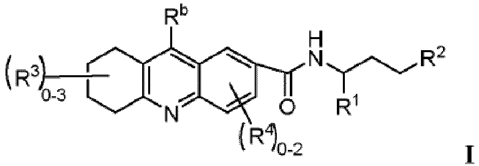
R^{b} est un hydrogène, un halogène, un C₁₋₆ alkyle, un hydroxy, un -(C₁₋₁₀ alkyl)OH ou un C₁₋₆ haloalkyle ;
R¹ est choisi parmi : phényle, pyridyle, thiazolyle, imidazolyle, pyrazinyle et oxadiazolyle, où R¹ est substitué par 0, 1, 2 ou 3 R⁶ ;
R² est indépendamment choisi parmi :
C₁₋₁₀ alkyle,
arylC₀₋₁₀ alkyle,
C₃₋₁₂ cycloalkylC₀₋₁₀ alkyle,
hétéroarylC₀₋₁₀ alkyle,
hétérocyclylC₀₋₁₀ alkyle,
C₁₋₁₀ alkylaminoC₀₋₁₀ alkyle,
hétéroarylC₀₋₁₀ alkylaminoC₀₋₁₀ alkyle,
hétérocyclylC₀₋₁₀ alkylaminoC₀₋₁₀ alkyle,
C₁₋₁₀ hétéroalkylaminoC₀₋₁₀ alkyle,
C₃₋₁₂ cycloalkylC₀₋₁₀ alkylaminoC₀₋₁₀ alkyle,
arylC₀₋₁₀ alkylaminoC₀₋₁₀ alkyle,
amino, et
(C₁₋₁₀ alkyl)₁₋₂aminoC₀₋₁₀ alkyle, où chaque R² est substitué par 0, 1, 2, 3 ou 4 substituants R⁵ ;
chaque R³ est indépendamment choisi parmi : halogène, C₁₋₆ alkyle, C₃₋₁₂ cycloalkyle, - Si(CH₃)₃ et C₁₋₆ haloalkyle, dans lequel chaque R³ est indépendamment substitué par 0, 1 ou 2 substituants R⁸ et chaque R⁸ est indépendamment choisi parmi : C₁₋₆ alkyle, C₁₋₆ alcoxy, halogène et C₁₋₆ haloalkyle ;
chaque R⁴ est indépendamment choisi parmi : halogène, C₁₋₆ alkyle et C₁₋₆ haloalkyle ;
chaque R⁵ est indépendamment choisi parmi :
halogène,
C₁₋₁₀ alkyle,
C₁₋₁₀ hétéroalkyle,
arylC₀₋₁₀ alkyle,
C₃₋₁₂ cyclalkylC₀₋₁₀ alkyle,
hétéroarylC₀₋₁₀ alkyle,
hétérocyclylC₀₋₁₀ alkyle,
aminoC₀₋₁₀ alkyle,
((C₁₋₁₀ alkyl)₁₋₂amino)C₀₋₁₀ alkyle,
-CO₂(C₁₋₁₀ alkyle),
-(C₀₋₁₀ alkyl)CO₂H,
-(C₀₋₁₀ alkyl)CO₂(C₁₋₁₀ alkyle),
oxo (=O),
hydroxy,
-(C₁₋₁₀ alkyl)OH,
C₁₋₁₀ alcoxyC₀₋₁₀ alkyle,
cyano, et
C₁₋₆ haloalkyle, où chaque R⁵ est indépendamment substitué par 0, 1, 2, 3 ou 4 R⁹ et chaque R⁹ est indépendamment choisi parmi : C₁₋₆ alkyle, hydroxy, C₁₋₆ alcoxy, halogène et C₁₋₆ haloalkyle ;
R⁶ est indépendamment choisi parmi :
halogène,
C₁₋₁₀ alkyle,
arylC₀₋₁₀ alkyle,
C₃₋₁₂ cycloalkylC₀₋₁₀ alkyle,
hétéroarylC₀₋₁₀ alkyle,
hétérocyclylC₀₋₁₀ alkyle,
((C₀₋₁₀)alkyle)₁₋₂aminocarbonyle,
C₁₋₁₀ alcoxy(C₀₋₁₀)alkyle,
(C₀₋₁₀)alkylaminocarbonyle,
(C₁₋₁₀)hétéroalkylaminocarbonyle,
aryl(C₀₋₁₀)alkylaminocarbonyle,
(C₃₋₁₂)cycloalkyl(C₀₋₁₀)alkylaminocarbonyle,
hétéroaryl(C₀₋₁₀)alkylaminocarbonyle,
hétérocyclyl(C₀₋₁₀)alkylaminocarbonyle,
-NHSO₂(C₀₋₆ alkyle),
-SO₂N(C₁₋₁₀ alkyle)₀₋₂,
-SO₂CF₃,
-SO₂CF₂H,
amino,
(C₀₋₁₀ alkyl)₁₋₂amino,
hydroxy,
-(C₁₋₁₀ alkyl)OH,
cyano,
C₁₋₆ haloalkyle,
-CO₂(C₁₋₁₀ alkyle),
oxo (=O) ;
C₁₋₁₀ alkylS(O)₁₋₂,
C₁₋₁₀ hétéroalkylS(O)₁₋₂,
(C₃₋₁₂)cycloalkylS(O)₁₋₂,
hétérocyclylS(O)₁₋₂,
hétéroarylS(O)₁₋₂, et
arylS(O)₁₋₂ ;
où chaque R⁶ est substitué par 0, 1, 2, 3 ou 4 R⁷ ;
chaque R⁷ est indépendamment choisi parmi :
halogène,
C₁₋₁₀ alkyle,
C₁₋₆ haloalkyle,
C₁₋₁₀ hétéroalkyle,
arylC₀₋₁₀ alkyle,
C₃₋₁₂ cycloalkylC₀₋₁₀ alkyle,
hétéroarylC₀₋₁₀ alkyle,
hétérocyclylC₀₋₁₀ alkyle,
aminoC₀₋₁₀ alkyle,
((C₁₋₁₀)alkyl)₁₋₂amino,
-CO₂(C₁₋₁₀ alkyle),
-(C₀₋₁₀ alkyl)CO₂H,
oxo (=O),
hydroxy,
-(C₁₋₁₀ alkyl)OH,
C₁₋₁₀ alcoxy,
cyano, et
aminocarbonyle ; dans lequel :
le terme « hétéroalkyle » se réfère à un groupe alkyle où 1, 2 ou 3 des atomes de carbone sont substitués par un hétéroatome indépendamment choisi parmi N, O ou S ;
le terme « hétérocyclyle » signifie un système monocyclique ou bicyclique de jusqu'à 10 atomes de carbone dans chaque cycle contenant 1-4 hétéroatomes choisis parmi O, N, S, SO ou SO₂ ;
le terme « aryle » se réfère à un cycle ou système cyclique carbocyclique aromatique monocyclique, bicyclique ou tricyclique contenant 5-14 atomes de carbone, dans lequel au moins un des cycles est aromatique ; et le terme « hétéroaryle » se réfère à un système cyclique monocyclique, bicyclique ou tricyclique stable contenant 5-14 atomes de carbone et contenant au moins un hétéroatome de cycle choisi parmi N, S (incluant SO et SO₂) et O, dans lequel au moins un des cycles contenant des hétéroatomes est aromatique.

2. Composé selon la revendication 1, ou un sel pharmaceutiquement acceptable de celui-ci, dans lequel R¹ est un phényle ou un pyridyle, dans lequel R¹ est substitué par 0, 1, 2 ou 3 R⁶.

3. Composé selon l'une quelconque des revendications 1 ou 2, ou un sel pharmaceutiquement acceptable de celui-ci, dans lequel chaque R⁶ est indépendamment choisi parmi : halogène, hétéroarylC₀₋₁₀ alkyle, hétérocyclylC₀₋₁₀ alkyle, hétérocyclyl(C₀₋10)alkylaminocarbonyle, -NHSO₂(C₀₋₆ alkyle), -SO₂N(C₁₋₆ alkyle)₀₋₂, C₁₋₁₀ alkylS(O)₁₋₂, C₁₋₁₀ hétéroalkylS(O)₁₋₂, (C₃₋₁₂)cycloalkylS(O)₁₋₂, hétérocyclylS(O)₁₋₂, hétéroarylS(O)₁₋₂ et arylS(O)₁₋₂, dans lequel chaque R⁶ est substitué par 0, 1, 2, 3 ou 4 R⁷.

4. Composé selon l'une quelconque des revendications 1 ou 2, ou un sel pharmaceutiquement acceptable de celui-ci, dans lequel chaque R⁶ est indépendamment choisi parmi : fluoro, chloro, bromo, iodo, oxazolidinyle, pyridazinyle, pipéridinyle, pyridinyle, oxa-azabicyclo-[2.2.2]octanyle, 2-oxa-5-azabicyclo-[2.2.2]octanyle, imidazolinyle, éthylsulfonyle, pyrazolyle, azétidinylcarbamoyle et -NS(O)₂H, dans lequel chaque R⁶ est substitué par 0, 1 ou 2 R7.

5. Composé selon l'une quelconque des revendications 1 à 4, ou un sel pharmaceutiquement acceptable de celui-ci, dans lequel chaque R² est indépendamment choisi parmi : C₁₋₁₀ alkyle, C₃₋₁₂ cycloalkylC₀₋₁₀ alkyle, hétéroarylC₀₋₁₀ alkyle, hétérocyclylC₀₋₁₀ alkyle, C₃₋₁₂ hétéroalkylC₀₋₁₀ alkylaminoC₀₋₁₀ alkyle, amino et (C₁₋₁₀ alkyl)₁₋₂aminoC₀₋₁₀ alkyle, dans lequel chaque R² est substitué par 0, 1, 2, 3 ou 4 R⁵.

6. Composé selon l'une quelconque des revendications 1 à 4, ou un sel pharmaceutiquement acceptable de celui-ci, dans lequel chaque R² est indépendamment choisi parmi : cyclopropylméthyle, diméthylamino, pyrrolidinyle, oxazépanyle, azétidinyle, azabicyclo[3.1.1]heptyle, décahydroisoquinoléinyle, azaspiro[2.5]octanyle, 6-azaspiro[2.5]octanyle, azaspiro[4.5]décanyle, oxa-azaspiro[4.5]décyle et oxa-azaspiro[3.5]nonyle, pipéridinyle, dans lequel chaque R² est substitué par 0, 1, 2, 3 ou 4 R⁵.

7. Composé selon l'une quelconque des revendications 1 à 6, ou un sel pharmaceutiquement acceptable de celui-ci, dans lequel R⁵ est indépendamment choisi parmi : C₁₋₁₀ alkyle, C₃₋₁₂ cyclalkylC₀₋₁₀ alkyle, hétéroarylC₀₋₁₀ alkyle, hétérocyclylC₀₋₁₀ alkyle, ((C₁₋₁₀ alkyl)₁₋₂amino)C₀₋₁₀ alkyle, -CO₂(C₁₋₁₀ alkyle), -(C₀₋₁₀ alkyl)CO₂H, -(C₀₋₁₀ alkyl)COa(C₁₋₁₀ alkyle), oxo, hydroxy, - (C₁₋₁₀ alkyl)OH, C₁₋₁₀ alcoxyC₀₋₁₀ alkyle et C₁₋₆ haloalkyle, dans lequel chaque R⁵ est indépendamment substitué par 0, 1, 2, 3 ou 4 R⁹.

8. Composé selon l'une quelconque des revendications 1 à 6, ou un sel pharmaceutiquement acceptable de celui-ci, dans lequel chaque R⁵ est indépendamment choisi parmi : méthyle, isopropyle, éthyle, butyle, tert-butyle, (cyclopropyl)méthyle, morpholinométhyle, méthoxyméthyle, méthoxypropyle, méthoxy, (diméthylamino)éthyle, (diméthylamino)méthyle, hydroxyméthyle, carboxy, hydroxy(méthyléthyle), trifluorométhyle, dans lequel chaque R⁵ est indépendamment substitué par 0, 1, 2, 3 ou 4 R⁹.

9. Composé selon l'une quelconque des revendications 1 à 8, ou un sel pharmaceutiquement acceptable de celui-ci, dans lequel chaque R³ est indépendamment choisi parmi : halogène, isopropyle, méthyle, éthyle tert-butyle, difluorométhyle, cyclopropyle et -Si(CH₃)₃, dans lequel chaque R³ est indépendamment substitué par 0, 1 ou 2 R⁸.

10. Composé selon l'une quelconque des revendications 1 à 9, ou un sel pharmaceutiquement acceptable de celui-ci, dans lequel R⁴ est indépendamment choisi parmi : fluoro, chloro et méthyle.

11. Composé selon l'une quelconque des revendications 1 à 10, ou un sel pharmaceutiquement acceptable de celui-ci, dans lequel R⁷ est indépendamment choisi parmi : halogène, C₁₋₁₀ alkyle, C₁₋₆ haloalkyle, aminoC₀₋₁₀ alkyle, ((C₁₋₁₀)alkyl)₁₋₂amino, oxo, hydroxy, - (C₁₋₁₀ alkyl)OH et C₁₋₁₀ alcoxy.

12. Composé selon l'une quelconque des revendications 1 à 11, ou un sel pharmaceutiquement acceptable de celui-ci, dans lequel chaque R⁸ est indépendamment choisi parmi : méthyle, éthyle, propyle, butyle, méthoxy, difluorométhyle, fluoro et chloro.

13. Composé selon l'une quelconque des revendications 1 à 12, ou un sel pharmaceutiquement acceptable de celui-ci, dans lequel chaque R⁹ est indépendamment choisi parmi : méthyle, éthyle, hydroxy, méthoxy, éthoxy, halogène et trifluorométhyle.

14. Composé selon la revendication 1, ou un sel pharmaceutiquement acceptable de celui-ci, choisi parmi :
(S)-N-((R)-3-(4-hydroxypipéridin-1-yl)-1-(4-(2-oxooxazolidin-3-yl)phényl)propyl)-7-(1-méthylcyclopropyl)-5,6,7,8-tétrahydroacridine-2-carboxamide ;
(S)-N-((R)-3-((S)-2-(hydroxyméthyl)pyrrolidin-1-yl)-1-(4-(2-oxooxazolidin-3-yl)phényl)propyl)-7-(1-méthylcyclopropyl)-5,6,7,8-tétrahydroacridine-2-carboxamide ;
(7S)-7-tert-butyl-N-{(1R)-3-(4-hydroxypipéridin-1-yl)-1-[4-(2-oxo-1,3-oxazolidin-3-yl)phényl]propyl}-5,6,7,8-tétrahydroacridine-2-carboxamide ;
(7S)-N-{(1R)-3-(4-hydroxypipéridin-1-yl)-1-[4-(2-oxo-1,3-oxazolidin-3-yl)phényl]propyl}-7-(triméthylsilyl)-5,6,7,8-tétrahydroacridine-2-carboxamide ;
(7S)-4,7-difluoro-N-{(1R)-3-(4-hydroxypipéridin-1-yl)-1-[4-(2-oxo-1,3-oxazolidin-3-yl)phényl]propyl} -7-(1 -méthyléthyl)-5,6,7,8-tétrahydroacridine-2-carboxamide ;
(7S)-3,4,7-trifluoro-N-{(1R)-3-(4-hydroxypipéridin-1-yl)-1-[4-(2-oxo-1,3-oxazolidin-3-yl)phényl]propyl} -7-(1 -méthyléthyl)-5,6,7,8-tétrahydroacridine-2-carboxamide ;
7-tert-butyl-3-fluoro-N-{(1R)-3-(4-hydroxypipéridin-1-yl)-1-[4-(2-oxo-1,3-oxazolidin-3-yl)phényl]propyl}-5,6,7,8-tétrahydroacridine-2-carboxamide ;
(S)-7-(tert-butyl)-4-chloro-N-((R)-3-(4-hydroxypipéridin-1-yl)-1-(4-(2-oxooxazolidin-3-yl)phényl)propyl)-5,6,7,8-tétrahydroacridine-2-carboxamide ;
7-(tert-butyl)-N-((R)-3-(4-hydroxypipéridin-1-yl)-1-(4-(2-oxooxazolidin-3-yl)phényl)propyl)-4-méthyl-5,6,7,8-tétrahydroacridine-2-carboxamide ;
(7S)-4,7-difluoro-N-[(1R)-3-(4-hydroxypipéridin-1-yl)-1-(6-pyridazin-4-ylpyridin-3-yl)propyl]-7-(1-méthyléthyl)-5,6,7,8-tétrahydroacridine-2-carboxamide ;
(7S)-4,7-difluoro-N-[(1R)-3-(4-hydroxy-2,2-diméthylpipéridin-1-yl)-1-(6-pyridazin-4-ylpyridin-3-yl)propyl]-7-(1-méthyléthyl)-5,6,7,8-tétrahydroacridine-2-carboxamide ;
(7S)-4,7-difluoro-N-[(1R)-3-(4-hydroxy-4-méthylpipéridin-1-yl)-1-(6-pyridazin-4-ylpyridin-3-yl)propyl]-7-(1-méthyléthyl)-5,6,7,8-tétrahydroacridine-2-carboxamide ;
acide 1-[(3R)-3-({[(7S)-4,7-difluoro-7-(1-méthyléthyl)-5,6,7,8-tétrahydroacridine-2-yl]carbonyl}amino)-3-(6-pyridazin-4-ylpyridin-3-yl)propyl]-4-hydroxypipéridine-4-carboxylique ;
(7S)-4,7-difluoro-N-[(1R)-3-[6-hydroxy-6-(trifluorométhyl)-3-azabicyclo[3.1.1]hept-3-yl]-1-(6-pyridazin-4-ylpyridin-3-yl)propyl]-7-(1-méthyléthyl)-5,6,7,8-tétrahydroacridine-2-carboxamide ;
(7S)-4,7-difluoro-N-[(1R)-3-(4a-hydroxyoctahydroisoquinoléin-2(1H)-yl)-1-(6-pyridazin-4-ylpyridin-3-yl)propyl]-7-(1-méthyléthyl)-5,6,7,8-tétrahydroacridine-2-carboxamide ;
(7S)-4,7-difluoro-7-(1-méthyléthyl)-N-[(1R)-3-(1-oxa-8-azaspiro [4.5] déc-8-yl)-1-(6-pyridazin-4-ylpyridin-3-yl)propyl]-5,6,7,8-tétrahydroacridine-2-carboxamide ;
(7S)-4,7-difluoro-7-(1-méthyléthyl)-N-[(1R)-3-(1-oxa-7-azaspiro[3.5]non-7-yl)-1-(6-pyridazin-4-ylpyridin-3-yl)propyl]-5,6,7,8-tétrahydroacridine-2-carboxamide ;
(7S)-4,7-difluoro-N-[(1R)-3-(4-méthoxypipéridin-1-yl)-1-(6-pyridazin-4-ylpyridin-3-yl)propyl]-7-(1-méthyléthyl)-5,6,7,8-tétrahydroacridine-2-carboxamide ;
(7S)-4,7-difluoro-N-[(1R)-3-[4-hydroxy-4-(hydroxyméthyl)pipéridin-1-yl]-1-(6-pyridazin-4-ylpyridin-3-yl)propyl]-7-(1-méthyléthyl)-5,6,7,8-tétrahydroacridine-2-carboxamide ;
(7S)-4,7-difluoro-N-[(1R)-1-(5-fluoro-6-pyridazin-4-ylpyridin-3-yl)-3-(4-hydroxypipéridin-1-yl)propyl]-7-(1-méthyléthyl)-5,6,7,8-tétrahydroacridine-2-carboxamide ;
(S)-N-((R)-3-((S)-2-(hydroxyméthyl)pyrrolidin-1-yl)-1-(3-((1-méthylazétidin-3-yl)carbamoyl)phényl)propyl)-7-(1-méthylcyclopropyl)-5,6,7,8-tétrahydroacridine-2-carboxamide ;
(S)-N-((R)-3-((S)-2-(hydroxyméthyl)pyrrolidin-1-yl)-1-(3-((1-méthylazétidin-3-yl)carbamoyl)phényl)propyl)-7-(1-(trifluorométhyl)cyclopropyl)-5,6,7,8-tétrahydroacridine-2-carboxamide ;
((S)-7-(1-(difluorométhyl)cyclopropyl)-N-((R)-3-((S)-2-(hydroxyméthyl)pyrrolidin-1-yl)-1-(3-((1-méthylazétidin-3-yl)carbamoyl)phényl)propyl)-5,6,7,8-tétrahydroacridine-2-carboxamide ;
1-((R)-3-((S)-4,7-difluoro-7-isopropyl-5,6,7,8-tétrahydroacridine-2-carboxamido)-3-(4-(5-fluoro-6-hydroxypyridin-3-yl)phényl)propyl)pipéridine-4-carboxylate de méthyle ;
acide 1-((R)-3-((S)-4,7-difluoro-7-isopropyl-5,6,7,8-tétrahydroacridine-2-carboxamido)-3-(4-(5-fluoro-6-hydroxypyridin-3-yl)phényl)propyl)pipéridine-4-carboxylique ;
(S)-N-((R)-1-(6-((N,N-diméthylsulfamoyl)amino)pyridin-3-yl)-3-(4-hydroxypipéridin-1-yl)propyl)-4,7-difluoro-7-isopropyl)-5,6,7,8-tétrahydroacridine-2-carboxamide ;
(S)-N-((R)-1-(6-((N,N-diméthylsulfamoyl)amino)-5-fluoropyridin-3-yl)-3-(4-hydroxypipéridin-1-yl)propyl)-4,7-difluoro-7-isopropyl)-5,6,7,8-tétrahydroacridine-2-carboxamide ;
(S)-N-((R)-1-(6-(2,4-dioxoimidazolidin-1-yl)pyridin-3-yl)-3-(4-hydroxypipéridin-1-yl)propyl)-4,7-difluoro-7-isopropyl-5,6,7,8-tétrahydroacridine-2-carboxamide ;
(7S)-4,7-difluoro-N-((1R)-3-(4-hydroxypipéridin-1-yl)-1-(6-(5-méthyl-2,4-dioxoimidazolidin-1-yl)pyridin-3-yl)propyl)-7-isopropyl-5,6,7,8-tétrahydroacridine-2-carboxamide ;
(S)-N-((R)-1-(6-(éthylsulfonyl)pyridin-3-yl)-3-(4-hydroxypipéridin-1-yl)propyl)-4,7-difluoro-7-isopropyl-5,6,7,8-tétrahydroacridine-2-carboxamide ;
(S)-9-(hydroxyméthyl)-N-((R)-3-(4-hydroxypipéridin-1-yl)-1-(4-(2-oxooxazolidin-3-yl)phényl)propyl)-7-(1-méthylcyclopropyl)-5,6,7,8-tétrahydroacridine-2-carboxamide ;
(S)-N-((R)-1-(6-(1H-pyrazol-4-yl)pyridin-3-yl)-3-(4-hydroxypipéridin-1-yl)propyl)-4,7-difluoro-7-isopropyl-5,6,7,8-tétrahydroacridine-2-carboxamide ;
(S)-4,7-difluoro-N-((R)-1-(6-((3R,4R)-3-fluoro-4-hydroxypipéridin-1-yl)pyridin-3-yl)-3-(4-hydroxypipéridin-1-yl)propyl)-7-isopropyl-5,6,7,8-tétrahydroacridine-2-carboxamide ;
(7S)-N-((1R)-1-(6-(2-oxa-5-azabicyclo[2.2.2]octan-5-yl)pyridin-3-yl)-3-(4-hydroxypipéridin-1-yl)propyl)-4,7-difluoro-7-isopropyl-5,6,7,8-tétrahydroacridine-2-carboxamide ;
(7S)-4,7-difluoro-7-isopropyl-N-[(1R)-1-(6-pyridazin-4-yl-3-pyridyl)-3-pyrrolidin-1-ium-1-propyl]-6,8-dihydro-5H-acridine-2-carboxamide ; 2,2,2-trifluoroacétate ;
(7S)-4,7-difluoro-7-isopropyl-N-[(1R)-1-(6-pyridazin-4-yl-3-pyridyl)-3-[(3S)-3-hydroxypipéridin-1-ium-1-yl]propyl]-6,8-dihydro-SH-acridine-2-carboxamide ; 2,2,2-trifluoroacétate ;
diméthyl-[(3R)-3-(6-pyridazin-4-yl-3-pyridyl)-3-[[(7S)-4,7-difluoro-7-isopropyl-6,8-dihydro-5H-acridine-2-carbonyl]amino]propyl]ammonium ; 2,2,2-trifluoroacétate ;
(7S)-4,7-difluoro-7-isopropyl-N-[(1R)-3-(2-oxa-8-azoniaspiro[4.5]décan-8-yl)-1-(6-pyridazin-4-yl-3-pyridyl)propyl]-6,8-dihydro-5H-acridine-2-carboxamide ; 2,2,2-trifluoroacétate ;
(7S)-4,7-difluoro-7-isopropyl-N-[(1R)-3-[2-(hydroxyméthyl)-6-azoniaspiro[2.5]octan-6-yl]-1-(6-pyridazin-4-yl-3-pyridyl)propyl]-6,8-dihydro-5H-acridine-2-carboxamide ; 2,2,2-trifluoroacétate ;
(7S)-4,7-difluoro-7-isopropyl-N-[(1R)-1-(6-pyridazin-4-yl-3-pyridyl)-3-[(3R)-3-hydroxypipéridin-1-ium-1-yl]propyl]-6,8-dihydro-5H-acridine-2-carboxamide ; 2,2,2-trifluoroacétate ;
(7S)-4,7-difluoro-7-isopropyl-N-[(1R)-3-[4-(1-hydroxyéthyl)pipéridin-1-ium-1-yl]-1-(6-pyridazin-4-yl-3-pyridyl)propyl]-6,8-dihy dro-5H-acridine-2-carboxamide ; 2,2,2-trifluoroacétate ;
(7S)-4,7-difluoro-7-isopropyl-N-[(1R)-3-(1,2,3,4,4a,5,6,7,8,8a-décahydroisoquinoléin-2-ium-2-yl)-1-(6-pyridazin-4-yl-3-pyridyl)propyl]-6,8-dihydro-SH-acridine-2-carboxamide ; 2,2,2-trifluoroacétate ;
(7S)-4,7-difluoro-7-isopropyl-N-[(1R)-3-[2-[2-(diméthylamino)éthyl]pipéridin-1-ium-1-yl]-1-(6-pyridazin-4-yl-3-pyridyl)propyl]-6,8-dihydro-SH-acridine-2-carboxamide ; 2,2,2-trifluoroacétate ;
(7S)-4,7-difluoro-7-isopropyl-N-[(1R)-3-(2-méthylpipéridin-1-ium-1-yl)-1-(6-pyridazin-4-yl-3-pyridyl)propyl]-6,8-dihydro-5H-acridine-2-carboxamide ; 2,2,2-trifluoroacétate ;
(7S)-4,7-difluoro-7-isopropyl-N-[(1R)-3-[4-(hydroxyméthyl)pipéridin-1-ium-1-yl]-1-(6-pyridazin-4-yl-3-pyridyl)propyl]-6,8-dihydro-5H-acridine-2-carboxamide ; 2,2,2-trifluoroacétate ;
(7S)-4,7-difluoro-7-isopropyl-N-[(1R)-3-[4-(méthoxyméthyl)pipéridin-1-ium-1-yl]-1-(6-pyridazin-4-yl-3-pyridyl)propyl]-6,8-dihy dro-5H-acridine-2-carboxamide ; 2,2,2-trifluoroacétate ;
(7S)-4,7-difluoro-7-isopropyl-N-[(1R)-3-[4-(1-hydroxy-1-méthyl-éthyl)pipéridin-1-ium-1-yl]-1-(6-pyridazin-4-yl-3-pyridyl)propyl]-6,8-dihydro-SH-acridine-2-carboxamide ; 2,2,2-trifluoroacétate ;
(7S)-4,7-difluoro-7-isopropyl-N-[(1R)-1-(6-pyridazin-4-yl-3-pyridyl)-3-[(3S)-3-hydroxy-8-azaspiro[4.5]décan-8-yl]propyl]-6,8-dihydro-SH-acridine-2-carboxamide ;
(7S)-4,7-difluoro-7-isopropyl-N-[(1R)-1-(6-pyridazin-4-yl-3-pyridyl)-3-[(3R)-3-hydroxy-8-azaspiro[4.5]décan-8-yl]propyl]-6,8-dihydro-5H-acridine-2-carboxamide ;
(7S)-4,7-difluoro-7-isopropyl-N-[(1R)-3-(2-oxa-7-azaspiro[3.5]nonan-7-yl)-1-(6-pyridazin-4-yl-3-pyridyl)propyl]-6,8-dihydro-5H-acridine-2-carboxamide ;
(7S)-4,7-difluoro-7-isopropyl-N-[(1R)-3-[4-(3-méthoxypropyl)-1-pipéridyl]-1-(6-pyridazin-4-yl-3-pyridyl)propyl]-6,8-dihydro-5H-acridine-2-carboxamide ;
(7S)-4,7-difluoro-7-isopropyl-N-[(1R)-3-(4a-hydroxy-1,3,4,5,6,7,8,8a-octahydroisoquinoléin-2-yl)-1-(6-pyridazin-4-yl-3-pyridyl)propyl]-6,8-dihydro-5H-acridine-2-carboxamide ;
(7S)-4,7-difluoro-7-isopropyl-N-[(1R)-3-[2-(2-hydroxyéthyl)-1-pipéridyl]-1-(6-pyridazin-4-yl-3-pyridyl)propyl]-6,8-dihydro-5H-acridine-2-carboxamide ;
(7S)-4,7-difluoro-7-isopropyl-N-[(1R)-3-[4-[hydroxy-[1-(hydroxyméthyl)cyclopropyl]-méthyl]-1-pipéridyl]-1-(6-pyridazin-4-yl-3-pyridyl)propyl]-6,8-dihydro-5H-acridine-2-carboxamide ;
(7S)-4,7-difluoro-7-isopropyl-N-[(1R)-3-(1-oxa-8-azaspiro[4.5]décan-8-yl)-1-(6-pyridazin-4-yl-3-pyridyl)propyl]-6,8-dihydro-5H-acridine-2-carboxamide ;
(7S)-4,7-difluoro-7-isopropyl-N-[(1R)-3-(4-isopropyl-1-pipéridyl)-1-(6-pyridazin-4-yl-3-pyridyl)propyl]-6,8-dihydro-5H-acridine-2-carboxamide ;
(7S)-4,7-difluoro-7-isopropyl-N-[(1R)-3-(1,4-oxazépan-4-yl)-1-(6-pyridazin-4-yl-3-pyridyl)propyl]-6,8-dihydro-5H-acridine-2-carboxamide ;
(7S)-4,7-difluoro-7-isopropyl-N-[(1R)-3-[2-(hydroxyméthyl)-1-pipéridyl]-1-(6-pyridazin-4-yl-3-pyridyl)propyl]-6,8-dihydro-5H-acridine-2-carboxamide ;
(7S)-4,7-difluoro-7-isopropyl-N-[(1R)-3-[2-[(diméthylamino)méthyl]-1-pipéridyl]-1-(6-pyridazin-4-yl-3-pyridyl)propyl]-6,8-dihydro-5H-acridine-2-carboxamide ;
(7S)-4,7-difluoro-7-isopropyl-N-[(1R)-3-[4-(morpholinométhyl)-1-pipéridyl]-1-(6-pyridazin-4-yl-3-pyridyl)propyl]-6,8-dihydro-5H-acridine-2-carboxamide ;
(7S)-4,7-difluoro-7-isopropyl-N-[(1R)-1-(6-pyridazin-4-yl-3-pyridyl)-3-[(2R,6S)-2,6-diméthyl-1-pipéridyl]propyl]-6,8-dihydro-5H-acridine-2-carboxamide ;
(7S)-4,7-difluoro-7-isopropyl-N-[(1R)-3-(1-oxa-7-azaspiro[3.5]nonan-7-yl)-1-(6-pyridazin-4-yl-3-pyridyl)propyl]-6,8-dihydro-5H-acridine-2-carboxamide ;
(7S)-4,7-difluoro-7-isopropyl-N-[(1R)-3-(3-hydroxyazétidin-1-ium-1-yl)-1-(6-pyridazin-4-yl-3-pyridyl)propyl]-6,8-dihydro-5H-acridine-2-carboxamide ; 2,2,2-trifluoroacétate ;
(7S)-4,7-difluoro-7-isopropyl-N-[(1R)-1-(6-pyridazin-4-yl-3-pyridyl)-3-[4-(trifluorométhyl)-1-pipéridinyl]propyl]-6,8-dihydro-SH-acridine-2-carboxamide ;
2-[1-[(3R)-3-[3-[(1-méthylazétidin-3-yl)carbamoyl]phényl]-3-[[(7S)-7-(1-méthylcyclopropyl)-5,6,7,8-tétrahydroacridine-2-carbonyl]amino]propyl]-4-pipéridyl]acétate de méthyle ;
acide 2-[1-[(3R)-3-[3-[(1-méthylazétidin-3-yl)carbamoyl]phényl]-3-[[(7S)-7-(1-méthylcyclopropyl)-5,6,7,8-tétrahydroacridine-2-carbonyl]amino]propyl]-4-pipéridyl]acétique ;
(7S)-7-(1-méthylcyclopropyl)-N-[(1R)-3-(diméthylamino)-1-[3-[(1-méthylazétidin-3-yl)carbamoyl]phényl]propyl]-5,6,7,8-tétrahydroacridine-2-carboxamide ; et
(7S)-7-(1-méthylcyclopropyl)-N-[(1R)-3-(4-hydroxy-1-pipéridyl)-1-[3-[(1-méthylazétidin-1-ium-3-yl)carbamoyl]phényl]propyl]-5,6,7,8-tétrahydroacridine-2-carboxamide ; 2,2,2-trifluoroacétate.

15. Composition pharmaceutique comprenant une quantité efficace d'un composé selon l'une quelconque des revendications 1 à 14, ou d'un sel pharmaceutiquement acceptable de celui-ci, et un véhicule pharmaceutiquement acceptable.

16. Composition pharmaceutique selon la revendication 15, comprenant en outre un ou plusieurs agents thérapeutiques supplémentaires.

17. Composé selon l'une quelconque des revendications 1-14, ou un sel pharmaceutiquement acceptable de celui-ci, pour utilisation en thérapie.

18. Composé selon l'une quelconque des revendications 1-14 pour utilisation dans le traitement de maladies cardiométaboliques, d'une maladie rénale ou d'un diabète.
